(19) Europäisches Patentamt
European Patent Office
Office européen des brevets

(11) Publication number: 0 016 565 B1

(12) EUROPEAN PATENT SPECIFICATION

(45) Date of publication of patent specification: 23.05.84

(21) Application number: 80300617.0

(22) Date of filing: 29.02.80

(51) Int. Cl.³: C 07 D 249/14, C 07 D 401/12, C 07 D 405/12, C 07 D 409/12, A 61 K 31/41 // (C07D401/12, 249/14, 211/70),(C07D405/12, 249/14, 307/54),(C07D409/12, 249/14, 333/24)

# (54) 1,2,4-Triazole derivatives, processes for their production and pharmaceutical compositions containing them.

(30) Priority: 02.03.79 GB 7907423
02.03.79 GB 7907421

(43) Date of publication of application:
01.10.80 Bulletin 80/20

(45) Publication of the grant of the patent:
23.05.84 Bulletin 84/21

(84) Designated Contracting States:
AT BE CH DE FR IT LU NL SE

(56) References cited:
DE-A-2 734 070
DE-A-2 821 409
DE-A-2 821 410
DE-A-2 917 026
US-A-3 681 374
US-A-3 813 400
US-A-4 076 938
US-A-4 089 962
US-A-4 104 381

**The file contains technical information submitted after the application was filed and not included in this specification**

(73) Proprietor: GLAXO GROUP LIMITED
Clarges House 6-12 Clarges Street
London W1Y 8DH (GB)

(72) Inventor: Clitherow, John Watson
54, Gilers
Sawbridgeworth Hertfordshire (GB)
Inventor: Bradshaw, John
22 Whitely Close
Dane End, Ware Hertfordshire (GB)
Inventor: Price, Barry John
155 Ware Road
Hertford Hertfordshire (GB)
Inventor: Martin-Schmith, Michael
"Leycroft", The Street
Haultwick Dane End Ware Hertfordshire (GB)
Inventor: Mackinnon, John Wilson Macfarlane
13 Parklands
Royston Hertfordshire (GB)
Inventor: Judd, Duncan Bruce
61 Musley Lane
Ware Hertfordshire (GB)
Inventor: Hayes, Roger
117 Warren Way
Digswell Welwyn Hertfordshire (GB)
Inventor: Carey, Linda
15 Roan Walk
Royston Hertfordshire (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 016 565 B1

(74) Representative: **Marchant, James Ian et al**
**Elkington and Fife High Holborn House 52/54**
**High Holborn**
**London WC1V 6SH (GB)**

## Description

This invention relates to novel heterocyclic derivatives having action on histamine receptors, to processes for the preparation thereof, to pharmaceutical compositions containing them and to their use in therapeutics.

Certain novel heterocyclic derivatives have now been found which have potent activity as $H_2$-antagonists. These compounds, which are more particularly described below, for example show inhibition of the secretion of gastric acid when this is stimulated via histamine receptors (Ash and Schild, Brit.J.Pharmacol. Chemother, 1966, *27*, 427). Their ability to do so can be demonstrated in the perfused rat stomach using the method described in German Offenlegungsschrift No. 2,734,070, modified by the use of sodium pentobarbitone (50 mg/kg) as anaesthetic instead of urethane, and in conscious dogs equipped with Heidenhain pouches using the method described by Black *et al*, Nature 1972 *236*, 385. Furthermore the compounds antagonise the effect of histamine on the contraction frequency of isolated guinea pig right atrium but do not modify histamine induced contractions of isolated gastrointestinal smooth muscle which are mediated via $H_1$-receptors. Certain compounds according to the invention have the advantage of an extended duration of action.

Compounds with histamine $H_2$-blocking activity may be used in the treatment of conditions where there is an advantage in lowering gastric acidity, particularly in gastric and peptic ulceration, as a prophylactic measure in surgical procedures, and in the treatment of allergic and inflammatory conditions where histamine is a known mediator. Thus they may be used for example, either alone or in combination with other active ingredients in the treatment of allergic and inflammatory conditions of the skin.

There have been a number of disclosures in the prior art of compounds having histamine $H_2$-blocking activity and in particular reference can be made to German Offenlegungsschrifts 2734070, 2821409 and 2821410.

German Offenlegungsschrift 2734070 discloses compounds of the general formula

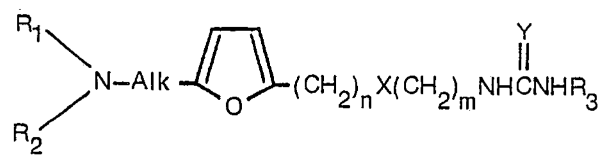

and physiologically acceptable salts thereof and N-oxides and hyrates, in which $R_1$ and $R_2$ which may be the same or different represent hydrogen, lower alkyl, cycloalkyl, lower alkenyl, aralkyl or lower alkyl interrupted by an oxygen atom or a group

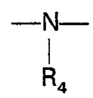

in which $R_4$ represents hydrogen or lower alkyl or $R_1$ and $R_2$ may, together with the nitrogen atom to which they are attached, form a heterocyclic ring which may contain other heteroatoms selected from O and

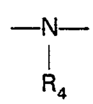

$R_3$ is hydrogen, lower alkyl, lower alkenyl or alkoxyalkyl;
X is $—CH_2—$, O or S;
Y represents =S, =O, $=NR_5$ or $=CHR_6$;
Alk denotes a straight or branched alkylene chain of 1 to 6 carbon atoms;
$R_5$ is H, nitro, cyano, lower alkyl, aryl, alkylsulphonyl, or arylsulphonyl;
$R_6$ represents nitro, arylsulphonyl or alkylsulphonyl;
m is an integer from 2 to 4; and
n is 1 or 2; or when X=S, or $—CH_2—$, n is zero, 1 or 2.

German Offenlegungsschrift 2821409 discloses compounds of the general formula

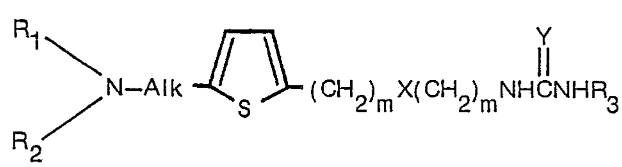

and physiologically acceptable salts, hydrates and bioprecursors thereof, in which

$R_1$ and $R_2$, which may be the same or different, represent hydrogen, lower alkyl, cycloalkyl, aralkyl

or lower alkenyl groups, which alkyl groups may be interrupted by an oxygen atom or a group

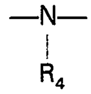

in which $R_4$ represents hydrogen or lower alkyl; or

$R_1$ and $R_2$ may, together with the nitrogen atom to which they are attached form a heterocyclic ring which may contain other hetero functions selected from —O— and

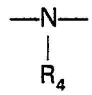

$R_3$ represents hydrogen, lower alkyl, lower alkenyl or alkoxyalkyl;

X represents —O—, —S— or —$CH_2$—;

Y represents =S, =O, =$NR_5$ or $CHR_6$; in which $R_5$ is hydrogen, nitro, cyano, lower alkyl, aryl, alkylsulphonyl or arylsulphonyl and $R_6$ represents nitro, alkylsulphonyl or arylsulphonyl;

m is an integer from 2 to 4 inclusive;

n is 1 or 2 or, when X is S or $CH_2$, n is zero, 1 or 2 and Alk denotes a straight or branched alkylene chain of 1 to 6 carbon atoms

German Offenlegungsschrift No. 2821410 discloses compounds of the general formula

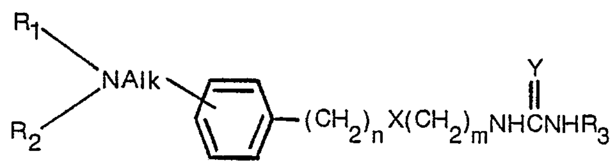

and physiologically acceptable salts, and hydrates, N-oxides and bioprecursors of such compounds and such salts in which the substituents attached to the benzene ring are ortho, meta or para to one another and, in which

$R_1$ and $R_2$, which may be the same or different, represent hydrogen or lower alkyl, cycloalkyl, aralkyl or lower alkenyl groups, or lower alkyl groups interrupted by an oxygen atom or a group

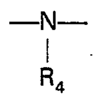

in which $R_4$ represents hydrogen or lower alkyl; or $R_1$ and $R_2$ may, together with the nitrogen atom to which they are attached form a heterocyclic ring which may contain the hetero functions —O— and

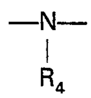

$R_3$ represents hydrogen, lower alkyl, alkenyl or alkoxyalkyl;

X represents —O—, —S— or —$CH_2$— or

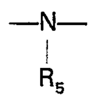

where $R_5$ is hydrogen or lower alkyl;

Y represents =S, =O, =$NR_6$ or =$CHR_7$;

in which $R_6$ represents hydrogen, nitro, cyano, lower alkyl, aryl, arylsulphonyl or lower alkylsulphonyl; $R_7$ represents nitro, lower alkylsulphonyl or arylsulphonyl; m is an integer from 2 to 4 inclusive; n is zero, 1 or 2; and Alk denotes a straight or branched alkylene chain of 1 to 6 carbon atoms.

The present invention provides compounds of the general formula I

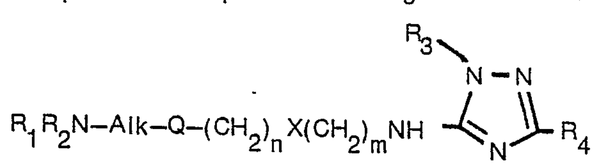

(I)

and physiologically acceptable salts and hydrates thereof, in which

$R_1$ represents hydrogen or $C_{1-4}$ alkyl and $R_2$ represents $C_{1-10}$ alkyl, $C_{3-8}$ cycloalkyl, $C_{3-6}$ alkenyl, phenyl $C_{1-6}$ alkyl (where the phenyl group may be optionally substituted by one or more $C_{1-3}$ alkyl groups, $C_{1-3}$ alkoxy groups or halogen atoms e.g. fluorine), trifluoro $C_{1-6}$ alkyl, or $C_{1-6}$ alkyl substituted by hydroxy, $C_{1-6}$ alkoxy, amino, $C_{1-6}$ alkylamino or di $C_{1-6}$ alkylamino or $R_1$ and $R_2$ may together with the nitrogen atom to which they are attached form a 5 to 10 membered ring which may be saturated or may contain at least one double bond, may be unsubstituted or may be substituted by one or more $C_{1-3}$ alkyl groups or a hydroxy group and/or may contain another heteroatom which is oxygen or sulphur;

Alk represents a straight or branched alkylene chain of 1 to 6 carbon atoms, preferably 1 to 4 carbon atoms;

Q represents a furan or thiophene ring in which incorporation into the rest of the molecule is through bonds at the 2- and 5-positions, the furan ring optionally bearing a further substituent $R_6$ adjacent to the group $R_1R_2N$-Alk, or Q represents a benzene ring in which incorporation into the rest of the molecule is through bonds at the 1- and 3- or 1- and 4-positions;

$R_6$ represents halogen or $C_{1-4}$ alkyl which may be substituted by hydroxy or $C_{1-4}$ alkoxy;

X represents $-CH_2-$, $-O-$, $-S-$ or

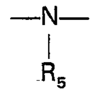

where $R_5$ represents hydrogen;

n represents zero, 1 or 2,

m represents 2, 3 or 4;

$R_3$ represents hydrogen, $C_{1-6}$ alkyl, $C_{3-6}$ alkenyl, phenyl $C_{1-6}$ alkyl (where the phenyl groups may be optionally substituted by one or more $C_{1-3}$ alkyl groups, $C_{1-3}$ alkoxy groups or halogen atoms e.g. fluorine), hydroxy $C_{2-6}$ alkyl or $C_{1-6}$ alkoxy $C_{1-6}$ alkyl; and

$R_4$ represents hydrogen, $C_{1-6}$ alkyl, $C_{3-6}$ alkenyl, phenyl $C_{1-6}$ alkyl (where the phenyl group may be optionally substituted by one or more $C_{1-3}$ alkyl groups, $C_{1-3}$ alkoxy groups or halogen atoms e.g. fluorine), hydroxy $C_{1-6}$ alkyl, acyloxy $C_{1-6}$ alkyl (where the acyl portion is benzoyl, phenyl $C_{2-7}$ alkanoyl or $C_{1-6}$ alkanoyl, in the first two cases the phenyl ring being optionally substituted by one or more $C_{1-3}$ alkyl groups, $C_{1-3}$ alkoxy groups or halogen atoms e.g. fluorine), $C_{1-6}$ alkoxy $C_{1-6}$ alkyl, phenoxy $C_{1-6}$ alkyl (where the phenyl group may be optionally substituted by one or more $C_{1-3}$ alkyl groups, $C_{1-3}$ alkoxy groups or halogen atoms e.g. fluorine), phenyl $C_{1-6}$ alkyloxy $C_{1-6}$ alkyl (where the phenyl group may be optionally substituted by one or more $C_{1-3}$ alkyl groups, $C_{1-3}$ alkoxy groups or halogen atoms e.g. fluorine), amino $C_{1-6}$ alkyl, $C_{1-6}$ alkylamino $C_{1-6}$ alkyl, di $C_{1-6}$ alkylamino $C_{1-6}$ alkyl, hydroxy, $C_{1-6}$ alkoxy or $C_{1-6}$ alkylthio, or the group $NR_7R_8$ where

$R_7$ represents hydrogen or $C_{1-6}$ alkyl and

$R_8$ represents the group $COR_9$ where $R_9$ represents hydrogen, $C_{1-6}$ alkyl, phenyl or phenyl $C_{1-6}$ alkyl (where in both cases, the phenyl group may be optionally substituted by one or more $C_{1-3}$ alkyl groups, $C_{1-3}$ alkoxy groups or halogen atoms e.g. fluorine), $C_{1-6}$ alkoxy, a 5 or 6 membered monocyclic heteroaryl group containing one or more heteroatoms selected from oxygen, nitrogen and sulphur, or a heteroaryl $C_{1-6}$ alkyl group where the heteroaryl group is a 5 or 6 membered monocyclic ring containing one or more heteroatoms selected from oxygen, nitrogen and sulphur, or

$R_8$ represents the group $SO_2R_{10}$ where $R_{10}$ represents $C_{1-6}$ alkyl or phenyl (optionally substituted by one or more $C_{1-3}$ alkyl groups, $C_{1-3}$ alkoxy groups or halogen atoms e.g. fluorine), or $R_8$ represents the group

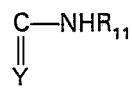

where the Y is oxygen or sulphur and $R_{11}$ represents hydrogen, $C_{1-6}$ alkyl, $C_{3-8}$ cycloalkyl, phenyl or phenyl $C_{1-6}$ alkyl (where, in both cases, the phenyl group may be optionally substituted by one or more $C_{1-3}$ alkyl groups, $C_{1-3}$ alkoxy groups or halogen atoms e.g. fluorine).

When X represents an oxygen atom or

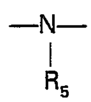

and n is zero, then Q preferably represents benzene.

The term "alkyl" as a group or part of a group means that the group is straight or branched and, unless otherwise stated, has 1 to 6 carbon atoms, and preferably 1 to 4 carbon atoms, e.g. methyl or ethyl, and the term "alkenyl" means that the group has 3 to 6 carbon atoms. The term "cycloalkyl" means that the group has 3 to 8 carbon atoms. The acyl portion of an acyloxyalkyl group means a benzoyl, phenyl $C_{2-7}$ alkanoyl or $C_{1-6}$ alkanoyl group, in the first two cases the phenyl ring being

optionally substituted by one or more $C_{1-3}$ alkyl groups, $C_{1-3}$ alkoxy groups or halogen atoms. Examples of acyloxyalkyl groups include acetoxymethyl, formyloxymethyl benzoyloxymethyl and phenylacetoxymethyl. The term "heteroaryl" means a 5 or 6 membered monocyclic ring which may contain 1 or more heteroatoms selected from oxygen, nitrogen and sulphur, e.g. furyl, pyridyl, thiazolyl or thienyl.

According to one aspect the invention provides compounds according to formula (I) and physiologically acceptable salts and hydrates thereof, in which

$R_4$ represents the group $NR_7R_8$ where $R_7$ and $R_8$ are as defined in formula (I) except that $R_9$ is other than a heteroaryl or heteroarylalkyl group and $R_{11}$ is other than $C_{3-8}$ cycloalkyl; or more preferably $R_4$ represents hydrogen, $C_{1-6}$ alkyl, $C_{3-6}$ alkenyl, phenyl $C_{1-6}$ alkyl (where the phenyl group may be optionally substituted by one or more $C_{1-3}$ alkyl groups, $C_{1-3}$ alkoxy groups or halogen atoms), hydroxy $C_{1-6}$ alkyl, $C_{1-6}$ alkoxy $C_{1-6}$ alkyl, phenoxy $C_{1-6}$ alkyl (where the phenyl group may be optionally substituted by one or more $C_{1-3}$ alkyl groups, $C_{1-3}$ alkoxy groups or halogen atoms), amino $C_{1-6}$ alkyl, $C_{1-6}$ alkylamino $C_{1-6}$ alkyl, di-$C_{1-6}$ alkylamino $C_{1-6}$ alkyl, hydroxy or $C_{1-6}$ alkoxy; provided that when X represents an oxygen atom or $-NR_5-$ and when n is zero then Q represents benzene.

The invention includes the compounds of formula (I) in the form of physiologically acceptable salts with inorganic and organic acids. Particularly useful salts include hydrochlorides, hydrobromides, sulphates, methanesulphonates, acetates, maleates, succinates, citrates, tartrates, fumarates and benzoates. The compounds of formula (I) and their salts may also form hydrates, which hydrates are also to be considered as part of the invention. The compounds of formula (I) can exhibit tautomerism and the formula is intended to cover all tautomers. Where optical isomers may exist the formula is intended to cover all diastereoisomers and optical enantiomers.

The compounds according to the invention, preferably in the form of a salt, may be formulated for administration in any convenient way and the invention includes within its scope pharmaceutical compositions containing at least one compound according to the invention adapted for use in human or veterinary medicine. Such compositions may be formulated in a conventional manner using one or more pharmaceutically acceptable carriers or excipients. Such compositions may also contain if required other active ingredients e.g. $H_1$-antagonists.

Thus the compounds according to the invention may be formulated for oral, buccal, topical, parenteral or rectal administration. Oral administration is preferred.

For oral administration, the pharmaceutical composition may take the form of for example, tablets, capsules, powders, solutions, syrups or suspensions prepared by conventional means with acceptable excipients. For buccal administration the composition may take the form of tablets or lozenges formulated in conventional manner.

The compounds of the invention may be formulated for parenteral administration by bolus injection or continuous infusion. Formulations for injection may be presented in unit dosage form in ampoules, or in multi-dose containers, with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for reconstitution with a suitable vehicle e.g. sterile pyrogen-free water before use.

The compounds of the invention may also be formulated in rectal compositions such as suppositories or retention enemas, e.g. containing conventional suppository bases such as cocoa butter or other glyceride.

For topical application, the compounds of the invention may be formulated as ointments, creams, gels, lotions, powders or sprays in a conventional manner.

For internal administration a convenient daily dosage regime of the compounds according to the invention would be 1 to 4 doses to the total of some 5 mg to 1 g per day, preferably 5 to 250 mg per day, dependant upon the condition of the patient.

In the compounds according to the invention, preferably the total of $m + n$ is 3 or 4.

Preferably Q is benzene incorporated into the rest of the molecule through bonds at the 1- and 3-positions. In the case where Q is benzene, preferably n is zero, X is oxygen and m is 3 or 4. If Q is furan, substituted furan or thiophen, preferably n is 1, X is sulphur and m is 2.

Preferably $R_1$ represents hydrogen or $C_{1-4}$ alkyl and $R_2$ represents $C_{3-5}$ alkenyl, $C_{5-7}$ cycloalkyl, benzyl, $C_{1-8}$ alkyl or $C_{1-4}$ alkyl substituted by $C_{1-3}$ alkoxy, hydroxy, di-$C_{1-3}$ alkylamino or trifluoromethyl or $R_1$ and $R_2$ together with the nitrogen atom to which they are attached form a ring with 5 to 8 members and optionally containing one double bond and/or substituted by hydroxy or one or two $C_{1-3}$ alkyl group(s) e.g. methyl. More preferably $R_1$ and $R_2$ are $C_{1-3}$ alkyl, e.g. methyl or $R_1$ and $R_2$ together with the nitrogen atom to which they are attached form a heterocyclic ring which is pyrrolidine, piperidine optionally substituted in the 4- position by $C_{1-3}$ alkyl e.g. methyl or hydroxy, tetrahydropyridine, morpholine, 2,6-dialkylmorpholine, e.g. 2,6-dimethylmorpholine, hexamethyleneimine or heptamethylenimine. Most preferably the heterocyclic ring is piperidine.

Preferably $R_3$ represents hydrogen, $C_{1-6}$ alkyl or $C_{2-4}$ hydroxyalkyl. More preferably $R_3$ represents methyl.

Preferably $R_4$ represents hydrogen, $C_{1-6}$ alkyl, hydroxy $C_{1-6}$ alkyl, $C_{1-6}$ alkoxy $C_{1-6}$ alkyl, acyloxy $C_{1-6}$ alkyl, phenyl $C_{1-6}$ alkyl (where the phenyl group may be optionally substituted by one or more $C_{1-3}$ alkyl groups, $C_{1-3}$ alkoxy groups or halogen atoms) or hydroxy or the group $NHCOR_9$ where $R_9$

represents hydrogen, $C_{1-6}$ alkyl, $C_{1-6}$ alkoxy, phenyl (optionally substituted by one or more $C_{1-3}$ alkyl groups, $C_{1-3}$ alkoxy groups or halogen atoms) or a 5 or 6 membered monocyclic heteroaryl group containing one or more heteroatoms selected from oxygen, nitrogen and sulphur or the group $NHCONHR_{11}$ where $R_{11}$ represents $C_{1-6}$ alkyl, phenyl (optionally substituted by one or more $C_{1-3}$ alkyl groups, $C_{1-3}$ alkoxy groups or halogen atoms) or $C_{3-8}$ cycloalkyl. More preferably $R_4$ is hydroxy $C_{1-6}$ alkyl, e.g. hydroxymethyl benzyl, hydroxy, $C_{1-6}$ alkoxy $C_{1-6}$ alkyl, e.g. methoxymethyl or the group $NHCOR_9$ where $R_9$ is hydrogen, phenyl, $C_{1-6}$ alkyl e.g. methyl or $C_{1-6}$ alkoxy e.g. ethoxy; or the group $NHCONHR_{11}$ where $R_{11}$ is phenyl. Most preferably $R_4$ is hydroxymethyl, formylamino or acetylamino.

Preferably Alk is $CH_2$.

A particularly preferred group of compounds of formula (1) are those of formula (11)

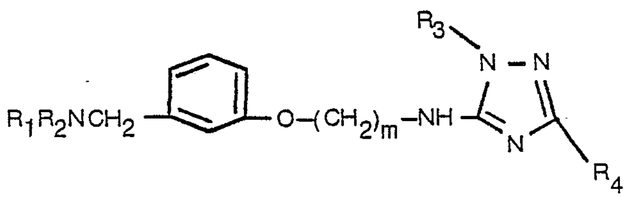

(II)

where $R_1$ and $R_2$ are methyl groups or together with the nitrogen atom to which they are attached form a pyrrolidino, piperidino or hexamethyleneimino group; m is 3 or 4, $R_3$ is hydrogen or methyl; and $R_4$ is a hydroxy $C_{1-6}$ alkyl, $C_{1-6}$ alkoxy $C_{1-6}$ alkyl, benzyl, formamido, $C_{2-7}$ alkanoylamino, $C_{1-6}$ alkoxycarbonyl-amino, hydroxy, benzoylamino or phenylcarbamoylamino group.

Particularly preferred compounds are

(1) 1-methyl-5-[[4-[3-(1-piperidinylmethyl)phenoxy]butyl]amino]-1H-1,2,4-triazole-3-methanol
(2) 1-methyl-5-[[3-[3-(1-piperidinylmethyl)phenoxy]propyl]amino]-1H-1,2,4-triazole-3-methanol
(3) N-[1-methyl-5-[[3-[3-(1-piperidinylmethyl)phenoxy]propyl]amino]-1H-1,2,4-triazol-3-yl] formamide
(4) 3-methoxy-methyl-1-methyl-5-[[4-[3-(1-piperidinylmethyl)phenoxy]butyl]amino]-1H-1,2,4-triazole
(5) 3-phenylmethyl-N-[3-[3-(dimethylaminomethyl)phenoxy]propyl]-1H-1,2,4-triazole-5-amine
(6) 5-[4-[3-(1-piperidinylmethyl)phenoxy]butyl]amino-1H-1,2,4-triazole-3-methanol
(7) 3-phenylmethyl-N-[3-[3-(1-piperidinylmethyl)phenoxy]propyl]-1H-1,2,4-triazole-5-amine
(8) Ethyl 1-methyl-5-[[3-[3-[(dimethylamino)methyl]phenoxy]propyl]amino]-1H-1,2,4-triazole-3-carbamate
(9) N-[5-[[3-[3-[(dimethylamino)methyl]phenoxy]propyl]amino]-1-methyl-1H-1,2,4-triazole-3-yl]benzamide
(10) N-[5-[[3-[3-(1-piperidinylmethyl)phenoxy]propyl]amino]-1-methyl-1H-1,2,4-triazole-3-yl]-N′-phenylurea
(11) 5-[[3-[3-(1-piperidinylmethyl)phenoxy]propyl]amino]-1H-1,2,4-1,2,4-triazole-3-one
and their physiologically acceptable salts.

Of the above mentioned compounds, compounds Nos. (1), (2), (3) and (4) and their salts are particularly preferred.

It will be appreciated that in the methods for the preparation of compounds of formula (I) given below, for certain reaction steps it may be necessary to protect various reactive substituents in the starting materials for a particular reaction and subsequently to remove the protecting group. Such protection and subsequent deprotection may be particualrly pertinent where $R_1$ and/or $R_2$ in intermediates used to prepare compounds of formula (1) are hydrogen atoms and/or when $R_3$ in intermediates is an alkyl group bearing a hydroxy substituent and/or when $R_4$ in certain intermediates is an alkyl group bearing a hydroxyl or a primary or secondary amino substituent. Standard protection and deprotection procedures can be employed, for example formation of phthalimide (in the case of primary amines), benzyl, benzyloxycarbonyl or trichloroethoxycarbonyl derivatives. Subsequent cleavage of the protecting group is achieved by conventional procedures. Thus a phthalimide group may be cleaved by treatment with a hydrazine e.g. hydrazine hydrate or a primary amine, for example methylamine; benzyl or benzyloxycarbonyl derivatives may be cleaved by hydrogenolysis in the presence of a catalyst, e.g. palladium, and trichloroethoxycarbonyl derivatives may be cleaved by treatment with zinc dust.

In describing the processes which may be used for preparing the compounds of formula (1) or intermediates useful in the preparation thereof, any of $R_1$ to $R_{11}$, Alk, Q, X, Y, n and m in the various formulae are as defined in formula (1) unless otherwise stated.

Compounds according to formula (I) in which $R_4$ represents a hydrogen atom may be prepared from the corresponding diazonium salt (III)

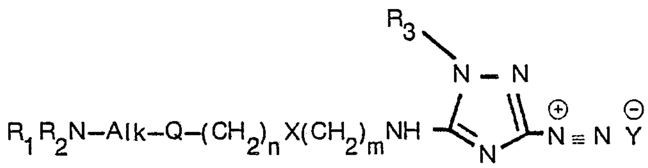

(III)

in which $R_1$, $R_2$ and $R_3$ are as defined in formula (I) or are groups convertible thereto and $\overset{\ominus}{Y}$ is the anion corresponding to the acid in the diazotisation reaction. Thus treatment of the diazonium salt (III) with a concentrated mineral acid such as sulphuric acid or hypophosphorous acid in a solvent such as ethanol gives the compound of formula (I) in which $R_4$ represents hydrogen.

Compounds of formula (I) in which $R_4$ is the group $NR_7R_8$ may be prepared by treating an amino triazole (IV)

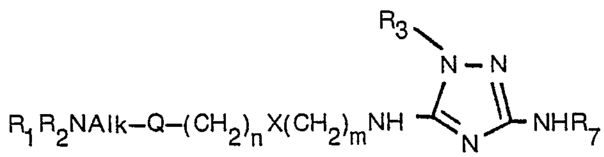

(IV)

in which $R_1$, $R_2$, $R_3$ and $R_7$ are as defined in formula (I) or are groups readily convertible thereto with a reagent capable of replacing the hydrogen atom in the group $NHR_7$ by the group $R_8$ where $R_8$ is as defined in formula (I).

Thus the aminotriazole (IV) may be reacted with an activated derivative of either a carboxylic acid $R_9COOH$ or a sulphonic acid $R_{10}SO_3H$ or the aminotriazole (IV) may be reacted with an isocyanate or isothiocyanate $R'_{11}NCY$ in which $R'_{11}$ has any of the meanings defined for $R_{11}$ in formula (I) except hydrogen or represents an alkali metal atom such as potassium or sodium or an alkoxycarbonyl group, e.g. ethoxycarbonyl, to give a compound of formula (I) in which $R_8$ is respectively the group $COR_9$, $SO_2R_{10}$ or

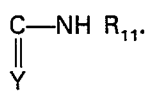

Suitable activated derivatives include acid halides e.g. acid chlorides, alkylchloroformates, acid anhydrides including mixed anhydrides (e.g. acetic formic anhydride), esters such as alkyl esters, ortho esters and (1-alkyl-2-pyridinyl) esters, or derivatives formed from a coupling agent such as carbonyldiimidazole or a carbodiimide such as dicyclohexylcarbodiimide.

The reaction with an acid halide is preferably carried out in the presence of a base e.g. an inorganic base such as sodium hydroxide or an organic base such as triethylamine or pyridine. The reaction with an alkylchloroformate is preferably carried out in the presence of a base, e.g. potassium carbonate or triethylamine, in a solvent such as dimethylformamide. The reaction with an acid anhydride may be carried out in the absence or presence of solvent such as pyridine.

Formylation may be effected by heating the aminotriazole (IV) in dimethylformamide in the presence of a base, e.g. sodium hydride.

In the reaction with an isocyanate or isothiocyanate compounds of formula (I) in which $R_{11}$ is other than hydrogen are conveniently prepared by carrying out the reaction in a solvent such as acetonitrile at an elevated temperature, e.g. reflux. Compounds of formula (I) in which $R_{11}$ is hydrogen may be prepared by heating the aminotriazole (IV) with an appropriate organic isocyanate or isothiocyanate such as ethylcarbonisothiocyanatidate, at an elevated temperature followed by hydrolysis of the resulting ester, for example with a base such as aqueous ethanolic sodium hydroxide.

Compounds of formula (I) in which $R_4$ is other than an alkoxy, alkylthio or acyloxyalkyl group may be prepared by cyclisation of a compound of formula (V)

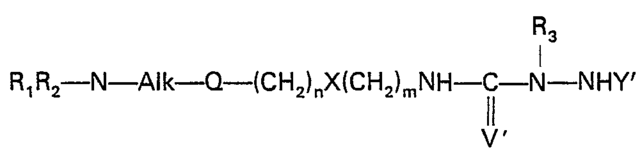

(V)

in which V' is

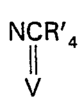

and Y' is hydrogen where V is oxygen or sulphur and $R_4'$ is a group as defined for $R_4$ or a group convertible thereto under the conditions of the cyclisation reaction or represents halogen or alkoxy; or V' is NH and Y' is

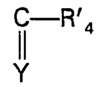

where Y is sulphur, oxygen or NH except that when $R_4'$ is halogen or alkoxy Y cannot be NH; or V' is sulphur or oxygen and Y' is

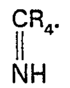

The above process is particularly suitable for the production of compounds of formula (I) in which $R_4$ is other than $NR_7R_8$. It should be noted that when V' is sulphur in compound (V) this compound is tautomeric with the thiol (VI).

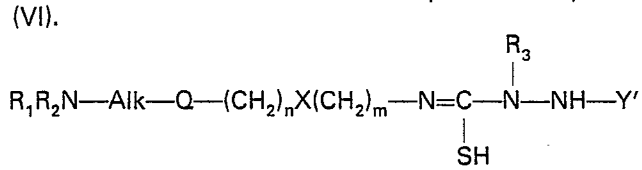
(VI)

and the corresponding S-alkyl, e.g. S-methyl, derivatives may also be used in the cyclisation.

Thus for example compounds according to formula (I) in which $R_4$ is other than an alkoxy, alkylthio, or acyloxyalkyl group may be prepared by thermal cyclisation of a compound of formula (VII)

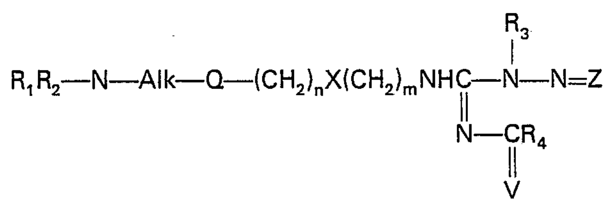
(VII)

where V represents sulphur or more preferably oxygen and Z represents two hydrogen atoms, in the absence or presence of a solvent, e.g. acetone or water.

It may be convenient to prepare compounds of formula (VII) in which Z represents two hydrogen atoms *in situ* by treating a compound of formula (VII) where Z represents a divalent protecting group which can readily be removed to yield two hydrogen atoms, for example a phenylmethylene group, with an acid, e.g. hydrochloric acid, preferably with heating and under such conditions cyclisation to give compounds of formula (I) will normally occur.

In a method for preparing the intermediate (VII) a compound of formula (VIII)

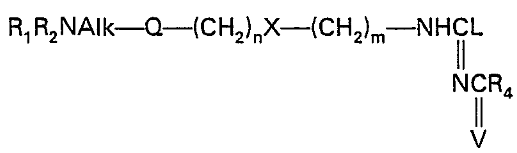
(VIII)

where V is oxygen or sulphur and L is a leaving group such as alkylthio may be reacted with hydrazine $R_3NHN{=}Z$ where Z is as defined above, followed by removal of the protecting group where necessary. Compounds of formula (VIII) may be prepared by treating a diamine of formula (IX)

$$R_1R_2NAlk—Q—(CH_2)_nX(CH_2)_m—NH_2 \qquad (IX)$$

with a compound of formula (X)

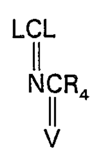
(X)

where L and V are as defined, above.

As a further possibility the diamine of formula (IX) may be reacted with a compound of formula (XI)

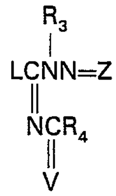

(XI)

where L, V and Z are as defined above, in the absence or presence of a solvent such as acetone or acetonitrile at a temperature of from room temperature to 70°C. Subsequent removal of the protecting group where appropriate then gives the intermediate (VII).

Compounds of formula (XI) may be prepared by acylating a compound of formula (XII)

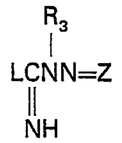

(XII)

with for example an acid chloride $R_4COCl$. The compound of formula (XII) may be prepared by treating a compound

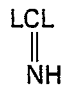

with the appropriate hydrazine $R_3NHN{=}Z$.

When preparing compounds of formula (I) in which $R_4$ is a hydroxyalkyl group it is preferable that in the intermediates (VII), (VIII) and (XI) the hydroxy group is in protected form, e.g. as an acyloxy derivative such as an alkanoyloxy or aroyloxy derivative. The protecting group will normally be removed during the cyclisation process.

In a further embodiment of the cyclisation of compounds of formula (V) compounds of formula (I) in which $R_4$ is other than an alkoxy, alkylthio or acyloxyalkyl may also be prepared by cyclisation of a compound of formula (XIII)

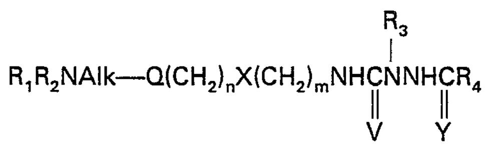

(XIII)

where V is NH and Y is sulphur, oxygen or NH, or V is sulphur or oxygen and Y is NH. The reaction is preferably carried out by heating the compound (XIII) in a suitable solvent such as acetonitrile or dimethylformamide.

In general intermediates of formula (XIII) may be prepared by methods analogous to those described in British Patent Specification No. 2023133. A.

In a particularly convenient embodiment of the above process an intermediate of formula (XIII) in which V is NH and Y is oxygen may be prepared in situ by the reaction of an aminoguanidine (XIV)

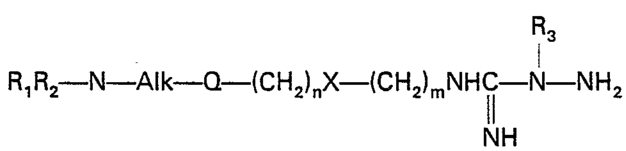

(XIV)

with an acid $R_4$ COOH or with an activated derivative thereof as defined above.

The acid and the amino guanidine (XIV) may be heated together, under which conditions cyclisation of the intermediate (XIII) takes place directly to give a compound of formula (I). In the case of an activated derivative an aprotic solvent, e.g. tetrahydrofuran may be used at temperatures from ambient to reflux. When using an acyl chloride as the activated derivative the reaction may also be carried out in the presence of a base e.g. potassium hydroxide.

Compounds of formula (I) may be prepared by reducing a compound of formula (XV).

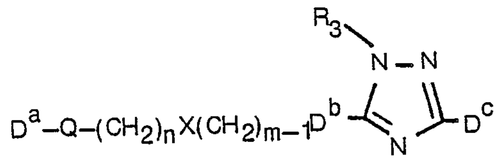

(XV)

in which at least one of $D^a$, $D^b$ and $D^c$ represents a reducible group and the other(s) take the appropriate meaning corresponding to formula (I).

Thus $D^a$ may represent $R_1R_2NAlk$ or a group convertible thereto under reducing conditions;

$D^b$ represents $-CH_2NH-$, CONH— or —CH=N—; and

$D^c$ represents $R_4$ or a group convertible thereto under reducing conditions.

Examples of the type of group that may be reduced are an amide, imide, imine, ester, aldehyde, ketone or nitrile group. Examples of the type of group $D^a$ which may be converted into the group $R_1R_2NAlk$ under reducing conditions are: an amide grouping $R_1R_2NCO(CH_2)_p$ where p is 0, 1, 2, 3, 4 or 5 or $R_1{}^a$ $CONR_2$ Alk — where $R_1{}^a$ CO represents a group which is reducible into $R_1$; an aralkylidenaminoalkyl grouping; a nitrile group $NC(CH_2)_p$ where p is 0, 1, 2, 3, 4 or 5; or an aldehyde group. When $D^a$ is an aldehyde group the conversion into $R_1R_2NAlk$ under reducing conditions is carried out in the presence of an appropriate amine $R_1R_2NH$, the reaction proceeding by reductive alkylation.

Examples of the group $D^c$ which may be converted into $R_4$ under reducing conditions are: an amide grouping $-(CH_2)_{q-1}CO\ NR_aR_c$ where $R_a$ represents hydrogen, alkyl, or aralkyl, $R_c$ represents hydrogen or alkyl and q is an integer from 1 to 6 inclusive with the possibility that the alkylene chain $(CH_2)_{q-1}$ may be straight or branched, or ann amide grouping $-(CH_2)_qNR_aCOR_b'$ where $R_a$ is as defined above, $R_b$ is alkyl and $COR_b'$ is a group which is reducible into $R_b$ and q is as defined above with the possibility that the alkylene chain $(CH_2)_q$ may be straight or branched; a nitrile group $-(CH_2)_{q-1}CN$; an adehyde group $-(CH_2)_{q-1}CHO$; an ester group $-(CH_2)_{q-1}CO_2R_{12}$ where q is as defined above and $R_{12}$ is an alkyl group, or a ketone group $-(CH_2)_rCOR_{12}$ where r is 0 to 4 and $R_{12}$ is an alkyl group and the total number of carbon atoms in $(CH_2)_r$ and $R_{12}$ is not more than 5.

$D^a$ and/or $D^c$ may also represent an aminoalkyl group, in which case the compound of formula (XV) is reacted with an appropriate aldehyde or ketone under reducing conditions to give the group $R_1R_2NAlk$— and/or $R_4$, the reaction proceeding by reductive alkylation.

A variety of reducing agents may be used in the above process.

Thus amides, imides, imines, esters, aldehydes, ketones and nitriles may conveniently be reduced using for example lithium aluminium hydride or aluminium hydride in a solvent such as tetrahydrofuran, dioxan or diethyl ether. Imines, aldehydes and ketones may be reduced using an alkali or alkaline earth metal borohydride e.g. sodium borohydride or by treatment with hydrogen and a suitable metal catalyst such as platinum, palladium or Raney nickel, in a suitable solvent such as a alkanol e.g. methanol or ethanol. Imies may also be reduced using formic acid.

In the particular process involving reductive alkylation of an amine with an aldehyde or ketone the reaction is conveniently carried out without isolation of the intermediate which is subsequently reduced using for example sodium borohydride, or hydrogen in the presence of a suitable catalyst.

Thus in one embodiment of the reduction process compounds of formula (I) in which $R_4$ is other than the group $NR_7R_8$ or acyloxyalkyl may be prepared by reduction of an amide of formula (XV) in which

a) $D^a$ represents $R_1R_2NCO(CH_2)_p$ or $R_1^aCONR_2Alk$,
   $D^b$ represents $-CH_2NH-$ and
   $D^c$ represents $R_4$; or

b) $D^a$ represents $R_1R_2NAlk$,
   $D^b$ represents —CONH— and
   $D^c$ represents $R_4$; or

c) $D^a$ represents $R_1R_2NAlk$,
   $D^b$ represents $-CH_2NH-$ and
   $D^c$ represents $(CH_2)_{q-1}CONR_aR_c$ or $(CH_2)_qNR_aCOR_b'$;

where p, q, $R_1^a$ and $R_b'$ are as defined above, with a suitable reducing agent such as lithium aluminium hydride or an aluminium hydride in a solvent such as tetrahydrofuran, dioxan or diethylether.

A particularly useful amide of formula (XV) is that in which $D^a$ represents $R_1R_2NAlk$, $D^b$ represents —CONH— and $D^c$ represents $R_4$.

In a further embodiment of the reduction process a compound of formula (XV) in which $D^a$ or $D^c$ is a cyanoalkyl group $NH(CH_2)_q$ or $(CH_2)_{q-1}CN$ respectively may be reduced to a compound of formula (I) in which $R_1R_2NAlk$ is a primary aminoalkyl group or $R_4$ is a primary aminoalkyl group $(CH_2)_{q-1}CH_2NH_2$. Reduction may be effected using for example lithium aluminium hydride in a solvent such as diethyl ether or tetrahydrofuran.

In a further embodiment of the reduction process compounds of formula (I) may be prepared by reduction of an imine of formula (XV) in which

a) $D^a$ represents an aralkylidenaminoalkyl group, $D^b$ represents —$CH_2NH$— and $D^c$ represents $R_4$; or

b) $D^a$ represents $R_1R_2NAlk$, $D^b$ represents —CH=N— and $D^c$ represents $R_4$; with a suitable reducing agent such as a metal hydride, e.g. an alkali or alkaline earth metal borohydride, such as sodium borohydride, in a solvent such as an alkanol, e.g. methanol or ethanol, or aluminium hydride or lithium aluminium hydride in a solvent such as tetrahydrofuran or dioxan. The imine (XV) may also be reduced with hydrogen and a suitable metal catalyst such as platinum, in a solvent such as an alkanol e.g. methanol or ethanol.

A particularly useful imine of formula (XV) is that in which $D^a$ is $R_1R_2NAlk$, $D^b$ is —CH=N— and $D^c$ is $R_4$.

In the above process a borohydride reducing agent is preferably used when preparing compounds in which $R_4$ is the group $NR_7R_8$.

The process involving reductive alkylation of an amine with an aldehyde or ketone to give the compounds of formula (I), without isolation of any intermediate, is also part of this embodiment. Thus, for example compounds of formula (I) in which Alk represents $CH_2$ may be prepared from the compound (XV) in which $D^a$ represents CHO, $D^b$ represents —$CH_2NH$— and $D^c$ represents $R_4$, by reaction with ammonia or an amine $R_1R_2NH$ in a solvent, e.g. tetrahydrofuran or an alkanol such as ethanol or methanol, followed by reduction e.g. with a hydride reducing agent such as an alkali or alkaline earth metal borohydride, e.g. sodium borohydride, or aluminium hydride or lithium aluminium hydride or with hydrogen and a metal catalyst e.g. palladium or platinum.

In another embodiment of the reduction process, a compound of formula (I) in which $R_4$ represents a hydroxyalkyl group may be prepared from a compound of formula (XV) in which $D^c$ is a group that may be reduced to a hydroxyalkyl group, e.g. an ester, aldehyde, or ketone, $D^a$ is $R_1R_2NAlk$ and $D^b$ is —$CH_2NH$—. Thus a compound of formula (XV) in which $D^c$ has the meaning $(CH_2)_{q-1}CO_2R_{12}$ may be reduced using for example lithium aluminium hydride to give a compound of formula (I) in which $R_4$ is the group —$(CH_2)_{q-1}CH_2OH$. Compounds of formula (XV) in which $D^c$ has the meaning $(CH_2)_{q-1}CHO$ or $(CH_2)_rCOR_{12}$ may be reduced using for instance sodium borohydride or lithium aluminium hydride to give a compound of formula (I) in which $R_4$ is the group $(CH_2)_{q-1}CH_2OH$ or $(CH_2)_rCHOH\ R_{12}$ where q, r and $R_{12}$ are as defined above.

In certain instances it is convenient to reduce for example more than one of the groups $D^a$, $D^b$ and $D^c$ simultaneously. Thus for example compounds of formula (XV) in which $D^c$ represents $(CH_2)_{q-1}CO_2R_{12}$ or $(CH_2)_{q-1}CONR_aR_c$ and $D^b$ is the group —CONH— and $D^a$ is $R_1R_2NAlk$ may be reduced using for example lithium aluminium hydride to give compounds of formula (I) in which $R_4$ is the group $(CH_2)_qOH$ or $(CH_2)_qNR_aR_c$ respectively, where q, $R_a$ and $R_c$ are defined above.

Amides and imines of formula (XV) where $D^b$ is —CONH— or —CH=N—, $D^a$ is $R_1\ R_2NAlk$ and $D^c$ is $R_4$ or a group convertible thereto may be prepared by the reaction of an activated derivative of a carboxylic acid or aldehyde (XVI)

$$R_1R_2N—Alk—Q—(CH_2)_nX(CH_2)_{m-1}G \qquad (XVI)$$

where G is $CO_2H$ or CHO respectively with the appropriate amino triazole (XVII)

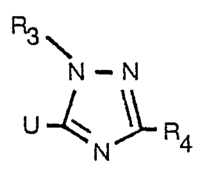

(XVII)

where U is $NH_2$ and $R_4$ is as defined in formula (I) or is a group convertible thereto, using methods analogous to those described in British Patent Specification No. 2023133 A.

Aldehydes and carboxylic acids of formula (XVI) may be prepared from an appropriate starting material (XVIII)

$$R_1R_2N—Alk—Q—(CH_2)_nXH \qquad (XVIII)$$

in which X is other than $CH_2$.

Thus for example a compound $R_1R_2NAlk—Q—OH$ may be treated with a haloalkyl nitrile Hal $(CH_2)_{m-1}CN$ in a solvent such as dimethylformamide and in the presence of a base e.g. sodium hydride to give the corresponding compound $R_1R_2NAlk—Q—O—(CH_2)_{m-1}CN$. Subsequent reduction using for example hydrogen in the presence of Raney Nickel affords the corresponding aldehyde which may conveniently be isolated as its semicarbazone. The desired aldehyde (XVI) may then be generated by treatment with hydrochloroic acid and aqueous formaldehyde.

Alternatively treatment of the compound $R_1R_2NAlk—Q—OH$ with an alkyl haloester Hal $(CH_2)_{m-1}CO_2Alk$ affords the ester $R_1R_2NAlk—Q—O(CH_2)_{m-1}CO_2Alk$, which may then be hydrolysed (e.g. by using potassium hydroxide in aqueous ethanol) to give the corresponding carboxylic acid (XVI).

The triazole (XVII) may be prepared by standard methods such as those described by F. Kurzer and L. E. A. Godfrey, Angew. Chem. International Edition *2*, 459—476 (1963) and K. T. Potts, Chem. Reviews *61*, 87 (1961).

The compounds of formula (XV) in which $D^a$ represents $R_1R_2NCO(CH_2)_p$ or CHO, $D^b$ represents $—CH_2NH$ and $D^c$ represents $R_4$ may be prepared from an amine of formula (XIX).

$$W—Q—(CH_2)_nX—(CH_2)_m—NH_2 \qquad (XIX)$$

in which W represents the group $R_1R_2CO(CH_2)_p$ or a protected aldehyde group e.g. a cyclic ketal such as an ethylene ketal, by methods analogous to those already described for preparing the corresponding compounds of formula (I).

The compounds of formula (XV) in which $D^a$ has the meaning $R_1^aCONR_2Alk—$ and/or $D^c$ has the meaning $(CH_2)_qNR_aCOR_b'$ may be prepared by treating the corresponding compounds in which $D^a$ is $HNR_2Alk—$ and/or $D^c$ is $(CH_2)_qNHR_a$ with an activated derivative of the appropriate acid $R_1^aCO_2H$ or $R_b'CO_2H$.

Compounds of formula (XV) in which $D^a$ is aralkylidenaminoalkyl may be prepared from the corresponding amine by standard procedures.

Compounds of formula (I) can be produced by reacting a compound of formula (XX)

$$R_1R_2NAlk—Q—E \qquad (XX)$$

in which E represents $(CH_3)_nX(CH_2)_mP$ or $CH_2P'$ where P and P′ are leaving groups, with a triazole of the formula (XVII)

where U represents amino, $HS(CH_2)_mNH$ or $HO(CH_2)_mNH$, and $R_4$ is as defined in formula (I) or is a group convertible thereto.

Thus for example compounds of formula (I) may be prepared by reacting a compound of formula (XX) in which E represents $(CH_2)_nX(CH_2)_mP$ and P represents a leaving group such as halogen, e.g. chlorine or bromine, *α* sulphonyloxy, e.g. mesyloxy or tosyloxy with the triazole (XVII) in which U represents an amino group, the reaction being carried out in a suitable solvent such as dimethylformamide or acetonitrile.

Compounds of formula (I) may also be prepared by introducing the group $R_1R_2NAlk—$ into the group Q present in a suitable intermediate or converting another group already present into a group $R_1R_2NAlk$. Thus compounds of formula (I) in which Alk represents a methylene group and Q represents a furan or substituted furan ring as defined in formula (I) may be prepared from a compound of formula (XXI)

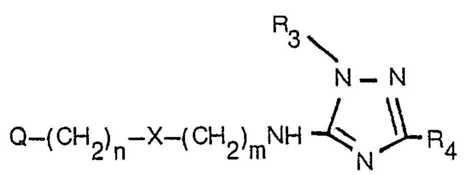

(XXI)

in which Q represents a furan or substituted furan ring as defined in formula (I), by means of a Mannich reaction with formaldehyde and an amine $R_1R_2NH$ or a salt thereof. The reaction may be carried out by reacting the amine salt with aqueous formaldehyde and the compound (XXI) or by refluxing the amine salt with paraformaldehyde and compound (XXI) in a suitable solvent such as ethanol.

Compounds of formula (I) in which $R_1$ and $R_2$ are both methyl, Alk is methylene and Q represents a thiophen ring, a furan ring or a substituted furan ring as defined in formula (I) may be prepared from compound (XXI) in which Q may additionally represent a thiophen ring, by reaction with a compound $(CH_3)_2\overset{\oplus}{N}=CH_2\overset{\ominus}{Cl}$ in a solvent such as acetonitrile at an elevated temperature, e.g. reflux. The compounds of formula (XXI) may be prepared by methods analogous to those already described for the compounds of formula (I).

Compounds of formula (I) in which $R_4$ is a secondaryor tertiary hydroxyalkyl group may be prepared by treating the corresponding compound where $R_4$ is the group $(CH_2)_{q-1}CHO$, $(CH_2)_{q-1}CO_2R_{12}$ or $(CH_2)_rCOR_{12}$ in which q, r and $R_{12}$ as defined previously with an organometallic derivative such as a Grignard reagent, e.g. a $C_{1-5}$ alkyl magnesium halide or an organolithium, e.g. alkyl lithium reagent in a suitable solvent such as diethyl ether or tetrahydrofuran.

Intermediates in preparing the compounds of formula (I) wherein $R_4$ is a group convertible into $R_4$ as defined in formula (I) include aldehydes, ketones, esters, amides and nitriles. Such intermediates may be prepared by methods analogous to those already described for preparing compounds of formulas (I).

Compounds of formula (I) may be prepared by reacting a compound of formula (XXII)

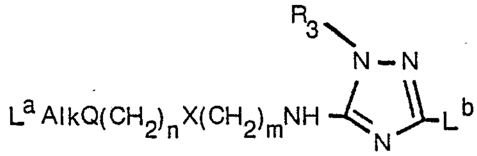

(XXII)

where either $L^a$ is $R_1R_2N$ and $L^b$ is the group $(CH_2)_q$ $L^c$ or $L^a$ is a group $L^d$ and $L^b$ is $R_4$ where $L^c$ and $L^d$ are leaving groups, with a compound capable of replacing $L^d$ by $R_1R_2N$ or $L^c$ so as to convert $L^b$ into the group $R_4$. Examples of the leaving group $L^d$ include halogen, e.g. chlorine or bromine or quaternary ammonium, e.g. trimethylammonium and examples of the leaving group $L^c$ include halogen, e.g. chlorine or bromine.

The leaving group $L^d$ may be displaced by treatment with an amine $R_1R_2NH$ to give a compound of formula (I). The leaving group $L^c$ may be displaced by treating the compound of formula (XXII) with either ammonia or a primary or secondary amine to give a compound of formula (I) in which $R_4$ is an aminoalkyl, alkylaminoalkyl or dialkylaminoalkyl group or with the anion of an appropriate alcohol or phenol, e.g. with alkoxide, to give a compound of formula (I) in which $R_4$ is an ether group, e.g. alkoxyalkyl.

The displacement of halide ions with ammonia or an amine may be carried out in the absence or presence of a solvent such as acetonitrile. Displacement with the anion of an appropriate alcohol or phenol may be effected by treatment with a suitable alkali metal derivative, e.g. sodium methoxide, in a suitable solvent e.g. dimethylformamide, or the alcohol corresponding to the anion. Displacement of a quaternary ammonium gorup may be effected by heating with an excess of the appropriate amine.

Compounds of formula (I) in which $R_4$ has a particular meaning may be converted into other compounds of the invention by standard methods of interconversion.

Thus a compound in which $R_4$ is an alkoxyalkyl group may be prepared by treating a corresponding compound of formula (I) in which $R_4$ is a hydroxyalkyl group with for example an alkyl halide in a suitable solvent such as dimethylformamide in the presence of a base e.g. sodium hydride.

According to a further possibility alkylation (e.g. methylation) of a compound in which $R_4$ is a primary or secondary aminoalkyl group may be effected by treatment with formaldehyde and formic acid (the Eschweiler-Clarke procedure).

Compounds of formula (I) in which $R_4$ is an acyloxyalkyl group may be prepared by treating the corresponding hydroxyalkyl compound with an appropriate acid, e.g. acetic or benzoic acid at elevated temperatures e.g. 80—120°C in the absence or presence of a solvent such as toluene.

Compounds of formula (I) in which $R_4$ is an alkenyl group may be prepared from the corresponding hydroxyalkyl compound by heating with an acid, e.g. toluene sulphonic acid, in a suitable solvent, e.g. acetonitrile.

Compounds of formula (I) in which $R_4$ represents a hydroxyalkyl group may be prepared by removal of a suitable hydroxyl protecting group. Examples of such protected hydroxyl groups include ethers, such as trialkylsilyl e.g. trimethyl silyl, aralkyl such as benzyl, benzhydryl or trityl, tetrahydro pyranyl or alkoxymethyl, e.g. methoxymethyl ethers, or esters of carboxylic acids such as alkanoic acids e.g. formic acid or acetic acid, or aromatic acids such as benzoic acid, or aralkanoic acids e.g. phenylacetic acid or haloalkanoic acids such as trifluoroacetic acid or trichloropropionic acid.

The protecting groups may be removed by conventional procedures c.f. Protective Groups in Organic Chemistry 1973 edited by J. F. W. McOmie. For example benzyl and benzhydryl ether groups may be removed by catalytic hydrogenolysis with for example hydrogen and a palladium catalyst; the trityl, tetrahydropyranyl, alkoxymethyl and trialkylsilyl ether groups may be removed by acid hydrolysis. The esters may be cleaved by acid or alkaline hydrolysis.

The diazonium salts (III), the aminotriazoles (IV), the aminoguanidines (XIV) and the compounds of formula (XX) in which E represents $(CH_2)_nX(CH_2)_mP$ may be prepared as described in British Patent Specification No. 2023133A.

Where the product of any of the above processes is a free base and a salt is required, the salt may be formed in a conventional manner. Thus for example, a generally convenient method of forming the salts is to mix appropriate quantities of the free base and the acid in an appropriate solvent(s) e.g. an alcohol such as ethanol or an ester such as ethyl acetate.

The invention is illustrated but not limited by the following Examples and preparations.

In the following examples temperatures are in °C "T.l.c." refers to thin layer chromatography carried out on silica using, unless otherwise stated, one of the following solvent systems:

| | |
|---|---|
| System A | methanol: 0.88 ammonia (79.1) |
| System B | ethyl acetate: water: isopropanol: 0.88 ammonia (25:8:15:2) |
| System C | ethyl acetate: ethanol: 0.88 ammonia (20:3:2) |

Preparative chromatography was carried out on silica using methanol as eluant unless otherwise stated.

Preparation 1

a) 4-[3-(1-piperidinylmethyl)phenoxy]butanal 4-[3-(1-piperidinylmethyl)phenoxy]butannitrile

A mixture of sodium hydride (1.5 g) and 3-(1-piperidinylmethyl)phenol (11.2 g) in dimethylformamide (60 ml) was stirred at room temperature for 5 h. 4-Bromobutannitrile (9.0 g) was added, and stirring was continued for a further 24 h. The mixture was poured onto ice and extracted with ethyl acetate which was washed with water and brine, and distilled to give the *title compound* as a colourless oil (14.78 g) b.p. 200°, 0.08 mm; tlc system A $R_f$ 0.8.

*4-[3-(1-piperidinylmethyl)phenoxy]butanal semi-carbazone*

A solution of 4-[3-(1-piperidinylmethyl)phenoxy]butannitrile (51.6 g) sodium acetate (73.8 g) and semi-carbazide hydrochloride (77.6 g) in 50% aqueous ethanol (1 L) was hydrogenated over Raney Nickel (50 g). The catalyst was removed by filtration. The filtrate was partially evaporated, made basic with potassium carbonate and extracted with ethyl acetate. The organic extract was evaporated to leave an oil which was purified by column chromatography to give the *title compound* as a pale yellow oil (48.6 g); tlc System B, $R_f$ 0.8.

N.M.R. ($CDCl_3$) 0.28, brs, (1H); 2.6, s, (1H); 2.9, t, (1H); 3—3.4, m, (3H); 4.4, brs, (2H); 6.02, t, (2H); 6.57, s, (2H); 7.4—7.8, m, (4H); 7.95, m, (2H); 8.2—8.9, m, (8H).

*4-[3-(1-piperidinylmethyl)phenoxy]butanal*

4-[3-(1-piperidinylmethyl)phenoxy]butanal semi-carbazone (9.43 g) and 37% formaldehyde (80 ml) in 2 N hydrochloric acid (80 ml) were stirred at room temperature for 2.5 h and then diluted with water. The solution was basified with sodium carbonate and extracted with ether. The organic extract was washed with brine, and distilled to give the *title compound* as a colourless oil (5.59 g) b.p. 200°, 0.06 mm; tlc methanol $R_f$ 0.7.

b) Similarly prepared from 3-[(dimethylamino)methyl]phenol (12.08 g) was 4-[3-[(dimethylamino)methyl]phenoxy]butanal (1.05 g) as a colourless oil b.p. 150° 0.06 mm; tlc System A, $R_f$ 0.59.

Preparation 2

4-[3-(1-piperidinylmethyl)phenoxy]butanoic acid Ethyl 4-[3-(1-piperidinylmethyl)phenoxy]-butanoate

A mixture of 3-(1-piperidinylmethyl)phenol (11.2 g) sodium hydride (1.5 g) and ethyl 4-bromobutyrate (11.7 g) in dimethylformamide (60 ml) was stirred at 25° during 20 h. The mixture was poured onto ice (300 g) and extracted with ethyl acetate (3 x 100 ml). The combined extracts were distilled to give the *title compound* as a colourless oil (16.9 g) b.p. 250°, 0.1 mm; tlc System B, $R_f$ 0.9.

*4-[3-(1-piperidinylmethyl)phenoxy]butanoic acid*

A solution of ethyl-4-[3-(1-piperidinylmethyl)phenoxy]butanoate (6.1 g) and potassium hydroxide (6.0 g) in ethanol (50 ml) and water (50 ml) was heated under reflux during 0.5 h. The cool solution was neutralised to pH 7 with 5 N hydrochloric acid, and evaporated to dryness under reduced pressure. The residue was dissolved in ethanol (100 ml), filtered and the filtrate was evaporated to leave a residue which was crystallised from a mixture of methanol and ether to give *the title compound* as a white crystalline solid (4.91 g) m.p. 73.5°; tlc System B, $R_f$ 0.3.

Preparation 3

Methyl 5-amino-1-methyl-1H-1,2,4-triazole-3-carboxylate

A stirred suspension of 5-amino-1-methyl-1H-1,2,4-triazole-3-carboxylic acid (9.10 g) in methanol (100 ml) was saturated with dry hydrogen chloride, and heated under reflux for 4.5 h. The cooled solution was partially evaporated, diluted with water (100 ml) and pH was adjusted to pH 7 with potassium carbonate. The solution was filtered, and the filtrate partially evaporated to give the *title compound* as a white solid (4.60 g) m.p. 190—1; tlc System B, $R_f$ 0.6.

Preparation 4

3-[3-(1-piperidinylmethyl)phenoxy]propanoic acid 3-[3-(1-piperidinylmethyl)phenoxy]-propannitrile

A solution of 3-(1-piperidinylmethyl)phenol (26 g), acrylonitrile (100 ml) and benzyltrimethylammonium hydroxide (40% methanolic solution, 5 ml) was heated at reflux for 40 h. The mixture was evaporated *in vacuo,* diluted with ether (300 ml) and filtered. The filtrate was washed with 2 N sodium hydroxide, water and distilled to give the *title compound* as a colourless oil (17.5 g) b.p. 170°, 0.07 mm; tlc system B, $R_f$ 0.8.

*3-[3-(1-piperidinylmethyl)phenoxy]propionic acid*

A solution of 3-[3-(1-piperidinylmethyl)phenoxy]propannitrile (1.5 g) in 2 N sulphuric acid (115 ml) was heated under reflux for 72 h. The pH of the cooled solution was adjusted to pH 7 and the suspension was treated with ethanol, filtered and evaporated to dryness. The resulting residue was

extracted with hot ethanol. Ether was added to the cooled extract to give the *title compound* as a white solid (12 g) m.p. 178.5—179.5°; tlc System B, $R_f$ 0.4.

Preparation 5

Methyl N-[2-(acetyloxy)acetyl]-1-methyl-2-(phenylmethylene)hydrazinecarboximidothioate

*Methyl 1-methyl-2-(phenylmethylene)hydrazinecarboximidothioate hydroiodide*

A mixture of dimethyl dithiocarbamate hydroiodide (10 g) and 1-methyl-2-(phenylmethylene) hydrazine (5.4 g) was heated at 60° under water pump pressure for 1 h. The residue was triturated with hot ethyl acetate (20 ml) to give the *title compound* (13.3 g) as a yellow solid, m.p. 183.4° tlc System A $R_f$ 0.75.

Methyl N-[2-(acetyloxy)acetyl]-1-methyl-2-(phenylmethylene)hydrazinecarboximidothioate

Methyl 1-methyl-2-(phenylmethylene)hydrazinecarboximidothioate, hydroiodide (1.34 g) was dissolved in saturated potassium carbonate solution and extracted with ether. The combined organic extracts were dried and evaporated to give an off-white solid which was dissolved in acetone (25 ml) and treated with potassium carbonate (1.1 g) followed by (actyloxy)acetyl chloride (0.85 g). The suspension was stirred at room temperature for 2 h and evaporated *in vacuo*. The residue was suspended in water (50 ml) and filtered to give the *title compound* (1.01 g) which was recrystallised from ethanol, m.p. 115—117°.

| | | | |
|---|---|---|---|
| Found: | C, 54.7; | H; 5.6; | N, 13.4% |
| $C_{14}H_{17}N_3O_3S$ requires: | C, 54.7; | H, 5.6; | N, 13.7% |

The following compounds were similarly prepared from the corresponding methyl-1-alkyl-2-(phenylmethylene) hydrazinecarboximidothioate hydroiodide (A) and the corresponding acid chloride.

b) A (3.4 g) and phenyl acetyl chloride (1.6 g) gave methyl-1-N-phenylacetyl-2-(phenylmethylene)hydrazinecarboximidothioate (1.3 g) m.p. 147—8°; tlc. silica; ethyl acetate $R_f$ 0.75.

Preparation 6

Following the method of Preparation 5, methyl 1 - methyl - 2 - (phenylmethylene)hydrazinecarboximidothioate (20 g) and ethyl malonyl chloride (12 g) gave ethyl 3-[[[1-methyl-2-(phenylmethylene)hydrazino](methylthio)methylene]amino]-3-oxopropanoate (5.6 g) m.p. 74—5°.

N.m.r. ($CDCl_3$) 2.2, s, (1H); 2.2—2.4, m, (2H); 2.5—2.7, m, (3H); 5.82, q, (2H); 6.49, s, (2H); 6.52, s, (3H); 7.6, s, (3H); 8.73, t, (3H).

Preparation 7

The following compound was prepared using the method of Example 16:

3 - [3 - (1 - piperidinylmethyl)phenoxy]propananamine (3.61 g) and ethyl 3 - [[[1 - methyl - 2 - (phenymethylene)hydrazino](methylthio)methylene]amino] - 3 - oxopropanoate (4.7 g) gave ethyl 3 - [[[1 - methyl - 2 - (phenylmethylene)hydrazino] [[3 - [3 - (1 - piperidinylmethyl)phenoxy]propyl]-amino]methylene]amino] - 3 - oxopropanoate (7.3 g)

N.m.r. ($CDCl_3$) 2.1, br, (1H); 2.23, s, (1H); 2.3—2.5, m, (2H); 2.55—2.7, m, (3H); 2.83, t, (1H), 3—3.3, (6H); 8.5, m, (3H); 5.8, q, (2H); 5.9, t, (2H); 6.4, m, (2H); 6.53, 2, (3H); 6.55, s, (2H); 6.68, s, (2H); 7.5—8, m, (6H); 8.7, t, (3H); t.l.c. System A Rf 0.65.

The above ethyl ester (1.84 g) was acidified to give ethyl 1 - methyl - 5 - [[3 - [3 - (1 - piperidinylmethyl)phenoxy]propyl]amino] - 1H - 1,2,4 - triazole - 3 - acetate (0.5 g).

N.m.r. ($CDCl_3$) 2.77, t, (1H); 3—3.3, m, (3H); 5.4, t, (1H); 5.8, q, (2H); 5.9, t, (2H); 6.3—6.6, 3xs + q, (9H); 7.6, m, (4H); 8.5, m, (6H); 8.2, t, (3H); T.l.c. System A Rf 0.63.

Preparation 8

5-[[3-(3-Formylphenoxy)propyl]amino]-1-methyl-1H-1,2,4-triazole-3-methanol

*N-[2-[(Acetyloxy)acetyl]-N'-[3-[3-(1,3-dioxolan-2-yl)phenoxy]propyl]-1-methyl-2-(phenylmethylene)-hydrazinecarboximidamide*

A mixture of 3 - [3 - (1,3 - dioxolan - 2 - yl)phenyoxy]propanamine (2.9 g) and methyl - N - [2-(acetyloxy)acetyl] - 1 - methyl - 2 - (phenylmethylene)hydrazinecarboximidothioate (4 g) was heated at 60° under water pump vacuum for 3h to give a glass which was triturated with ether to give the *title compound* as a white solid (5.39 g) m.p. 78—80.2 t.l.c. silica methanol $R_f$ 0.8.

5-[[3-(3-Formylphenoxy)propyl]amino]-1-methyl-1H-1,2,4-triazole-3-methanol

A suspension of N - [2 - [(acetyloxy)acetyl] - N' - [3 - [3 - (1,3 - dioxolan - 2 - yl)phenoxy]-propyl] - 1 - methyl - 2 - (phenylmethylene)hydrazinecarboximidamide (5 g) in 2 *N* hydrochloric acid (100 ml) and ethanol (25 ml) was stirred at room temperature for 16 h. The solution was neutralised to

pH 7 with potassium carbonate, treated with potassium hydroxide (1.7 g) diluted with ethanol (50 ml) and stirred for a further 15 min. The pH of the solution was adjusted to pH 7 with 2 *N* hdyrochloric acid, and the solvent was partially evaporated to leave an aqueous solution which was extracted with ethyl acetate. The combined organic extracts were dried and evaporated to give the *title compound* as a foam (2.63 g) t.l.c. silica ethyl acetate:methanol: 2:1 $R_f$0.5.

N.m.r. ($CDCl_3$) 0.00, s, (1H); 2.5—2.9, m, (4H); 5.4, t, (1H); 6.4, q, (2H); 6.50, s, (3H); 7.85, m, (2H).

Example 1

a) 3-methyl-N-[3-[3-(N,N-dimethylaminomethyl)phenoxy]propyl]-1H-1,2,4-triazole-5-amine

A mixture of 3-(3-aminopropoxy)-N,N-dimethylbenzenemethanamine (A) (2.0 g) and methylisothio semicarbazide hydroiodide (2.24 g) was heated at 40° for 6h. The residual gum was dissolved in ethanol and treated with a solution of tartaric acid in ethyl acetate. The solid that precipitated was filtered off and heated under reflux in glacial acetic acid (15 ml) for 4 h. The mixture was cooled, basified with potassium carbonate and extracted with ether. The organic extract was purified by column chromatography to give the *title compound* after recrystallization from a mixture of ethyl acetate and light petroleum (b.p. 60—80°) as a buff solid (0.8 g) m.p. 133—133.5°. tlc System A, $R_f$ 0.58.

The following were similarly prepared from the appropriate acid, the appropriate diamine and methylisothiosemicarbazide hydroiodide (B).

b) Diamine A (1.46 g), B (2.75 g) and phenyl acetic acid (3.0 g) gave 3-phenylmethyl-N-[3-[3-[(dimethylamino)methyl]phenoxy)-1H-1,2,4-triazole-5-amine (0.06 g) m.p. 140—141°. tlc system A, $R_f$ 0.49.

c) 3-[3-(1-piperidinylmethyl)phenoxy]propanamine (4.96 g), B(4,94 g) and phenyl acetic acid (8.16 g) gave 3-phenylmethyl-N-[3-[3-(1-piperidinylmethyl) phenoxy]propyl]-1H-1,2,4-triazole-5-amine (1.3 g) m.p. 143—4° tlc System A, $R_f$ 0.5.

Example 2

a) 1-methyl-N-[3-[3-(N,N-dimethylaminomethyl)phenoxy]propyl]-1H-1,2,4-triazole-5-amine

A solution of sodium nitrite (1.74 g) in water (15 ml) was added dropwise to a solution of 1-methyl-$N^5$-[3-[3-(N,N-dimethylaminomethyl)phenoxy]propyl]-1H-1,2,4-triazole-3,5-diamine (5 g) in cor centrated sulphuric acid (3.68 g) and ethanol (200 ml). The reaction mixture was heated at 65° for 4 h, cooled, treated with saturated brine (100 ml) and extracted with ethyl acetate. Evaporation of the organic extract gave a red oil which was extracted with ether. Distillation of the ether extracts gave the *title compound* as a pale yellow oil (1.8 g) b.p. 190° (0.04 mm). tlc System A, $R_f$ 0.6.

The following compounds were similarly prepared:

b) 1-methyl-$N^5$-[3-[3-(1-piperidinylmethyl)phenoxy]propyl]-1H-1,2,4-triazole-3,5-diamine (1.72 g) gave 1-methyl-N-[3-[3-(1-piperidinylmethyl)phenoxy]propyl]-1H-1,2,4-triazole-5-amine (1.25 g) as a yellow oil; b.p. 220°, 0.08 mm; tlc System B, $R_f$ 0.5.

c) 1-methyl-$N^5$-[3-[3-[(hexamethyleneiminyl)methyl]phenoxy]propyl-1H-1,2,4-triazole-3,5-diamine (1.79 g) gave 1-methyl-N-[3-[3-](hexamethyleneiminyl)methyl]phenoxy]propyl]-1H-1,2,4-traizole-5-amine (500 mg) b.p. 240° 0.15 mm; tlc System B, $R_f$ 0.75.

Example 3

a) N-[4-[3-](dimethylamino)methyl]phenoxy]butyl]-3-phenylmethyl-1H-1,2,4-triazole-5-amine

A solution of 4-[3-[(dimethylamino)methyl]phenoxy]butanal (0.5 g) and 3-phenyl methyl-1H-1,2,4-triazole-5-amine (0.39 g) in dry toluene (60 ml) was heated under reflux during 3h. The solvent was evaporated and replaced with methanol (50 ml). Sodium borohydride (0.4 g) was added and the mixture was stirred at 20° during 2h. The methanol was evaporated and the residue partitioned between water and ethyl acetate. The organic extract was evaporated and the residue was purified by column chromatography to give the *title compound,* which crystallised from a mixture of ethyl acetate and cyclohexane as pale yellow crystals (0.09 g) m.p. 131—3°; tlc system A. $R_f$ 0.6.

The following compounds were similarly prepared from the appropriate aldehyde and aminotriazole:

b) 4-[3-(1-piperidinylmethyl)phenoxy]butanal (0.4 g), 3-methyl-1H-1,2,4-triazole-5-amine (0.15 g) and sodium borohydride (0.4 g) gave 3-methyl-N-[4-[3-(1-piperidinylmethyl)phenoxy]butyl]-1H-1,2,4-triazole-5-amine (50 mg) m.p 138—9°; tlc System A, $R_f$ 0.64.

c) 4-[3-(1-piperidinylmethyl)phenoxy]butanal (0.5 g) 3-phenylmethyl-1H-1,2,4-triazole-5-amine (0.33 g) and sodium borohydride (0.4 g) gave 3-phenylmethyl-N-[4-[3-(1-piperidinylmethyl)phenoxy]-butyl]-1H-1,2,4-triazole-5-amine (0.16 g) m.p. 145—6° tlc. System A, $R_f$ 0.6.

Example 4

a) 3-(2-phenylethyl)-N-[4-[3-(1-piperidinylmethyl)phenoxy]butyl]-1H-1,2,4-triazole-5-amine

*N-[3-(2-phenylethyl)-1H-1,2,4-triazole-5-yl]-4[3-(piperidinylmethyl)phenoxy]butanamide*

A solution of 4-[3-(1-piperidinylmethyl)phenoxy]butanoic acid (1.0 g), thionyl chloride (1.29 g) and dimethylformamide (6 drops) in methylene chloride (50 ml) was stirred at 25° during 5h. The solvent was evaporated and the residue was dissolved in methylene chloride (50 ml). 3-phenylethyl-1H-1,2,4-triazole-5-amine (0.68 g) was added and the mixture was stirred at 25° during 12h. The solvent was removed and the residue was diluted with water (50 ml). The pH of the aqueous solution was adjusted to pH8 with sodium bicarbonate and extracted with ethyl acetate. The combined organic extracts were evaporated and the residue was heated at 155° during 2h. The resulting solid was triturated under hot methanol to give the *title compound* (0.5 g) as a white powder m.p. 192—3° tlc System A, $R_f$ 0.62

3-(2-phenylethyl)-N-[4-[3-(1-piperidinylmethyl)phenoxy]butyl]-1H-1,2,4-triazole-5-amine

A suspension of N-[3-(2-phenylethyl)-1H-1,2,4-triazol-5-yl]-4-[3-(1-piperidinylmethyl)phenoxy] butanamide (0.4 g) and lithium aluminium hydride (0.33 g) in dioxan (50 ml) was heated under reflux in a nitrogen atmosphere during 12h. The cool mixture was quenched with water (50 ml) and extracted with ethyl acetate. The combined organic extracts were evaporated and the residue was crystallised from ethyl acetate to give the *title compound* as a white solid (0.23 g) m.p. 146—7° tlc System A, $R_f$ 0.71.

The following compounds were similarly prepared from 4-[3-(1-piperidinylmethyl)phenoxy]-butanoic acid (A) and the corresponding triazoles:

b) A (1.0 g) and 3-methylthio-1H-1,2,4-triazole-5-amine (0.47 g) gave 3-methylthio-N-[4-[3-(1-piperidinylmethyl)phenoxy]butyl]-1H-1,2,4-triazole-5-amine (0.1 g), m.p. 122—30 tlc System A $R_f$ 0.73

c) A (1.0 g) and 3-(2-methylpropyl)-1H-1,2,4-triazole-5-amine (0.68 g) gave 3-(2-methylpropyl)-N-[4-[3-(1-piperidinylmethyl)phenoxy]butyl]-1H-1,2,4-triazole-5-amine (0.08 g), m.p. 141—142°. N.m.r. ($CDCl_3$) 2.78, t, (1H; 3.0—3.3, m, (3H); 5.02, br, (1H); 6.0, m, (2H); 6.60, s+m, (4H); 7.2—8.8, m, (18H); 9.05, d, (6H).

Example 5

5-[4-[3-(1-piperidinylmethyl)phenoxy]butyl]amino-1H-1,2,4-triazole-3-methanol

*Ethyl 5-[4-[3-(1-piperidinylmethyl)phenoxy]butyl]amino-1H-1,2,4-triazole-3-carboxylate*

Methyl 5-amino-1H-1,2,4-triazole-3-carboxylate (0.568 g) and 4-[3-(1-piperidinylmethyl)-phenoxy]butanal in ethanol (40 ml) were heated at reflux for 2h. The cooled reaction mixture was treated with sodium borohydride (0.4 g) and stirred at room temperature for 15h. The solvent was evaporated and the residue was dissolved in 2*N* hydrochloric acid which was washed with ethyl acetate, basified with sodium carbonate and extracted with ethyl acetate. The combined organic extracts were evaporated to yield the *title compound* as a white solid which was recrystallised from a mixture of ethyl acetate and ethanol (1:1) (0.5 g) m.p. 170—1° dec.; tlc System B, $R_f$ 0.8.

5-[4-[3-(1-piperidinylmethyl)phenoxy]butyl]amino-1H-1,2,4-triazole-3-methanol

A mixture of ethyl 5-[4-[3-(1-piperidinylmethyl)phenoxy]butyl]amino-1H-1,2,4-triazole-3-carboxylate (0.3 g) and lithium aluminium hydride (0.1 g) in tetrahydrofuran (50 ml) was stirred at room temperature for 0.5 h, then quenched with water. The solid was filtered off and washed with methanol. The filtrate and washings were evaporated to leave a residue which was extracted with hot ethyl acetate. Evaporation of the extract gave an oil that solidified. It was crystallised from ethyl acetate to give the *title compound* as an off-white solid (0.094 g) m.p. 127—8° dec. tlc System B, $R_f$ 0.7.

Example 6

1-methyl-5-[[4-[3-(1-piperidinylmethyl)phenoxy]butyl]amino]-1H-1,2,4-triazole-3-methanol

*Methyl 1-methyl-5-[[1-oxo-4-[3-(1-piperidinylmethyl)phenoxy]butyl]amino]-1H-1,2,4-triazole-3-carboxylate*

A solution of 4-[3-(1-piperidinylmethyl)phenoxy] butanoic acid (2.77 g), thionyl chloride (2.5 ml) and dimethylformamide (6 drops) in methylene chloride (80 ml) was stirred at room temperature for 2h. The mixture was evaporated *in vacuo.* The resulting solid was dissolved in dimethylformamide (50 ml) and treated with methyl 5-amino-1-methyl-1H-1,2,4-triazole-3-carboxylate (1.56 g) After 18h at room temperature, the mixture was evaporated *in vacuo* and the residue was dissolved in water. The combined organic extracts were washed with water and evaporated *in vacuo.* The resulting solid was recrystallised from a mixture of ethyl acetate and cyclohexane to yield the *title compound* as a white powder (2.0 g) m.p. 108—10° tlc System B, $R_f$ 0.7

1-Methyl-5-[[4-[3-(1-piperidinylmethyl)phenoxy]butyl]amino]-1H-1,2,4-triazole-3-methanol

A suspension of methyl 1-methyl-5-[[1-oxo-4-[3-(1-piperidinylmethyl)phenoxy]butyl]amino]-1H-1,2,4-triazole-3-carboxylate (1.25 g) and lithium aluminium hydride (1.0 g) in tetrahydrofuran (30 ml) was stirred under nitrogen at room temperature for 5h, cooled to 0° and quenched with water. The mixture was diluted with ethyl acetate, filtered and the filtrate was evaporated *in vacuo*. The residue was chromatographed to give an oil, which crystallised on trituration with ether to yield the *title compound* as a white solid (0.33 g) m.p. 84—5°, tlc System B, $R_f$ 0.6.

Example 7

1-methyl-5-[[3-[3-(1-piperidinylmethyl)phenoxy]butyl]amino]-1H-1,2,4-triazole-3-methanol

A stirred suspension of 3-[3-(1-piperidinylmethyl)phenoxy]propanoic acid (1.84 g) in methylene chloride (50 ml) and dimethylformamide (5 drops) was treated with thionyl chloride (2.0 ml). The solution was maintained for 2h at room temperature and then evaporated *in vacuo*. The oily product was dissolved in dimethylformamide (50 ml) and treated with methyl-5-amino-1-methyl-1H-1,2,4-triazole-3-carboxylate (1.09 g). The reaction mixture was stirred at room temperature for 16h, evaporated *in vacuo*, the residue was dissolved in 8% sodium bicarbonate solution and washed with ethyl acetate. The organic extracts were washed with water and evaporated *in vacuo* to give methyl 1-methyl-5-[[1-oxo-4-[3-(1-piperidinylmethyl)phenoxy]propyl]amino-1H-1,2,4-triazole-3-3-carboxylate as an oil (0.832 g) which was dissolved in tetrahydrofuran (30 ml) and treated with lithium aluminium hydride (0.6 g) at 0°. The mixture was stirred at room temperature for 6h, then quenched with water, diluted with ethyl acetate and filtered. The filtrate was evaporated *in vacuo* to yield an oil (0.586 g), which was purified by column chromatography to give an oil, which crystallised on trituration with ether to yield the *title compound* (0.136 mg) as a white solid m.p. 118—9° tlc System B, $R_f$ 0.7.

Example 8

a) N-[5-[[3-[3-[(dimethylamino)methyl]phenoxy]propyl]amino]-1-methyl-1H-1,2,4-traizol-3-yl)-N′-methylthiourea

A solution of 1-methyl-$N^5$-[3-[3-[(dimethylamino)methyl]phenoxy]propyl]-1H-1,2,4-triazole-3,5-diamine (A) (1.0 g) and methyl isothiocyanate (0.24 g) in dry acetonitrile was heated under reflux for 48 hours. The solvent was removed and the residual oil purified by column chromatography. The residue was dissolved in ethyl acetate. A solution of tartaric aced in ethyl acetate was added to give the *title compound* as the tartrate salt as a white powder (0.22 g). tlc System A $R_f$ 0.51. NMR (free base) $CDCl_3$, 0.4 brs. (1H); 2.78 t (1H); 3.0—3.3 m (3H); 4.77 t (1H); 5.90 t (2H); 6.46 s+q (5H); 6.60 s (2H); 6.80 d (3H); 7.76 s (6H); 7.90 m (2H).

The following compounds were similarly prepared from the appropriate aminotriazole and isocyanate.

b) Amino triazole (1.0 g) and methylisocyanate (0.2 g) gave N-[5-[[3-[3-[(dimethylamino)methyl]-phenoxy]propyl]amino]-1-methyl-1H-1,2,4-triazol-3-yl]-N′-methylurea tartrate (1.2 g). tlc System A, $R_f$ 0.55. Nmr (free base) ($CDCl_3$) 1.48, s, (1H); 2.10, q, (1H); 2.77, t, (1H); 3.0—3.3, m, (3H); 5.0, t, (1H); 5.9, t, (2H); 6.43, q, (2H); 6.48, s, (3H); 6.62, s, (2H); 7.15, d, (3H); 7.77, s, (6H); 7.9, m, (2H).

c) 1-methyl-$N^5$-[3-[3-(1-piperidinylmethyl)phenoxy]propyl]-1H-1,2,4-triazole3,5-diamine (B) (1.5 g) and phenylisocyanate (0.52 g) gave N-[5-[[3-(1-piperidinylmethyl)phenoxy]propyl]amino]-1-methyl-1H-1,2,4-triazol-3-yl]-N′-phenylurea (1.07 g) m.p. 137—9° tlc System A, $R_f$ 0.57.

d) Aminotriazole (B) (1.5 g) and cyclohexylisocyanate (0.64 g) gave N-[5-[[3-[3-(1-piperidinylmethyl)-phenoxy]propyl]amino]-1-methyl-1H-1,2,4-triazol-3-yl]-N′cyclohexylurea (0.43 g) m.p. 129—130°. tlc System B, $R_f$ 0.6.

e) 1-methyl-$N^5$-[2-[[[5-[(dimethylamino)methyl]-4-methyl-2-furanyl]methyl]thio]ethyl]-1H-1,2,4-triazole-3,5-diamine (1 g) and phenylisocyanate (0.35 ml) gave N-[5-[[2-[[[5-[(dimethylamino)methyl]-4-methyl-2-furanyl]methyl]thio]ethyl]amino]-1-methyl-1*H*-1,2,4-triazol-3-yl]-N′-phenylurea (0.65 g) m.p. 166—7° tlc System A (79.1) $R_f$ 0.5.

f) 1-methyl-$N^5$-[2-[[[5-[(dimethylamino)methyl]-2-furanyl]methyl]thio]ethyl]-1H-1,2,4-triazole-3,5-diamine (3.1 g) and methyl isocyanate (0.97 g) gave N-methyl-N′-[1-methyl-5-[[2-[[[5-[(dimethylamino)-methyl]-2-furanyl]methyl]thio]ethyl]amino]-1H-1,2,4-triazole-3-yl]urea (2.5 g) tlc. System C $R_f$ 0.41. N.m.r. ($CDCl_3$) 1.70, s, (1H); 2.15, br q (1H); 3.92, s, (2H); 5.06, t, (1H); 6.52, s, (3H); 6.63, s+q, (4H), 7.20, d+t, (5H). 7.80, s, (6H).

Example 9

Ethyl 1-methyl-5-[[3-[3-(N,N-dimethylaminomethyl)phenoxy]propyl]amino]-1H-1,2,4-triazole-3-carbamate, tartrate

A solution of ethyl chloroformate (0.6 ml) and 1-methyl-$N^5$-[3-[3-(N,N-dimethylaminomethyl)-phenoxy]propyl]-1H-1,2,4-triazole-3,5-diamine (2.0 g) in dry dimethylformamide (30 ml) was stirred at

25° for 12 h. The reaction mixture was diluted with water and extracted with ethyl acetate. The organic extracts were evaporated to leave a yellow oil which was triturated with a mixture of ether, ethyl acetate and light petroleum (b.p. 60—80°). Insoluble material was filtered off and the residual oil dissolved in ethyl acetate. A saturated solution of tartaric acid in ethyl acetate was added until no more precipitate was formed, to give the *title compound* as a white solid (0.88 g) m.p. 110—115° tlc System A, $R_f$ 0.61.

b) Similarly prepared from 1-methyl-$N^5$-[3-[3-(1-piperidinylmethyl)phenoxy]propyl]-1H-1,2,4-triazole-3,5-diamine (5 g) and ethyl chloroformate (1.56 g) was ethyl [1-methyl-5-[[3-[3-(1-piperidinylmethyl)-phenoxy]propyl]amino]-1H-1,2,4-triazol-3-yl]carbamate tartrate (0.85 g) m.p. 82° N.m.r. ($D_2O$) 2.51, m, (1H); 2.7—3.1, m, (3H); 5.42, s, (2H); 5.5—6, m, (6H); 6.3—6.7 m+s, (7H); 6.8—7.3, m, (2H); 7.6—8.6, m, (8H); 8.71, t, (3H).

Example 10

a) N-[5[[3-[3-[(dimethylamino)methyl]phenoxy]propyl]amino]-1-methyl-1H-1,2,4-triazol-3-yl]acetamide, tartrate

Acetic anhydride (0.35 g) was added dropwise to a solution of 1-methyl-$N^5$-[3-[3-[(dimethyl-amino)methyl]phenoxy]propyl]-1H-1,2,4-triazole-3,5-diamine (A) (1 g) in dry pyridine and the reaction was stirred at 25° for 12h. Solvent was removed and the residue was dissolved in ethyl acetate, washed with aqueous saturated sodium carbonate solution, dried and evaporated. The residual oil was purified by chromatography and treated with a solution of tartaric acid in ethyl acetate to give the *title compound* (1.2 g) m.p. 103—6° tlc System A, $R_f$ 0.5.

The following compounds were similarly prepared from the appropriate diamine:

b) 1-methyl-$N^5$-[3-[3-(1-piperidinylmethyl)phenoxy]propyl]-1H-1,2,4-triazole-3,5-diamine (B) (2.5 g) and acetic anhydride (0.75 g) gave N-[5-[[3-[3-[(1-piperidinylmethyl)phenoxy]propyl]amino]-1-methyl-1H-1,2,4-triazole-3-yl]acetamide, tartrate (2.7 g) m.p. 90° (softens) tlc System A, $R_f$ 0.6.

c) Diamine (B) (1.0 g) and methanesulphonic anhydride (0.59 g) gave N-[5-[[3-[3-(1-piperidinyl-methyl)phenoxy]propyl]amino]-1-methyl-1H-1,2,4-triazol-3-yl]methane sulphonamide, methane sulphonate (1.2 g) m.p. 130—1°. tlc System A, $R_f$ 0.54

d) Diamine (A) (1 g) and benzoic anhydride (0.8 g) gave N-[5-[[3-[3-[(dimethylamino)methyl]phenoxy]-propyl]amino]-1-methyl-1H-1,2,4-triazol-3-yl]benzamide tartrate (0.48 g) m.p. 126—130°. tlc System A, $R_f$ 0.47.

e) 1-methyl-$N^5$-[2-[[[5-[(dimethylamino)methyl]-2-furanyl]methyl]thio]ethyl]-1H-1,2,4-triazole-3,5-diamine (1.9 g) and benzoic anhydride (1.2 g) gave N-[1-methyl-5-[[2-[[[5-[(dimethylamino)methyl]-2-furanyl]methyl]thio]ethyl]amino]-1H-1,2,4-triazol-3-yl]benzamide (0.95 g). tlc System C, $R_f$ 0.45.

Found: C, 57.7; H, 6.4; N, 19.8
$C_{20}H_{26}N_6O_2S$ requires C, 58.0; H, 6.3; N, 20.3%

f) 1-methyl-$N^5$-[2-[[[5-[(dimethylamino)methyl]-4-methyl-2-furanyl]methyl]thio]ethyl]-1H-1,2,4-triazole-3,5-diamine (0.96 g) and benzoic anhydride (1.0 g) gave N-[1-methyl-5-[[2-][[-5-[(dimethylamino)methyl]-4-methyl-2-furanyl]methyl]thio]ethyl]amino]-1H-1,2,4-triazol-3-yl]benzamide (0.6 g). tlc System C, $R_f$ 0.42 N.m.r. ($CDCl_3$) 0.90, s, (1H); 2.10, m, (2H); 2.5, m, (3H; 4.06, s, (1H); 5.10, t, (1H); 6.43, s, (2H); 6.52, s, (3H); 6.68, s+q, (4H); 7.32, t, (2H); 7.82, s, (6H); 8.10, s, (3H).

g) 1-methyl-$N^5$-[2-[[[5-[(dimethylamino)methyl]-2-thienyl]methyl]thio]ethyl]-1H-1,2,4-triazole-3,5-diamine (0.98 g) and benzoic anhyride (1.0 g) gave N-[1-methyl-5-[[2-[[[5-[(dimethylamino)methyl]-2-thienyl]methyl]thio]ethyl]amino]-1H-1,2,4-triazol-3-yl]benzamide (0.5 g). tlc System C, $R_f$ 0.46 N.m.r. ($CDCl_3$ 1.02, br. s, (1H); 2.07, m, (2H); 2.5, m, (3H); 3.30, m, (2H); 5.43, t, (1H); 6.18, s, (2H); 6.5, 2s+q, (7H); 7.28, t, (2H); 7.77, s, (6H).

Example 11

N-[1-methyl-5-[[3-[3-(1-piperidinylmethyl)phenoxy]propyl]amino]-1H-1,2,4-triazol-3-yl]formamide

A mixture of sodium hydride (0.72 g) and 1 - methyl - $N^5$ - [3 - [3 - (1 - piperidinylmethyl)phenoxy]-propyl] - 1H - 1,2,4 - triazole - 3,5 - diamine (3.44 g) in dimethylformamide (50 ml) was heated under reflux for 8h. The reaction mixture was cooled, poured onto water (300 ml) and extracted with ether. Evaporation of the organic extract gave a yellow oil, which was extracted with boiling cyclohexane (500 ml). As the cyclohexane extract cooled a brown oil precipitated out. This oil was discarded and the supernatant solution was cooled to room temperature to give the *title compound* which was recrystallised from a mixture of ethyl acetate and light petroleum (b.p. 60—80°) as a pale yellow solid (0.35 g) m.p. 68—75° (decomposition). tlc. System C $R_f$ 0.6.

Example 12

N-[1-methyl-5-[[2-[[[5-[(dimethylamino)methyl]-2-furanyl]methyl]thio]ethyl]amino]-1H-1,2,4-triazol-3-yl]formamide

A stirred suspension of 1-methyl-$N^5$-[2-[[[5](dimethylamino)methyl-2-furanyl]methyl]thio]ethyl]-1H-1,2,4-triazole-3,5-diamine, dihydrochloride (3.83 g) and potassium carbonate (3.04 g) in acetone (60 ml) was heated at reflux for 30 min, and then cooled to 5°C. Aceticformic anhydride (1.32 g) was added and the suspension was allowed to warm up to room temperature. The inorganic material was filtered off and the solution evaporated to give an oil which was chromatographed. The resulting oil was dissolved in ethyl acetate, washed with saturated aqueous sodium bicarbonate and evaporated to afford the *title compound* (0.47 g) as a colourless oil. tlc System B, $R_f$ 0.61.

Nmr. ($CDCl_3$) 0.28 brd, (1H); 0.93 d, (1H); 3.81, s, (2H; 5.25, brt (1H); 6.28, s, (2H); 6.47, s, (3H); 6.52 s+m (2H); 7.16, t, (2H) 7.74, s, (6H).

Example 13

N-[1-methyl-5-[[3-(1-piperidinylmethyl)phenoxy]propyl]amino]-1H-1,2,4-triazol-3-yl]-2-furancarboxamide

a) A solution of 1-methyl-$N^5$-[3-[3-(1-piperdinylmethyl)phenoxy]propyl]-1H-1,2,4-triazole-3,5-diamine (a) (1.0 g) and 2-furancarboxylic acid, chloride (0.42 g) in pyridine (25 ml) was kept at ambient temperature for 1h. The pyridine was evaporated *in vacuo* and the residue was azeoptropically distilled with toluene to give an oil which was partitioned between ethyl acetate and 2M hydrochloric acid. The pH of the aqueous extract was adjusted to pH 9 and it was extracted with ethyl acetate. The organic extracts were evaporated to a gum which was chromatographed on silica using ethyl acetate: methanol (9:1) to give the *title compound* (0.30 g) as a glassy solid. tlc System B, $R_f$ 0.57.

Nmr ($CDCl_3$) 1.5, brs, (1H); 2.53, m, (1H); 2.75, t+m, (2H); 3.0—3.3, m, (3H); 3.5, dd (1H); 5.31, t, (1H); 5.92, t, (2H); 6.42, q, (2H); 6.50, s, (3H); 6.59, s, (3H); 7.6, brs; (4H); 7.9, m, (2H); 8.5, m, (6H).

b) Similarly prepared from diamine (A) (1 g) and 3-pyridinecarboxylic acid, chloride, hydrochloride (0.57 g was N-[1-methyl-5-[[3-[3-(1-piperidinylmethyl)phenoxy]propyl]amino]1H-1,2,4-triazol-3-yl]-3-pyridinecarboxamide (0.98 g). tlc System B, $R_f$ 0.64.

Nmr ($CDCl_3$) 0.85, d, (1H); 1.07, brs, (1H); 1.22, dd (1H); 1.78, m, (1H); 2.62, q, (1H); 2.77, t, (1H); 3.0—3.3, m, (3H); 5.35, t, (1H); 5.92, t, (2H); 6.48, s, (3H); 6.50, m (2H) 6.55, s, (2H); 7.5—7.7, m, (4H); 7.90, m, (2H); 8.3—8.6, m, (6H).

The following compounds were similarly prepared from the appropriate aminotriazole and acid chloride

c) The triazole (A) (2.00 g) and 4-methoxybenzoic acid, chloride (1.10 g) gave 4-methoxy-N-[1-methyl-5-[[3-[3-(1-piperidinylmethyl)phenoxy]propyl]amino]-1H-1,2,4-triazol-3-yl]benzamide (2.00 g), m.p. 93.5—97°. tlc. System B $R_f$ 0.78

| | |
|---|---|
| Found: | C, 65.0; H, 7.0; N, 17.0; |
| $C_{26}H_{34}N_6O_3$ requires: | C, 65.2; H, 7.1; N, 17.5% |

d) The triazole (A) (2.00 g) and 4-methylbenzenesulphonic acid, chloride (1.22 g) was 4-methyl-N-[1-methyl-5-[[3-[3-(1-piperidinylmethyl)phenoxy]propyl]amino]-1H-1,2,4-triazol-3-yl]benzenesulphonamide (1.58 g) m.p. 100.5—102°

| | |
|---|---|
| Found: | C, 57.1; H, 6.7; N |
| $C_{25}H_{34}N_6O_3S \cdot 3/2H_2O$ requires | C, 57.1; H |

Example 14

5-[[3-[3-(1-piperidinylmethyl)phenoxy]propyl]amino]-1H-1,2,4-triazol-3-one

A solution of 3-[3-(1-piperidinylmethyl)phenoxy]propanamine (1.76 g) in tetrahydrofuran (70 ml) was treated with dimethyl N-methoxycarbonylcarbonimidodithioate (1.27 g) at room temperature, and the mixture was stirred for 6 h. Hydrazine hydrate (2 g) was added, and the mixture was heated under reflux for 18 h. The solution was evaporated, and the residue was chromatographed to give the *title compound* (250 mg) as a colourless oil. tlc. System B, $R_f$ 0.5.

Nmr ($D_2O$) 2.5, t, (1H); 2.8—3.0, m, (3H); 5.72, s, (1H); 5.8, t, (3H); 6.5, m, (2H); 6.65, t, (2H); 7.05, br. t, (2H); 7.7—8.6, m, (8H).

Example 15

1-methyl-3-phenylmethyl-N-[3-[3-(1-piperidinylmethyl)phenoxy]propyl]-1H-1,2,4-triazole-5-amine, tartrate

*N-[[1-methyl-2-(phenylmethylene)hydrazino][[3-[3-(1-piperidinylmethyl)phenoxy]propyl]amino]-methylene]benzeneacetamide*

A mixture of 3-[3-(1-piperidinylmethyl)phenoxy]propanamine (0.38 g) and methyl 1-methyl-N-phenylacetyl-2-(phenylmethylene) hydrazinecarboximidothioate (0.5 g) was heated at 50° under water pump vacuum during 4 h. The residue was dissolved in cyclohexane (20 ml) and light petroleum (b.p. 60—80°) was added. The precipitate was removed by filtration and the filtrate was evaporated to give the *title compound* (0.65 g) as a colourless oil. tlc. System A $R_f$ 0.7.

N.m.r. ($CDCl_3$) 2.2—3.0, m, (12H); 3.0—3.3, m, (3H); 6.01, t, (2H); 6.30, s, (2H); 6.55, br, q., (2H); 6.62, s, (3H); 6.66, s (2H); 7.65, m, (4H); 8.00, m, (2H); 8.3—8.6, m, (6H).

1-methyl-3-phenylmethyl-N-[3-[3-(1-piperidinylmethyl)phenoxy]propyl]-1H-1,2,4-triazole-5-amine

A solution of N-[[1-methyl-2-(phenylmethylene)hydrazino][[3-[3-(piperidinylmethyl)phenoxy propyl]amino]methylene]benzeneacetamide (0.65 g) in acetone (50 ml) was acidified to pH 1 with 2N hydrochloric acid and heated under reflux during 4 h. The solution was washed with ethyl acetate, basified with potassium carbonate and extracted with ethyl acetate. The combined organic extracts were dried and evaporated to give a residue which was purified by column chromatography. The resulting oil (0.4 g) was dissolved in ethyl acetate (10 ml), and a solution of tartaric acid (125 mg) in ethyl acetate (50 ml) was added to give the *title compound* (0.42 g) as a white powder. tlc. System A, $R_f$ 0.65.

N.m.r. ($CDCl_3$) (free base) 2.6—3.0, m, (6H); 3.0—3.4, m, (3H); 5.65, t, (1H); 5.95, t, (2H); 6.12, s, (2H); 6.47, q, (2H); 6.57, brs, (5H); 7.67, m, (4H); 7.93, m, (2H); 8.3—8.6, m, (6H).

Example 16

5-[[2-[[[5-[(dimethylamino)methyl]-2-furanyl]methyl]thio]ethyl]amino]-1-methyl-1H-1,2,4-triazole-3-methanol

*2-(Acetyloxy)-N-[[[2-[[[5-[(dimethylamino)methyl]-2-furanyl]methyl]thio]ethyl]amino][1-methyl(2-phenylmethylene)hydrazino]methylene]acetamide*

A mixture of methyl N-[2-(acetyloxy)acetyl]-1-methyl-2-(phenylmethylene) hydrazinecarboximidothioate (0.92 g) and 5-[[(2-aminoethyl)thio]methyl]-N,N-dimethyl-2-furanmethanamine (0.64 g) in acetonitrile (5 ml) was stirred at room temperature for 3 h. The solution was evaporated *in vacuo*. The oily residue was suspensed in ether (20 ml), filtered and the solid which crystallised was collected to give the *title compound* (0.8 g), m.p. 78—80°. tlc. System A $R_f$ 0.65.

5-[[2-[[[5-[(dimethylamino)methyl]-2-furanyl]methyl]thio]ethyl]amino]-1-methyl-1H-1,2,4-triazole-3-methanol

A solution of 2-(acetyloxy)-N-[[[2-[[[5-[(dimethylamino)methyl]-2-furanyl]methyl]thio]ethyl]-amino][1-methyl-2-(phenylmethylene)hydrazino]methylene]acetamide (0.74 g) in 2N hydrochloric acid was heated at 98—100° for 1 h. Water (5 ml) was added to the cooled solution which was washed with ethyl acetate. The aqueous fraction was made alkaline with sodium carbonate and the solution was evaporated to dryness *in vacuo*. The residue was suspended in ethyl acetate (20 ml), excess anhydrous sodium carbonate and decolourising charcoal were added. The suspension was boiled for 10 mins, cooled, filtered and the filtrate was evaporated *in vacuo*. The residual oil was chromatographed on silica using methanol:0.88 ammonia, (79.1) to give the *title compound* (0.35 g) as a pale brown oil. tlc. System A $R_f$ 0.6.

N.m.r. ($CDCl_3$) 3.92, s, (2H); 5.18, t, 5.32, brs, 5.49, s, (4H); 6.33, s, (2H); 6.51, s, 6.61, s+q, (7H); 7.23, t, (2H); 7.78, s, (6H).

Example 17

1-methyl-5-[[4-[3-(1-piperidinylmethyl)phenoxy]butyl]amino]-1H-1,2,4-triazole-3-methanol acetate

A solution of 1-methyl-5-[[4-[3-(1-piperidinylmethyl)phenoxy]butyl]amino]-1H-1,2,4-triazole-3-methanol (100 mg) in glacial acetic acid (5 ml) was heated under reflux for 8 h. The mixture was evaporated and the residue was triturated with ether to give the *title compound* as a white solid (97 mg) m.p. 119—20°. tlc. System B, $R_f$ 0.8.

Example 18

3-Methoxymethyl-1-methyl-5-[[4-[3-(1-piperidinylmethyl)phenoxy]butyl]amino]-1H-1,2,4-triazole

A solution of 1-methyl-3-[[4-[3-(1-piperidinylmethyl)phenoxy]butyl]amino]-1H-1,2,4-triazole-3-methanol (149 mg) and thionyl chloride (3 ml) was stirred at room temperature for 0.5 h and evaporated. The residue was dissolved in methanol (5 ml) and added to a solution of sodium hydride (200 mg) in methanol (5 ml). The mixture was stirred at room temperature for 18 h, evaporated, and

the residue was partitioned between ethyl acetate and water. The organic extracts were dried and evaporated to give an oil which was chromatographed on silica using ethyl acetate: isopropanol:water: 0.88 ammonia (25:15:8:2), to yield the *title compound* (108 mg). tlc. System B, $R_f$ 0.6.
N.M.R. ($CDCl_3$) 2.8, t, (1H); 3.0—3.3, m, (4H); 5.67, s, (2H): 5.72, t, (1H); 6.00, br. t, (2H); 6.3—6.6, m, (10H); 7.6, m, (4H); 8.8—8.7, m, (10H).

Example 19

1,3-dimethyl-N-[4-[3-(1-piperidinylmethyl)phenoxy]butyl]-1H-1,2,4-triazole-5-amine

A stirred suspension of 4-[3-(1-piperidinylmethyl)phenoxy]butanoic acid (4.0 g) in methylene chloride (100 ml) and dimethyl formamide (24 drops) was treated with thionyl chloride (5.16 g). The solution was stirred at room temperature for 18 h and evaporated *in vacuo*, to give a pale yellow solid which was dissolved in methylene chloride (100 ml) and treated with 5-amino-1,3-dimethyl-1,2,4-triazole (1.61 g). The reaction mixture was stirred at room temperature for 24 h and evaporated *in vacuo*. The residue was dissolved in aqueous sodium bicarbonate solution and extracted with ethyl acetate. The organic extracts were dried and evaporated to give a brown oil which was dissolved in tetrahydrofuran (500 ml), treated with lithium aluminium hydride (3.04 g), stirred at room temperature for 18 h and refluxed for 3 h. The cooled reaction mixture was quenched with water (300 ml) and extracted with ethyl acetate. The combined extracts were washed with brine, dried and evaporated *in vacuo* to give an oil which was distilled to give the *title compound* (1.7 g) as a pale yellow oil, b.p. 230°/0.06 mm.
N.m.r. 2.80, t, (1H); 3.0—3.3, m, (3H); 5.82, t, (1H) 6.02, m, (2H); 6.58, s, s+m, (7H); 7.65, 7.77 m, s, (7H) 8.0—8.7, m, (10H).

Example 20

The following compounds were prepared using the method of Example 2: 1-methyl-$N^5$-[2-[[[5-[(dimethylamino)methyl]-2-furanyl]methyl]thio]ethyl]-1H-1,2,4-triazole-3,5-diamine (1.0 g) gave 1-methyl-N-[2-[[[5][(dimethylamino)methyl]-2-furanyl]methyl]thio]ethyl]1H-1,2,4-triazole-5-amine (0.32 g) T.l.c. System C $R_f$ 0.54.
N.m.r. ($CDCl_3$); 2.50, s, (1H); 3.90, s, (2H); 6.30, s, (24); 6.5, 2s+q, (7H); 7.18, t, (2H) 7.79, s, (6H).

b) 1-methyl-$N^5$-[2-[[[5-[(dimethylamino)methyl]-2-thienyl]methyl]thio]ethyl]-1H-1,2,4-triazole-3, 5-diamine (1.0 g) gave 1-methyl-N-[2-[[[5-[(dimethylamino)methyl]-2-thienyl]methyl]thio]ethyl]-1H-1,2,4-triazole-5-amine (0.31 g). T.l.c. System C $R_f$ 0.52.
N.m.r. ($CDCl_3$): 2.57, s, (1H); 3.3, m, (2H); 5.58, br, s, (1H); 6.16, s, (2H); 6.48, 2s+q, (7H); 7.20, m, (2H); 7.76, s, (6H).

Example 21

1-methyl-N-[[3-[3-(1-piperidinylmethyl)phenoxy]propyl]amino]-1H-1,2,4-triazole-3-ethanol tartrate salt

A suspension of ethyl 1-methyl-5-[[3-[3-(1-piperidinylmethyl)phenoxy]propyl]amino]-1H-1,2,4-triazole-3-acetate (0.3 g) and lithium aluminium hydride (100 mg) in tetrahydrofuran (50 ml) was stirred at 25° under nitrogen during 24h. The mixture was quenched with water and extracted with ethyl acetate. The combined organic extracts were evaporated and the residue was chromatographed to give a pale yellow oil which dissolved in boiling cyclohexane. The hot solution was allowed to cool to room temperature, and the oil that precipitated was treated with a saturated solution of (d)-tartaric acid in ethyl acetate to give the *title compound* as a white powder (80 mg).
N.m.r. (free base) ($CDCl_3$); 2.8, t, (1H); 3—3.3, m, (3H); 5.3, t, (1H); 5.9, t, (2H); 6.13, t, (2H); 6.0, br, s, (1H) 6.4, q, (2H); 6.52, s, (3H) 6.57, s, (2H); 7.2, t, (2H); 7.65, m, (4H); 7.88, m, (2H); 8.5, m, (6H) T.l.c. System A $R_f$ 0.55.

Example 22

a) 5-[2-[[[5-[(dimethylamino)methyl]-2-thienyl]methyl]thio]ethyl]-1-methyl-3-phenylmethyl-1H-1,2,4-triazole-5-amine

*2-(phenyl)-N[[[2-[[[5-[(dimethylamino)methyl]-2-thienyl]methyl]thio]ethyl]amino][1-methyl]2-(phenylmethylene)hydrazino]methylene]acetamide*

A mixture of 5-[[[2-(amino)ethyl]thio]methyl]-N,N-dimethyl-2-thiophene methanamine (C) (1.0 g) and methyl 1-methyl N-phenylacetyl-2-(phenylmethylene) hydrazinecarboximidothioate (A) (1.42 g) was heated at 60° under water-pump vacuum during 3h. to give the *title compound* (1.9 g) as a colourless oil. T.l.c. System A $R_f$ 0.61.
N.m.r. ($CDCl_3$): 2.1, br, m, (1H); 2.2—2.4 m (3H); 2.5—2.85, m, (8H); 3.3, dd (2H); 6.18, s, (2H); 6.33, s, (2H) 6.48, s, (2H); 6.68—6.70, m, (5H); 7.4, t, (2H); 7.78, s, (6H);

5-[2-[[[5-[(dimethylamino)methyl]-2-thienyl]methyl]thio]ethyl]-1-methyl-3-phenylmethyl-1H-1,2,4-triazole-5-amine

2N hydrochloric acid (5 ml) was added to a solution of 2-(phenyl-N-[[[2-[[[5-[(dimethylamino)-

methyl]-2-thienyl]methyl]thio]ethyl]amino][1-methyl-2-(phenylmethylene)hydrazino]methylene]-acetamide (1.9 g) in toluene (20 ml) and the mixture was heated on a steam bath for 30 min. The aqueous layer was washed with toluene, treated with potassium carbonate, and evaporated *in vacuo*. The residue was dissolved in ethyl acetate, filtered and the filtrate was evaporated to yield an oil which was chromatographed to give the *title compound* (0.47 g) as a pale orange oil. T.l.c. System A. $R_f$ 0.55.

| | |
|---|---|
| Found | C, 60.0; H, 6.8; N, 17.1; |
| $C_{20}H_{27}N_5S_2$ requires: | C, 59.8; H, 6.8; N, 17.4% |

The following compounds were similarly prepared from the appropriate methyl 1-methyl N-acyl-2-(phenylmethylene) hydrazinecarboximidothioate and the corresponding diamine

b) A (0.5 g) and 5[[(2-aminoethyl)thio]methyl]-N,N-dimethyl-2-furanmethanamine (0.33 g) gave 5-[2-[[[5-[(dimethylamino)methyl]-2-furanyl]methyl]thio]ethyl]-1-methyl-3-phenylmethyl-1H-1,2,4-triazole-5-amine (0.21 g). T.l.c. System A $R_f$ 0.61
N.m.r. ($CDCl_3$): 2.6—2.9, m, (5H); 4.06, s, (1H): 6.16, s, (2H); 5.53, t, (1H); 6.40, s, (2H), 6.58—6.61, m, (7H); 7.25, t, (2H); 7.79, s, (6H).

c) A (1.21 g) and 5-[[(2-aminoethyl)thio]methyl]-3-N,N-trimethyl-2-furanmethanamine (0.85 g) gave 5-[2-[[[5-[(dimethylamino)methyl]-4-methyl-2-furanyl]methyl]thio]ethyl]-1-methyl-3-phenylmethyl-1H-1,2,4-triazole-5-amine (0.42 g) as a pale yellow oil. T.l.c. System A $R_f$ 0.58.
N.m.r. ($CDCl_3$): 2.6—2.9, m, (5H); 4.06, s, (1H): 6.15, s, (2H); 5.53, t, (1H); 6.40, s, (2H), 6.58—6.61, m, (7H); 7.27, t, (2H); 7.8, s, (6H); 8.1, s, (3H);

d) N-[2-(acetyloxy)acetyl]-1-methyl-2-phenylmethylene)hydrazinecarboximidothioate (B) (0.75 g) and 3-[3-[(1-hexamethyleneiminyl)methyl]phenoxy]propanamine (0.64 g) gave 1-methyl-5-[3-[3-[(1-hexamethyleneiminyl)methyl]phenoxy]propyl]amino1H-1,2,4-triazole-3-methanol (0.32 g), m.p. 80—82°.
N.m.r. ($CDCl_3$); 2.8, t, (1H); 3.0—3.3 m, (3H); 5.42—5.45, m, (3H); 5.88—7.0, m, (11H); 7.4, m, (4H); 7.9, m, (2H); 8.4, m, (8H).

e) B (1.37 g) and diamine C (1.0 g) gave 5-[[2-[[[5-[(dimethylamino)methyl]-2-thienyl]methyl]thio]ethyl]amino]-1-methyl-1H-1,2,4-triazole-3-methanol (0.2 g). T.l.c. System B. $R_f$ 0.48.
N.m.r. ($CDCl_3$); 3.25, m, (2H); 5.1, m, (2H); 5.5, s, (2H); 6.16, s, (2H); 6.35—6.5, m, (7H); 7.22, t, (2H) 7.77, s, (6H).

f) B (0.75 g) and 3-[3-(1-pyrrolidinylmethyl)phenoxy]propanamine (0.57 g) gave 1-methyl-5-[3-[3-(1-pyrrolidinylmethyl)phenoxy]propyl]amino]-1H-1,2,4-triazole-3-methanol (0.4 g) m.p. 105—7°C.

| | |
|---|---|
| Found: | C, 62.9; H, 7.9; N, 20.7 |
| $C_{18}H_{27}N_5O_2$ requires: | C, 62.6; H, 7.9; M, 20.3% |

g) B (0.75 g) and 5-[[(2-aminoethyl)thio]methyl]-3-N,N-trimethyl-2-furanmethanamine (0.5 g) gave 5-[[2-[[[5-[(dimethylamino)methyl]-4-methyl-2-furanyl]methyl]thio]ethyl]amino]-1-methyl-1H-1,2,4-triazole-3-methanol (0.32 g) T.l.c. System B $R_f$ 0.53.
N.m.r. ($CDCl_3$) 4.02, s, (1H); 5.32—5.48, m, (3H); 6.37, s, (2H); 6.50—6.7, m, (7H); 7.25, t, (2H); 7.82, s, (6H); 8.08, s, (3H).

## Example 23

$\alpha,\alpha$,1-trimethyl-5-[[4-[3-(1-piperidinylmethyl)phenoxy]butyl]amino]-1H-1,2,4-triazole-3-methanol
*N-[3-(1-hydroxy-1-methylethyl)-1-methyl-1H-1,2,4-triazole-5-yl]-4-[3-(1-piperidinylmethyl)phenoxy]-butanamide*

A solution of methyl 1-methyl-5-[[1-oxo-4-[3-(1-piperidinylmethyl)phenoxy]butyl]amino]-1H-1,2,4-triazole-3-carboxylate (508 mg) in tetrahydrofuran (10 ml) at −78° was treated with a solution of methyl magnesium iodine [(1.6 ml) of a solution prepared from magnesium turnings (400 mg) and methyl iodine (0.7 ml) in ether (10 ml)]. The mixture was maintained at −78° for 0.5 h. allowed to reach room temperature and quenched with water. The mixture was partitioned between ethyl acetate and 2N sodium hydroxide, the aqueous layer was extracted with ethyl acetate, and the combined organic extracts were dried and evaporated *in vacuo*. The residue was chromatographed to give the *title compound* (358 mg) as an oil. T.l.c. System B. $R_f$ 0.8
N.m.r. ($CDCl_3$): 2.82, t, (1H); 3.0—3.3, m, (4H); 6.0, t, (2H); 6.20, s, (1H); 6.32, s, (3H); 6.60, s, (2H); 7.3, m, (2H); 7.5—8.0, m, (6H); 8.45, s, (6H); 8.5, m, (6H).

$\alpha,\alpha$,1-trimethyl-5-[[4-[3-(1-piperidinylmethyl)phenoxy]butyl]amino]-1H-1,2,4-triazole-3-methanol

A stirred solution of N-[3-(1-hydroxy-1-methylethyl)-1-methyl-1H-1,2,4-triazole-5-yl]-4-[3-(1-piperidinylmethyl)phenoxy]butanamide (308 mg) in tetrahydrofuran (20 ml) under nitrogen was treated, with lithium aluminium hydride (0.5 g) in an ice-bath. The mixture was stirred at room temperature for 24h, quenched with water and extracted with ethyl acetate. The combined organic extracts were dried and evaporated to give an oil (300 mg) which was chromatographed on silica using ethyl acetate: isopropanol:water: 0.88 ammonia (25:15:8:2) to give the *title compound* (224 mg) as an oil. T.l.c. System B $R_f$ 0.7.

N.m.r. ($CDCl_3$): 2.80, t, (1H); 3.0—3.3, m, (3H); 5.65, t, (1H); 6.02, s, (2H); 6.52, m, (7H); 7.60, m, (4H); 8.0—8.7, m, (10H); 8.46, s, (6H).

Example 24

1-methyl-5-[[4-[3-(1-piperidinylmethyl)phenoxy]butyl]amino]-1H-1,2,4-triazole-3-methanamine

A solution of methyl 1-methyl-5-[[1-oxo-4-[3-(1-piperidinylmethyl)phenoxy]butyl]amino]-1H-1,2,4-triazole-3-carboxylate (1.94 g) in 0.88 ammonia was stirred at room temperature for 18 h. The mixture was evaporated to yield a yellow foam, which was triturated with ether to give a yellow solid (1.56 g) which was used without further purification. A portion of this solid (1.20 g) was suspended in tetrahydrofuran (50 ml) and treated at room temperature with lithium aluminium hydride (1.0 g). The mixture was heated under reflux for 24 h, cooled and quenched with water. The resulting mixture was extracted with ethyl acetate, and the combined organic extracts were evaporated to yield an oil (0.90 g) which was chromatographed to give the *title compound* a an oil (210 mg) T.l.c. System B $R_f$ 0.5. N.m.R. ($D_2O$); 2.5, t, (1H); 2.8—3.0, m, (3H); 5.43, s, (4H); 5.72, s, (2H); 5.8, t, (2H); 5.86, s, (2H); 6.47, s, (3H); 6.6, m, (4H); 7.05, bt, (2H); 8.0—8.6, m, (10H).

Example 25

a) N,N,1-Trimethyl-5-[[4-[3-(1-piperidinylmethyl)phenoxy]butyl]amino]-1H-1,2,4-triazole-3-methanamine

A solution of 1-methyl-5-[[4-[3-(1-piperidinylmethyl)phenoxy]butyl]amino]-1H-1,2,4-triazole-3-methanol (200 mg) in thionyl chloride (4 ml) was stirred at room temperature for 0.5 h. The mixture was evaporated to give a white foam which was used without further purification, and dissolved in a 33% solution of dimethylamine in ethanol (25 ml). The resulting solution was maintained at room temperature for 16h, and evaporated to give an oil which was chromatographed on silica using ethyl acetate:isopropanol:water:0.88 ammonia (25:15:8:2) to give the *title compound* (132 mg) as an oil. T.l.c. System B. $R_f$ 0.6.

N.m.r. ($CDCl_3$): 2.8, t, (1H); 3.0—3.3, m, (3H); 5.66, t, (1H); 6.02, t, (2H); 6.4—6.7, m, (9H); 7.6, m, (4H); 7.7, s. (6H); 8.0—8.7, m, (10H).

b) Similarly prepared from the same triazole methanol (150 mg), thionyl chloride (3 ml) and 0.88 aqueous ammonia (50 ml) was 1-methyl-5-[[4-[3-(1-piperidinylmethyl)phenoxy]butyl]amino]-1H-1,2,4-triazole-3-methanamine (109 mg). T.l.c. System B and n.m.r. ($D_2O$) were the same as for the product of Example 25.

Example 26

1-methyl-5-[[4-[3-(1-piperidinylmethyl)phenoxy]butyl]amino]-1H-1,2,4-triazole-3-methanol

A stirred solution of 1-methyl-5-[[4-[3-(1-piperidinylmethyl)phenoxy]butyl]amino]-1H-1,2,4-triazole-3-methanol, acetate (5 mg) in ethanol (1 ml) was treated with 2N sodium hydroxide solution (0.5 ml). The mixture was maintained at room temperature for 2h, evaporated *in vacuo*, and the residue was partitioned between ethyl acetate and water. The aqueous layer was extracted with ethyl acetate and the combined organic extracts were dried and evaporated *in vacuo* to yield the *title compound* (4 mg) as a white solid, m.p. 82—83°. T.l.c. System B, $R_f$ identical to product of Example 6.

Example 27

1-methyl-5-[[3-[3-[(1-piperidinyl)methyl]phenoxy]propyl]amino]-1H-1,2,4-triazole-3-methanol

3-[3-(1-piperidinylmethyl)phenoxy]propanamine (1.24 g) and methyl-N-[2-acetyloxy)acetyl]-1-methyl-2-(phenylmethylene)hydrazinecarboximidothioate (1.535 g) were heated together at 50° for 3 h. The residual oil was used without purification, and dissolved in acetone (30 ml). 2N hydrochloric acid (5 ml) was added, the solution was stirred at room temperature for 1.5 h and left overnight without stirring. A further quantity of 2N hydrochloric acid (5 ml) was added and the solution was heated at reflux for 2 h. Water (50 ml) was added, the aqueous solution was washed with ethyl acetate, and basified with excess solid sodium carbonate. The basic solution was extracted with ethyl acetate, and the organic extracts were evaporated to give the *title compound* as a white solid (1.1 g) m.p. 119°. T.l.c. System B, $R_f$ 0.7.

Example 28

Following the method of Example 8, 1-methyl-$N^5$-[2-[[[5-[(dimethylamino)methyl]-2-thienyl]-methyl]thio]ethyl]-1H-1,2,4-triazole-3,5-diamine (0.67 g) and methyl isocyanate (0.17 ml'

gave N-[5-[[2-[[[5-[(dimethylamino)methyl]-2-thienyl]methyl]thio]ethyl]amino]-1-methyl-1H-,2,4-triazol-3-yl]-N'-methyl urea (0.4 g).
N.m.r. ($CDCl_3$): 3.23, q, (2H); 6.02, s, (2H); 6.4—6.5, s+s+t, (7H); 7.1—7.2, s+s, (5H); 7.75, s, (6H). T.l.c. System B $R_f$ 0.55.

Example 29

The following compounds were prepared using the method of Example 10

a) 1-methyl-$N^5$-[2-[[[-5-[(dimethylamino)methyl]-2-thienyl]methyl]thio]ethyl]-1H-1,2,4-triazole-3,5-diamine (0.5 g) and methane sulphonic anhydride (0.3 g) gave N-[1-methyl-5-[[2-[[[5-[(dimethylamino)-methyl]-2-thienyl]methylthio]ethyl]amino]-1H-1,2,4-triazole-3-yl]methane sulphonamide (0.05 g).
N.m.r. ($CDCl_3$): 3.22, q, (2H); 4.28, t, (1H); 6.10, s, (2H); 6.4—6.5, m, (7H); 6.80, s, (*ca* 3H); 7.28, t, (2H); 7.73, s, (6H). T.l.c. System B $R_f$ 0.25.

b) 1-methyl-$N^5$-[2-[[[5-[(dimethylamino)methyl]-2-furanyl]methyl]thio]ethyl]-1H-1,2,4-triazole-3,5-diamine (1.55 g) and acetic anhydride (0.5 g) gave N-[1-methyl-5-[[2-[[[5-[(dimethylamino)methyl]-2-furanyl]methyl]thio]ethyl]amino]-1H,1,2,4-triazol-3-yl]acetamide, oxalate (1.3 g) m.p. 132—4°.
N.m.r. ($D_2O$): 3.30, d, (1H); 3.60, d, (1H); 5.67, s, (2H); 6.13, s, (2H); 6.45, s+t, (5H); 7.15, s+t, (8H) 7.80, s, (3H).

c) 1-methyl-$N^5$-[2-[[[5-[(dimethylamino)methyl]-4-methyl-2-furanyl]methyl]thio]ethyl]-1H-1,2,4-triazole-3,5-diamine (1 g) and acetic anhydride (0.32 ml) gave N-[1-methyl-5-[[2-[[[5-[(dimethylamino)methyl]-4-methyl-2-furanyl]methyl]thio]ethyl]amino]-1H-1,2,4-triazol-3-yl]acetamide (0.5 g). T.l.c. System B $R_f$ 0.4.
N.m.r. ($CDCl_3$): 1.2, br.s. (1H); 4.05, s, (1H); 5.2, t, (1H); 6.4, s, (2H); 6.5—6.7, m, (7H); 7.25, t, (2H); 7.75, s, (9H); 8.10, s, (3H).

d) 1-methyl-$N^5$-[3-[4-(1-piperidinylmethyl)phenoxy]propyl]-1H-1,2,4-triazole-3,5-diamine (0.5 g) and acetic anhydride (157 mg) gave 1-methyl-N-[5-[[3-[4-(1-piperidinylmethyl)phenoxy]propyl]amino]-1H-1,2,4-triazol-3-yl]acetamide (0.58 g).
N.m.r. ($CDCl_3$): 2.75, d, (2H); 3.18, d, (2H); 5.95, t, (2H); 6.4—6.7, m, (2H); 6.55, s, (3H); 6.60, s, (2H) 7.5—8.1, m, (6.H); 7.87, s, (3H); 8.3—8.7m, (6H). T.l.c. System A, $R_f$ 0.52.

Example 30

The following compounds were prepared using the method of Example 13:

a) 1-methyl-$N^5$-[3-[3-(1-piperidinylmethyl)phenoxy]propyl]-1H-1,2,4-triazole-3,5-diamine (A) (2.00 g) and phenylacetylchloride (0.99 g) gave N-[1-methyl-5-[[3-[3-(1-piperidinylmethyl)phenoxy]propyl]-amino]-1H-1,2,4-triazol-3-yl]benzeneacetamide (0.92 g).
N.m.r. ($CDCl_3$): 1.9, br, s, (1H); 2.70, 2.80, s+t, (6H); 3.0—3.3, m, (3H); 5.30, t, (1H); 5.98, t, (2H); 6.28, br. s, (2H) 6.55, s+s+q, (7H); 7.65, m, (4H); 8.0, m, (2H); 8.5, m, (6H). T.l.c. System B, $R_f$ 0.71.

b) The triazole (A) (2.00 g) and benzoyl chloride (0.90 g) gave N-[1-methyl-5-[[3-(1-piperidinylmethyl)-phenoxy]propyl]amino]-1H-1,2,4-triazole-3-yl]benzamide (1.05 g).
N.m.r. ($CDCl_3$): 0.9, br, s, (1H); 2.00—2.25, m, (2H) 2.4—2.7, 2.8, m+t, (4H); 3.00—3.35, m, (3H); 5.22, t, (1H) 5.98, t, (2H); 6.50, 6.55, 6.60, s+s+q, (7H); 7.60, m, (4H) 8.0, m, (2H); 8.5, m, (6H);

| | |
|---|---|
| Found: | C, 66.77; H, 7.30; N, 18.72; |
| $C_{25}H_{32}N_6O_2$ requires: | C, 66.94; H, 7.19; N, 18.74% |

Example 31

N-methyl-N'-[1-methyl-5-[[3-[3-(1-piperidinylmethyl)phenoxy]propyl]amino]-1H-1,2,4,-triazol-3-yl]urea

A mixture of 1-methyl-$N^5$-[3-[3-(1-piperidinylmethyl)phenoxy]propyl]-1H-1,2,4-triazole-3,5-diamine (2.00 g) and methylisocyanate (1.2 g) in dry acetonitrile (60 ml) was heated at reflux for 2 h. and allowed to cool. The precipitated solid was collected and recrystallised from methanol to give the *title compound* (1.9 g). T.l.c. System B: $R_f$ 0.71.
N.m.r. ($CDCl_3$): 1.22, s, (1H); 2.07, q, (1H); 2.78, t, (1H); 3.00—3.30, m, (3H); 4.90, t, (1H); 5.90, t, (2H); 6.42, 6.48, 6.58, q+s+s, (7H); 7.10, d, (3H); 7.60, 7.90, m+m, (6H); 8.50, m, (6H).

Example 32

Ethyl-[5-[[2-[[[5-[(dimethylamino)methyl]-2-thienyl]methyl]thio]ethyl]amino]-1-methyl-1H-1,2,4-triazole-3-yl]carbamate

A cooled mixture of ethylchloroformate (0.45 g) in pyridine was treated with a solution of $N^5$-[2-[[[5-[(dimethylamino)methyl]-2-thienyl]methyl]thio]ethyl]-1-methyl-1*H*-1,2,4-triazole-3,5-diamine (0.7 g) in pyridine (8 ml). Sodium carbonate (2 g) and water (10 ml) were added and the mixture evaporated *in vacuo*. The residue was dissolved in water (15 ml) and extracted with hot isopropanol (30 ml). The extract was evaporated and the residue purified by column chromatography (silica:methanol:0.88 ammonia; 79:1) to give the *title compound* (0.48 g). T.l.c. System A $R_f$ 0.6.

N.m.r. ($CDCl_3$) 1.8, brs (1H); 3.25, m, (2H); 5.47, brt (1H); 5.88, q, (2H); 6.13, s, (2H); 6.47, 2s+q, (7H); 7.24, t, (2H); 7.75, s, (6H); 8.73, t, (3H).

Example 33

The following compound was prepared using the method of Example 15:
3-[4-(1-piperidinylmethyl)phenoxy]propanamine (0.61 g) and methyl N-[2-(acetyloxy)acetyl]-1-methyl-2-(phenylmethylene) hydrazinecarboximidothioate (0.75 g) gave 2-(acetyloxy)-N-[[[3-[4-(1-piperidinylmethyl)phenoxy]propyl]amino][1-methyl-(2-phenylmethylene) hydrazino]methylene]acetamide (1,2 g). T.l.c. System A $R_f$ 0.59.
N.m.r. ($CDCl_3$) 2.2, s, (1H); 2.3—2.45, m, (2H); 2.5—2.75, m, (3H); 2.85, d, (2H); 3.2, d, (2H); 5.4, s, (2H); 5.9, t, (2H); 6.45, q, (2H); 6.6, s, (3H); 6.46, s, (2H); 7.55—7.8, m, (6H) 7.85, s, (3H); 8.3—8.6, m, (6H).

The above acetamide (1.2 g) was acidified to give 1-methyl-N-[[3-[4-(1-piperidinylmethyl)-phenoxy]propyl]amino]-1H-1,2,4-triazole-3-methanol. T.l.c. System A. $R_f$ 0.6. N.m.r. ($CDCl_3$) 2.78, d, (2H) 3.20, d, (2H); 5.48, s, (2H); 5.94, t, (2H); 6.42, q, (2H); 6.52, s, (3H); 6.62, s, )2H); 6.8, br, s, (1H); 7.68, m, (4H) 7.9, m, (2H); 8.3—8.7, m, (6H).

Example 34

α,1-dimethyl-N-[[4-[3-(1-piperidinylmethyl)phenoxy]butyl]amino]-1H-1,2,4-triazole-3-methanol

*1-Methyl-N-[[4-[3-(1-piperidinylmethyl)phenoxy]butyl]amino]-1H-1,2,4-triazole-3-carbaldehyde*

Dimethyl sulphoxide (304 mg) was added to a solution of oxalyl chloride (254 mg) in dichloromethane (20 ml) at —60° under nitrogen. The solution was stirred at —50 to —60° for 2 min and a solution of 1-methyl-N-[[4-[3-(piperidinylmethyl)phenoxy]butyl]amino]-1H-1,2,4-triazole-3-methanol (0.5 g) in dichloromethane (10 ml) was added. The mixture was stirred at —50 to —60° for 15 min and then quenched with triethylamine (657 mg). The solution was allowed to warm to 25° and was diluted with water. The aqueous phase was extracted with dichloromethane and the combined organic extracts were dried and evaporated to leave the *title compound* as a pale yellow oil (0.4 g).
N.m.r. $CDCl_3$ 0.2, s, (1H); 2.78, t, (1H), 3.0—3.3, m, (3H); 5.25, br, t, (1H); 6.06, br t, (2H); 6.40, s, (3H); 6.5, q, (2H); 6.60, s, (2H); 7.68, br, (4H); 8.0—8.6, m, (10H). Tlc System A $R_f$ 0.55.

α,1-dimethyl-N-[[4-[3-(1-piperidinylmethyl)phenoxy]butyl]amino]-1H-1,2,4-triazole-3-methanol

A solution of methyl lithium [(0.6 m in ether), 10 ml] was added to a solution of the above carbaldehyde (0.4 g) in tetrahydrofuran (20 ml) at 25° under nitrogen. The mixture was stirred for 12 h, quenched with water and extracted with ethyl acetate. The combined organic extracts were evaporated to give a yellow oil (0.3 g). Tlc System C $R_f$ 0.3.

Example 35

Following the method of Example 22, N-[2-(acetyloxy)acetyl]-1-methyl-2-(phenylmethylene) hydrazinecarboximidothioate (0.75 g) and 3-[3-[1-(4-hydroxypiperidinyl)methyl]phenoxy]propanamine 0.65 g) gave 1-methyl-5-[[3-[3-[1-(4-hydroxypiperidinyl)methyl]phenoxy]propyl]amino]-1H-1,2,4-triazole-3-methanol (0.3 g), t.l.c. System B $R_f$ 0.4.
N.m.r. ($CDCl_3$) 2.80, t, (1H); 3.0—3.3, m, (3H); 4.63, t, (1H); 5.52, s, (2H); 5.8—6.1, m, (5H); 6.3—6.7, m, (7H); 7.1—7.4, m, (2H) 7.7—8.7, m, (8H).

Example 36

1-methyl-[5-[[3-[3-(1-piperidinylmethyl)phenoxy]propyl]amino]-1H-1,2,4-triazole-3-one

The following compound was prepared using the method of preparation 5:methyl 1-methyl-2-(phenylmethylene) hydrazinecarboximidothioate hydroiodide (2,35 g) and methylchloroformate (0.58 ml) gave methyl-N-methoxycarbonyl-1-methyl-2-(phenymethylene)hydrazinecarboximidothioate (0.65 g), m.p. 85—6°.

The following compound was prepared using the method of Example 15:
3-[3-(1-piperidinylmethyl)phenoxy]propanamine (0.62 g) and methyl-N-methoxy carbonyl-1-methyl-2-(phenylmethylene) hydrazinecarboximidothioate (0.59) gave methyl[[1-methyl-2-(phenylmethylene)-hydrazino][[3-[3-(1-piperidinylmethyl)phenoxy]propyl]amino]methylene]carbamate (0.9 g). T.l.c. silica; methanol. $R_f$ 0.35.
The above carbamate (0.6 g) was acidified to give 1-Methyl-[5-[[3-[3-(1-piperidinylmethyl)phenoxy]-propyl]amino]-1H-1,2,4-triazole-3-one tartrate (0.03 g). T.l.c. System B. $R_f$ 0.23.
N.m.r. ($D_2O$); 2.50, t, (1H); 2.8—3.0, m, (3H); 5.45, s, (2H); 5.8, s+m, (4H); 6.4—6.6, m, (4H); 6.73, s, (3H); 7.05, t, (2H); 7.7—8.6 m, (8H).

Example 37

The following compound was prepared using the method of Example 22:
Methyl-N-[2-(acetyloxy)acetyl]-1-methyl-2-(phenylmethylene) hydrazinecarboximidothioate (0.75 g) and and 3-(3 aminopropoxy)-N,N-dimethylbenzenemethanamine (0.51 g) gave 1-methyl-5-[[3-[3-[(dimethylamino)methyl]phenoxy]propyl]amino]-1H-1,2,4-triazole-3-methanol (0.3 g). T.l.c. B. $R_f$ 0.52.

N.m.r. ($CDCl_3$); 2.76, t, (1H); 3—3.3, m, (3H); 5.4—5.9, m, (6H); 6.42—6.64, m, (8H); 7.8—7.9, m, (8H).

Example 38

1-Methyl-5-[[3-[3-[(phenylmethylamino)methyl]phenoxy]propyl]amino]-1H-1,2,4-triazole-3-methanol

A solution of 5-[[3-(3-formyl)phenoxy)propyl]amino]-1-methyl1H-1,2,4-triazole-3-methanol (532 mg) in ethanol (15 ml) was treated with benzylamine (5 ml) and stirred at room temperature for 1.5 h. The solution was treated with sodium borohydride (500 mg) and stirred at room temperature for 16 h. The mixture was evaporated, and the residue partitioned between 2N hydrochloric acid and ethyl acetate. The aqueous layer was washed with ethyl acetate, neutralised with potassium carbonate, and extracted with ethyl acetate. The combined extracts were dried and evaporated *in vacuo* to yield an oil. This oil was chromatographed on silica gel, using a mixture of methanol-ethyl acetate (1:1) to give the *title compound* as an oil (315 mg). T.l.c. silica: methanol, $R_f$ 0.5.

N.m.r. ($CDCl_3$): 2.72, s+t, (6H); 3.0—3.3, m, (3H); 5.24, br. t, (1H); 5.52, s, (2H); 5.98, t, (2H); 6.1—6.7, m, (8H); 6.60, s, (3H); 7.98, m, (2H).

The following compound was similarly prepared from the above aldehyde (A) and 1-heptylamine.

(1) A (558 mg) and 1-heptylamine (5 ml) gave 1-methyl-5-[[3-[3-[(1-heptylamino)methyl]phenoxy]-propyl]amino]-1H-1,2,4-triazole-3-methanol, as an oil (153 mg). T.l.c. Silica; methanol $R_f$ 0.3.

N.m.r. ($CDCl_3$): 2.80, t, (1H); 3.1—3.3, m, (3H); 5.50, s+m, (3H); 5.97, t, (2H); 6.30, s, (2H); 6.50, q, (2H); 6.58, s, (3H); 6.90, bs, (2H); 7.41, t, (2H); 7.96, m, (2H); 8.3—8.9, m, (10); 9.2, bt, (3H).

Example 39

N[5-[[4-[5-[(Dimethylamino)methyl]-2-furanyl]butyl]amino]-methyl-1H-1,2,4-triazol-3-yl]-N[1]-phenyl urea

*N[5]-[4-[5-[(Dimethylamino)methyl]-2-furanyl]butyl] 1-methyl-1H-1,2,4-triazole-3,5-diamine*

A mixture of N-cyano-1-methyl-2-(phenylmethylene)-hydrazine carboximidothioic acid methyl ester (2.32 g) and 4-[5-[(dimethylamino)methyl]-2-furanyl]butanamine (1.96 g) was heated at 60° under reduced water-pump pressure for 1 h. The residual oil was used without further purification, and stirred with 2N hydrochloric acid (10 ml) for 0.5 h. The pH of the acidic solution was adjusted to pH 9 with potassium carbonate, and washed with toluene. An excess of potassium carbonate was added to the aqueous phase which was then extracted with ethyl acetate. The organic extracts were dried and evaporated to give a solid which was recrystallised from ethyl acetate to give the *title compound* as a white solid (2.1 g), m.p. 105°C.

Using the method of Example 8:

N[5]-[4-[5-[(Dimethylamino)methyl]-2-furanyl]butyl]-1-methyl-1H-1,2,4-triazole-3,5-diamine (0.73 g) and phenylisocyanate (0.25 ml) gave N-[5-[[4-[5-[(dimethylamino)methyl]-2-furanyl]butyl]amino]-1-methyl-1H-1,2,4-triazol-3-yl]-N[1]-phenyl urea (0.67 g), m.p. 124—5°. T.l.c. system C. $R_f$ 0.42.

Example 40

The following compounds were prepared using the method of Example 22.

a) 4-[5-[(dimethylamino)methyl]-2-furanyl]butanamine (1.96 g) and methyl 1-methyl-N-phenyl-acetyl-2-(phenylmethylene)hydrazinecarboximidothioate (3.25 g) gave N-[4-[5-[(dimethylamino)methyl]-2-furanyl]butyl]-1-methyl-3-phenylmethyl-1H-1,2,4-triazole-5-amine (2.0 g). T.l.c. system B, $R_f$ 0.52.

N.m.r. ($CDCl_3$): 2.6—2.9, m, (5H); 3.98, d, (1H); 4.13, d, (1H); 5.80, t, (1H); 6.13, s, (2H); 6.13, s, (2H); 6.60—6.65, m, (7H); 7.38, t, (2H); 7.80, s, (6H); 8.3. m. (4H).

b) 4-[5-[(Dimethylamino)methyl]-2-furanyl]butanamine (1.1 g) and N-[2-(acetyloxy)acetyl]-1-methyl-2-(phenylmethylene)hydrazinecarboximidothioate (1.9 g) gave 5-[[4-[-[(dimethylamino)methyl]-2-furanyl]butyl]amino]-1-methyl-1H-1,2,4-triazole-3-methanol. (0.5 g), m.p. 88—90°. T.l.c. System C, $R_f$ 0.34.

Example 41

(a) 1-Methyl-5-[[3-[3-(1-piperidinylmethyl)phenoxy]propyl]amino-1H-1,2,4-triazole-3-methanol, sulphate (1:1)

1-Methyl-5-[[3-[3-(1-piperidinylmethyl)phenoxy]propyl]amino]—1H-1,2,4-triazole-3-methanol (300 mg) was dissolved in ethyl acetate (20 ml) with heating and the addition of a few drops of ethanol to give a clear solution.

A 0.45 ml aliquot of a hot solution of concentrated sulphuric acid (1 ml) in ethanol (9 ml) was added dropwise. The solid which separated on cooling and standing was filtered off, washed with diethyl ester and dried *in vacuo* to give the *title compound* as a white crystalline solid (400 mg) m.p. 170°.

In a similar manner:

(b) A solution of the free base (300 mg) as in (a) above was treated with a hot solution of tartaric acid (125 mg) in ethanol (10 ml) to give the *tartrate salt* (2:1) as a white crystalline solid )50 mg), m.p. 144°.

(c) A solution of free base (300 mg) as in (a) above was treated with hot solution of succinic acid (99 mg) in ethanol (10 ml) to give the *succinate salt* (2:1) as a white crystalline solid (150 mg). m.p. 137°.

Example 42

Following the method of Example 38

a) 5-[[3-(3-formylphenoxy)propyl]amino]-1-methyl-1H-1,2,4-triazole 3-methanol (430 mg) and morpholine (4 ml) gave 1-methyl-5-]]3-]3-(1-morpholinylmethyl) phenoxy]propyl]amino]-1H-1,2,4-triazole-3-methanol isolated as a salt with tartaric acid (1:2) (80 mg) m.p. 62—63°.

| | |
|---|---|
| Found: | C, 47.33: H, 5.97; N, 10.93; |
| $C_{18}H_{27}N_5O_3 \cdot 2C_4H_6O_6$ requires | C, 47.20; H, 5.90; N, 10.59% |

b) The above aldehyde (A) (430 mg) and heptamethyleneimine (4.48 g) gave 5-[[3-[3-[1-(heptamethyleneiminyl)methyl]phenoxy]propyl]amino]-1-methyl1H-1,2,4-triazole-3-methanol isolated as a salt with tartaric acid (1:1) (100 mg).
N.m.r. ($D_2O$): 2.5, t, (1H); 2.8—3.0, m, (3H); 5.52, s, (2H); 5.55, s, (2H); 5.72, s, (2H); 5.80, t, (2H); 6.48, s, (3H); 6.5, t, (2H); 6.7, m, (4H); 7.9, m, (2H); 8.0—8.5, m, (10H).

| | |
|---|---|
| Found: | C, 54.51; H, 7.13; N, 12.39; |
| $C_{21}H_{33}N_5O_2 \cdot C_4H_6O_6 \cdot H_2O$ requires | C, 54.1; H, 7.38; N, 12.61% |

c) The above aldehyde (A) (430 mg) and 1,2,5,6-tetrahydropyridine (4 ml) gave 1-methyl-5-[[3-[3-[1-(1,2,5,6-tetrahydropyridyl)methyl]phenoxy]propyl]amino] - 1H - 1,2,4 - triazole - 3 - methanol (50 mg) m.p. 86—90°, t.l.c. system B, $R_f$ 0.6.

Examples of Pharmaceutical compositions according to the invention are as follows:

| (a) TABLETS | mg/tablet | mg/tablet |
|---|---|---|
| Active ingredient | 20.0 | 40.0 |
| Microcrystalline cellulose BPC | 99.5 | 199.0 |
| Magnesium stearate B.P. | 0.5 | 1.0 |
| Compression weight | 120.0 | 240.0 |

The drug is sieved through a 250 $\mu$m sieve, blended with the excipients and compressed using 6.5 mm and 8.0 mm diameter punches for the 20 and 40 mg strengths respectively. Tablets of other strengths may be prepared by increasing the compression weight and using punches to suit.

The tablets may be film coated with suitable film forming materials, e.g. methyl cellulose, ethyl cellulose or hydroxypropylmethyl cellulose, using standard techniques. Alternatively the tablets may be sugar coated.

| (b) CAPSULES | mg/capsule |
|---|---|
| Active ingredient | 20.0 |
| **Sta-Rx 1500 Starch | 79.5 |
| Magnesium Streate B.P. | 0.5 |
| Fill weight | 100.0 |

**A form of directly compressible starch supplied by Colorcon Ltd., Orpington, Kent.

The active ingredient is sieved through a 250 $\mu$ sieve and blended with other materials. The mix is filled into No. 3. hard gelatin capsules using a suitable filling machine. Other doses may be prepared by increasing the fill weight and if necessary changing the capsule size to accommodate the increase.

| (c) SUSTAINED RELEASE TABLETS | mg/tablet |
|---|---|
| Active ingredient | 80 |
| *Cutina HR | 25 |
| Lactose B.P. | 142.5 |
| Magnesium Stearate B.P. | 2.5 |
| Compression weight | 250.0 |

*Cutina HR is a grade of microfine hydrogenated castor oil supplied by Sipon Products Ltd., London.

The drug is sieved through a 250 μ sieve and blended with the Cutina HR and lactose. The mixed powders are moistened with Industrial Methylated Spirits 74 O.P., granules are made, dried, screened and blended with the magnesium stearate. The lubricated granules are compressed using 8.5 mm punches to produce tablets with a hardness of not less than 10Kp (Schleuniger tester).

(d) INJECTION FOR INTRAVENOUS ADMINISTRATION

| | % w/v |
|---|---|
| Active ingredient | 0.25 |
| Water for Injections BP to | 100.00 |

Sodium chloride may be added to adjust the tonicity of the solution and the pH may be adjusted to that of maximum stability using either dilute acid or alkali.

The solution is prepared, clarified and filled under nitrogen into appropriate sized ampoules sealed by fusion of the glass. The injection is sterilised by heating in an autoclave using one of the acceptable cycles. Alternatively the solution may be sterilised by filtration and filled into sterile ampoules under aseptic conditions.

| (e) SYRUP | | mg/5 ml dose |
|---|---|---|
| Active ingredient | | 20.0 mg |
| Sucrose | | 2750.0 mg |
| Glycerine | | 500.0 mg |
| Buffer<br>Flavour<br>Colour<br>Preservative | as necessary | |
| Distilled water | to | 5.0 ml |

The active ingredient, buffer, flavour, preservative and colour are dissolved in some of the water. The remainder of the water is heated to approximately 80°C and the sucrose is dissolved in this and cooled. The two solutions are mixed, adjusted to volume and clarified by filtration.

The compounds of the invention have in general low toxicity at doses at which they effectively inhibit gastric acid secretion. Thus for instance the compounds listed below produced no toxic effects when administered intravenously to anaesthetised rats at doses of between 3 and 20 times their $ED_{50}$ values. Furthermore, an intravenous dose of 10 mg/kg of the compound of Example 2(b) produced no adverse effects in the anaesthetised dog.

The ability of the compounds of the invention to inhibit histamine induced gastric acid secretion has been demonstrated in the perfused rat stomach preparation referred to previously, the effectiveness of a compound conveniently being expressed in terms of its $ED_{50}$ (mg/kg) value. The results obtained for a representative number of compounds according to the invention are given below:—

| Example No. | $ED_{50}$ (mg/kg) |
|---|---|
| 1(b) | 0.06 |
| 2(b) | 0. 3 |
| 5 | 0.05 |
| 7 | 0.05 |
| 8(c) | 0.07 |
| 10(a) | 0.15 |
| 10(d) | 0.07 |
| 11 | 0.06 |
| 14 | 0.03 |
| 17 | 0.23 |
| 18 | 0.08 |

**Claims for the Contracting States: BE CH DE FR GB IT LU NL SE**

1. Compounds of the general formula (I)

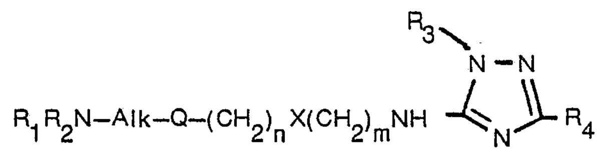

(I)

and physiologically acceptable salts and hydrates thereof, in which

$R_1$ represents hydrogen or $C_{1-4}$ alkyl, and $R_2$ represents $C_{1-10}$ alkyl, $C_{3-8}$ cycloalkyl, $C_{3-6}$ alkenyl, phenyl $C_{1-6}$ alkyl (where the phenyl group may be optionally substituted by one or more $C_{1-3}$ alkyl groups, $C_{1-3}$ alkoxy groups or halogen atoms), trifluoro $C_{1-6}$ alkyl, or $C_{1-6}$ alkyl substituted by hydroxy, $C_{1-6}$ alkoxy, amino, $C_{1-6}$ alkylamino or di $C_{1-6}$ alkylamino or $R_1$ and $R_2$ may together with the nitrogen atom to which they are attached form a 5 to 10 membered ring which may be saturated or may contain at least one double bond, may be unsubstituted or may be substituted by one or more $C_{1-3}$ alkyl groups or a hydroxy group and/or may contain another heteroatom which is oxygen or sulphur;

Alk represents a straight or branched alkylene chain of 1 to 6 carbon atoms;

Q represents a furan or thiophene ring in which incorporation into the rest of the molecule is through bonds at the 2- and 5- positions, the furan ring optionally bearing a further substituent $R_6$ adjacent to the group $R_1R_2N$—Alk-, or Q represents a benzene ring in which incorporation into the rest of the molecule is through bonds at the 1- and 3- or 1- and 4-positions;

$R_6$ represents halogen or $C_{1-4}$ alkyl which may be substituted by hydroxy or $C_{1-4}$ alkoxy;

X represents —$CH_2$—, —O—, —S— or

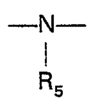

where $R_5$ represents hydrogen;

n represents zero, 1 or 2,

m represents 2, 3 or 4;

$R_3$ represents hydrogen, $C_{1-6}$ alkyl, $C_{3-6}$ alkenyl, phenyl $C_{1-6}$ alkyl (where the phenyl group may be optionally substituted by one or more $C_{1-3}$ alkyl groups, $C_{1-3}$ alkoxy groups or halogen atoms), hydroxy $C_{2-6}$ alkyl or $C_{1-6}$ alkoxy $C_{1-6}$ alkyl; and

$R_4$ represents hydrogen, $C_{1-6}$ alkyl, $C_{3-6}$ alkenyl, phenyl $C_{1-6}$ alkyl (where the phenyl group may be optionally substituted by one or more $C_{1-3}$ alkyl groups, $C_{1-3}$ alkoxy groups or halogen atoms), hydroxy $C_{1-6}$ alkyl, acyloxy $C_{1-6}$ alkyl (where the acyl portion is benzoyl, phenyl $C_{2-7}$ alkanoyl or $C_{1-6}$ alkanoyl, in the first two cases the phenyl ring being optionally substituted by one or more $C_{1-3}$ alkyl groups, $C_{1-3}$ alkoxy groups or halogen atoms), $C_{1-6}$ alkoxy $C_{1-6}$ alkyl, phenoxy $C_{1-6}$ alkyl (where the phenyl group

may be optionally substituted by one or more $C_{1-3}$ alkyl groups, $C_{1-3}$ alkoxy groups or halogen atoms), phenyl $C_{1-6}$ alkyloxy, $C_{1-6}$ alkyl (where the phenyl group may be optionally substituted by one or more $C_{1-3}$ alkyl groups, $C_{1-3}$ alkoxy groups, or halogen atoms), amino $C_{1-6}$ alkyl, $C_{1-6}$ alkylamino $C_{1-6}$ alkyl, di $C_{1-6}$ alkylamino $C_{1-6}$ alkyl, hydroxy, $C_{1-6}$ alkoxy or $C_{1-6}$ alkylthio, or the group $NR_7R_8$ where

$R_7$ represents hydrogen or $C_{1-6}$ alkyl and

$R_8$ represents the group $COR_9$ where $R_9$ represents hydrogen, $C_{1-6}$ alkyl, phenyl or phenyl $C_{1-6}$ alkyl (where in both cases, the phenyl group may be optionally substituted by one or more $C_{1-3}$ alkyl groups, $C_{1-3}$ alkoxy groups or halogen atoms), $C_{1-6}$ alkoxy, a 5 or 6 membered monocyclic heteroaryl group containing one or more heteroatoms selected from oxygen, nitrogen and sulphur, or a heteroaryl $C_{1-6}$ alkyl group where the heteroaryl group is a 5 or 6 membered monocyclic ring containing one or more heteroatoms selected from oxygen, nitrogen and sulphur, or

$R_8$ represents the group $SO_2R_{10}$ where $R_{10}$ represents $C_{1-6}$ alkyl or phenyl (optionally substituted by one or more $C_{1-3}$ alkyl groups, $C_{1-3}$ alkoxy groups or halogen atoms), or $R_8$ represents the group

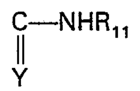

where Y is oxygen or sulphur and $R_{11}$ represents hydrogen, $C_{1-6}$ alkyl, $C_{3-8}$ cycloalkyl, phenyl or phenyl $C_{1-6}$ alkyl (where, in both cases, the phenyl group may be optionally substituted by one or more $C_{1-3}$ alkyl groups, $C_{1-3}$ alkoxy groups or halogen atoms).

2. Compounds as claimed in Claim 1 in the form of a hydrochloride, hydrobromide, sulphate, methanesulphonate, acetate, maleate, succinate, citrate, fumarate, benzoate or tartrate salt.

3. Compounds as claimed in Claim 1 in which:

$R_4$ represents the group $NR_7R_8$ where $R_7$ and $R_8$ are as defined in Claim 1 except that $R_9$ is other than a heteroaryl or heteroarylalkyl group and $R_{11}$ is other than $C_{3-8}$ cycloalkyl; or $R_4$ represents hydrogen, $C_{1-6}$ alkyl, $C_{3-6}$ alkenyl, phenyl $C_{1-6}$ alkyl (where the phenyl group may be optionally substituted by one or more $C_{1-3}$ alkyl groups, $C_{1-3}$ alkoxy groups or halogen atoms), hydroxy $C_{1-6}$ alkyl, $C_{1-6}$ alkoxy $C_{1-6}$ alkyl, phenoxy $C_{1-6}$ alkyl (where the phenyl group may be optionally substituted by one or more $C_{1-3}$ alkyl groups, $C_{1-3}$ alkoxy groups or halogen atoms), amino $C_{1-6}$ alkyl, $C_{1-6}$ alkylamino $C_{1-6}$ alkyl, di $C_{1-6}$ alkylamino $C_{1-6}$ alkyl, hydroxy or $C_{1-6}$ alkoxy;

provided that when X represents an oxygen atom or $—NR_5—$ and when n is zero then Q represents benzene.

4. Compounds as claimed in any of Claims 1 to 3, wherein Q represents benzene incorporated into the molecule through bonds at the 1- and 3- positions, n is zero and X is oxygen.

5. Compounds as claimed in Claim 4, wherein $R_4$ represents hydrogen, $C_{1-6}$ alkyl, hydroxy $C_{1-6}$ alkyl, $C_{1-6}$ alkoxy $C_{1-6}$ alkyl, acyloxy $C_{1-6}$ alkyl, phenyl $C_{1-6}$ alkyl (where the phenyl group may be optionally substituted by one or more $C_{1-3}$ alkyl groups, $C_{1-3}$ alkoxy groups or halogen atoms) or hydroxy or the group $NHCOR_9$ where $R_9$ represents hydrogen, $C_{1-6}$ alkyl, $C_{1-6}$ alkoxy, phenyl (optionally substituted by one or more $C_{1-3}$ alkyl groups, $C_{1-3}$ alkoxy groups or halogen atoms), or a 5 or 6 membered monocyclic heteroaryl group containing one or more heteroatoms selected from oxygen, nitrogen and sulphur; or the group $NHCONHR_{11}$ where $R_{11}$ represents $C_{1-6}$ alkyl, phenyl (optionally substituted by one or more $C_{1-3}$ alkyl groups, $C_{1-3}$ alkoxy groups or halogen atoms) or $C_{3-8}$ cycloalkyl.

6. Compounds as claimed in Claim 5 wherein $R_4$ represents hydroxy $C_{1-6}$ alkyl, hydroxy, $C_{1-6}$ alkoxy $C_{1-6}$ alkyl, benzyl or the group $NHCOR_9$ where $R_9$ is hydrogen, phenyl, $C_{1-6}$ alkyl or $C_{1-6}$ alkoxy, or the group $NHCONHR_{11}$ where $R_{11}$ is phenyl.

7. Compounds as claimed in any of Claims 1 to 4, wherein $R_4$ represents hydroxy $C_{1-6}$ alkyl, acyloxy $C_{1-6}$ alkyl or $C_{1-6}$ alkoxy $C_{1-6}$ alkyl.

8. Compounds as claimed in Claim 7 wherein $R_4$ represents hydroxy $C_{1-6}$ alkyl.

9. Compounds as claimed in any of Claims 1 to 8, wherein m+n is 3 or 4.

10. Compounds as claimed in any of Claims 1 to 9, in which Alk is $—CH_2—$.

11. Compounds as claimed in any of Claims 1 to 10, wherein $R_1$ represents hydrogen or $C_{1-4}$ alkyl and $R_2$ represents $C_{3-5}$ alkenyl, $C_{5-7}$ cycloalkyl, benzyl, $C_{1-8}$ alkyl or $C_{1-4}$ alkyl substituted by $C_{1-3}$ alkoxy, hydroxy, di-$C_{1-3}$ alkylamino or trifluoromethyl or $R_1$ and $R_2$ together with the nitrogen atom to which they are attached form a ring with 5 to 8 members and optionally containing one double bond and/or substituted by hydroxy or one or two methyl group(s).

12. Compounds as claimed in Claim 11 wherein $R_1$ and $R_2$ represent $C_{1-3}$ alkyl, or $R_1$ and $R_2$ together with the nitrogen atom to which they are attached form a heterocyclic ring which is pyrrolidine, piperidine optionally substituted in the 4-position by methyl or hydroxy, tetrahydropyridine, morpholine, 2,6-dimethylmorpholine, hexamethyleneimine or heptamethyleneimine.

13. Compounds as claimed in any of Claims 1 to 12, wherein $R_3$ represents hydrogen, $C_{1-6}$ alkyl or hydroxy $C_{2-4}$ alkyl.

14. Compounds as claimed in Claim 1 or 2, corresponding to the formula (II)

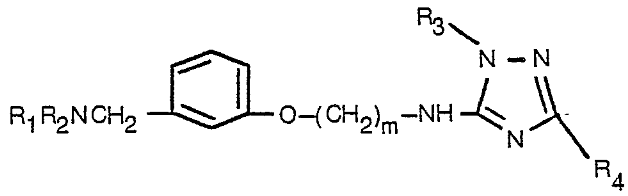

(II)

where $R_1$ and $R_2$ are methyl groups or together with the nitrogen atom to which they are attached form a pyrrolidino, piperidino or hexamethyleneimino group; m is 3 or 4; $R_3$ is hydrogen or methyl, and $R_4$ is a hydroxy $C_{1-6}$ alkyl, $C_{1-6}$ alkoxy $C_{1-6}$ alkyl, benzyl, formamido, $C_{2-7}$ alkanoylamino, $C_{1-6}$ alkoxycarbonylamino, hydroxy, benzoylamino or phenylcarbamoylamino group.

15. The compound claimed in Claim 1 or 2, which is 1-methyl-5-[[3-[3-(1-piperidinylmethyl)-phenoxy]propyl]amino]-1H-1,2,4-triazole-3-methanol and physiologically acceptable salts thereof.

16. The succinate salt of 1-methyl-5-[[3-[3-(1-piperidinylmethyl)phenoxy]propyl]amino]-1H-1,2,4-triazole-3-methanol.

17. The compound claimed in Claim 1 or 2, which is 5-[[2-[[[5-[(dimethylamino)methyl]-2-furanyl]methyl]thio]ethyl]amino] - 1 - methyl - 1H - 1,2,4 - triazole - 3 - methanol and physiologically acceptable salts thereof.

18. The compound claimed in Claim 1 or 2, which is 5-[[2-[[[5-[(dimethylamino)methyl]-4-methyl-2-furanyl]methyl]thio]ethyl]amino]-1-methyl-1H-1,2,4-triazole-3-methanol and physiologically acceptable salts thereof.

19. The compound claimed in Claim 1 or 2, which is 1-methyl-5-[[3-[3-[(dimethylamino)methyl]-phenoxy]propyl]amino]-1H-1,2,4-triazole-3-methanol and physiologically acceptable salts thereof.

20. The compound claimed in Claim 1 or 2, which is 5-[[3-[3-(piperidinylmethyl)phenoxy]propyl]-amino]-1H-1,2,4-triazole-3-one and physiologically acceptable salts thereof.

21. Compounds as claimed in Claim 1 or 2 which are:
1-methyl-5-[[4-[3-(1-piperidinylmethyl)phenoxy]butyl]amino]-1H-1,2,4-triazole-3-methanol,
N-[1-methyl-5-[[3-[3-(1-piperidinylmethyl)phenoxy]propyl]amino]-1H-1,2,4-triazole-3-yl]formamide
3-methoxymethyl-1-methyl-5-[[4-[3-(1-piperidinylmethyl)phenoxy]butyl]amino]-1H-1,2,4-triazole and physiologically acceptable salts thereof.

22. Compounds as claimed in Claim 1 or 2 which are:
3-phenylmethyl-N-[3-[3-(dimethylaminomethyl)phenoxy]propyl]-1H-1,2,4-triazole-5-amine,
5-[4-[3-(1-piperidinylmethyl)phenoxy]butyl]amino-1H-1,2,4-triazole-3-methanol,
3-phenylmethyl-N-[3-[3-(1-piperidinylmethyl)phenoxy]propyl]-1H-1,2,4-triazole-5-amine,
ethyl 1-methyl-5-[[3-[3-[(dimethylamino)methyl]phenoxy]propyl]amino]-1H-1,2,4-triazole-3-carbamate,
N-[5-[[3-[3-[(dimethylamino)methyl]phenoxy]propyl]amino]-1-methyl-1H-1,2,4-triazole-3-yl]benzamide,
N-[5-[[3-[3-(1-piperidinylmethyl)phenoxy]propyl]amino]-1-methyl-1H-1,2,4-triazole-3-yl]-N'-phenylurea and physiologically acceptable salts thereof.

23. A process for the production of compounds as claimed in Claim 1 or 2 which comprises:

(a) for the production of compounds in which $R_4$ represents a hydrogen atom, treating a diazonium salt (III)

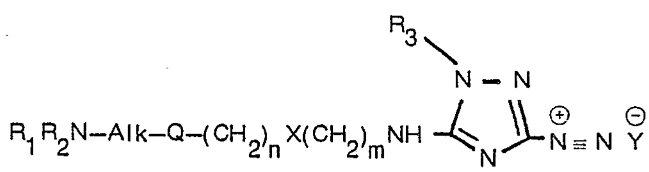

(III)

in which $R_1$, $R_2$ and $R_3$ are as defined in formula (I) or are groups convertible thereto, Alk, Q, n, X and m are as defined in formula (I) and $\overset{\ominus}{Y}$ is the anion corresponding to the acid used in the diazotization reaction, with a concentrated mineral acid to convert the group $-\overset{\oplus}{N}\equiv N\ \overset{\ominus}{Y}$ into a hydrogen atom;

(b) for the production of compounds in which $R_4$ represents the group $NR_7R_8$ where $R_8$ represents $COR_9$, $SO_2R_{10}$ or $C(=Y)NHR_{11}$, treating an amino triazole (IV)

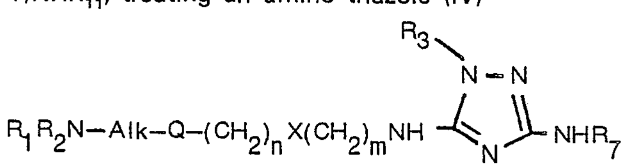

(IV)

in which $R_1$, $R_2$, $R_3$ and $R_7$ are as defined in formula (I) or are groups readily convertible thereto, with an

activated derivative of either a carboxylic acid $R_9COOH$ or a sulphonic acid $R_{10}SO_3H$, where $R_9$ and $R_{10}$ are as defined in formula (I), or with an isocyanate or isothiocyanate $R'_{11}NCY$, in which $R'_{11}$ has any of the meanings defined for $R_{11}$ in formula (I) except hydrogen or represents an alkali metal atom or an alkoxycarbonyl group, to replace the hydrogen atom in the group $NHR_7$ by the group $COR_9$, $SO_2R_{10}$ or $C(=Y)NHR_{11}$ respectively;

(c) for the production of compounds in which $R_4$ is other than an alkoxy, alkylthio or acyloxyalkyl group, cyclising a compound of formula (V)

$$R_1R_2N—Alk—Q—(CH_2)_nX(CH_2)_mNH—\underset{\underset{V'}{\|}}{\overset{\overset{R_3}{|}}{C}}—NNHY' \qquad (V)$$

in which $R_1$, $R_2$, Alk, Q, X, $R_3$, n and m are as defined in formula (I) and V' is

$$\underset{V}{\overset{NCR_4'}{\|}}$$

and Y' is hydrogen where V is oxygen or sulphur and $R_4'$ is a group as defined for $R_4$ or a group convertible thereto under the conditions of the cyclisation reaction or represents halogen or alkoxy; or V' is NH and Y' is

$$\underset{Y}{\overset{CR_4'}{\|}}$$

where Y is sulphur, oxygen or NH, except that when $R_4'$ is halogen or alkoxy Y cannot be NH, or V' is sulphur or oxygen and Y' is

$$\underset{NH}{\overset{CR_{4'}}{\|}}$$

with optional protection and subsequent deprotection of any reactive groups in the starting material if desired;

(d) reducing a compound of formula (XV)

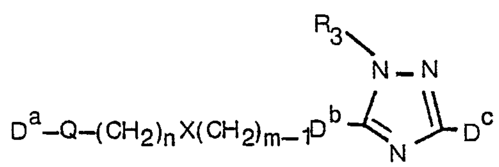

(XV)

in which Q, n, X, m and $R_3$ are as defined in formula (I), at least one of $D^a$, $D^b$ and $D^c$ represents a reducible group and the other(s) take the appropriate meaning corresponding to formula (I),

$D^a$ represents $R_1R_2NAlk$- or a group convertible thereto under reducing conditions where $R_1$, $R_2$ and Alk are as defined in formula (I);

$D^b$ represents —$CH_2NH$—, —CONH— or —CH=N—

$D^c$ represents $R_4$ as defined in formula (I) or a group convertible thereto under reducing conditions, with optional protection and subsequent deprotection of any reactive groups in the starting material if desired;

(e) reacting a compound of formula (XX)

$$R_1R_2NAlkQE \qquad (XX)$$

in which E represents $(CH_2)_nX(CH_2)_mP$ or $CH_2P'$ where P and P' are leaving groups, with a triazole of the formula (XVII)

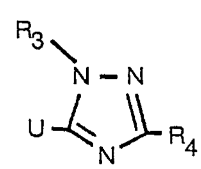

(XVII)

where U represents amino, HS $(CH_2)_mNH$ or HO $(CH_2)_mNH$, and $R_4$ is as defined in formula (I) or is a

group convertible thereto with optional protection and subsequent deprotection of any reactive groups in the starting material if desired;

(f) introducing the group $R_1R_2N$-Alk into the group Q present in a suitable intermediate by

(i) for the production of compounds in which Q is furan or substituted furan and Alk is methylene, reacting the intermediate with formaldehyde and an amine $R_1R_2NH$ or a salt thereof (where $R_1$ and $R_2$ are as defined in formula (I)) or

(ii) for the production of compounds in which $R_1$ and $R_2$ both represent methyl, Alk is methylene and Q is furan, substituted furan or thiophene, reacting the intermediate with the compound $(CH_3)_2\overset{\oplus}{N}=CH_2\overset{\ominus}{Cl}$, with in both cases optional protection and subsequent deprotection of any reactive groups in the starting material if desired;

(g) for the production of compounds in which $R_4$ is a secondary or tertiary hydroxyalkyl group, treating the corresponding compound where $R_4$ is the group $(CH_2)_{q-1}$ CHO, $(CH_2)_{q-1}$ $CO_2R_{12}$ or $(CH_2)_rCOR_{12}$ in which q is an integer from 1 to 6 inclusive, r is 0 to 4 and $R_{12}$ is an alkyl group, with an organometallic derivative;

(h) reacting a compound of formula (XXII)

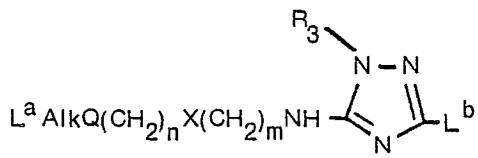

(XXII)

where either $L^a$ is $R_1R_2N$ and $L^b$ is the group $(CH_2)_q$ $L^c$ or $L^a$ is a group $L^d$ and $L^b$ is $R_4$, where $L^c$ and $L^d$ are leaving groups and q is an integer from 1 to 6 inclusive, with a compound capable of replacing $L^d$ by $R_1R_2N$ or $L^c$ so as to convert $L^b$ into the group $R^4$ with optional protection and subsequent deprotection of any reactive groups in the starting material if desired;

(i) for the production of compounds in which $R_4$ is an alkoxyalkyl group, converting the group $R_4$ in an intermediate in which this group is hydroxyalkyl into an alkoxyalkyl group by standard methods of interconversion;

(j) for the production of compounds in which $R_4$ is an acyloxyalkyl group, treating the corresponding hydroxyalkyl compound with an appropriate acid at elevated temperature; or

(k) for the production of compounds in which $R_4$ is a hydroxyalkyl group removing a hydroxyl protecting group from the hydroxyl group;

and where the compound of formula (I) is produced in the form of a free base, optionally converting the free base into a salt.

24. A pharmaceutical composition comprising a compound as claimed in any of Claims 1 to 22 together with at least one pharmaceutically acceptable carrier or diluent, and optionally one or more further active ingredients.

25. A pharmaceutical composition as claimed in Claim 24 in a form adapted for oral administration.

26. A pharmaceutical composition as claimed in Claim 25 containing 5 mg to 1 g of the compound of formula (I).

27. A pharmaceutical composition as claimed in Claim 26 containing 5 mg to 250 mg of the compound of formula (I).

**Claims for the Contracting State: AT**

1. A process for the preparation of compounds of the general formula (I)

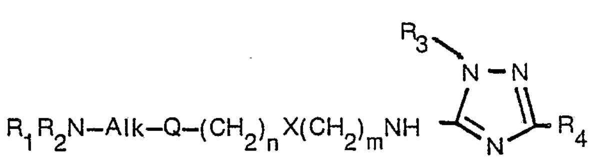

(I)

and physiologically acceptable salts and hydrates thereof, in which

$R_1$ represents hydrogen or $C_{1-4}$ alkyl, and $R_2$ represents $C_{1-10}$ alkyl, $C_{3-8}$ cycloalkyl, $C_{3-6}$ alkenyl, phenyl $C_{1-6}$ alkyl (where the phenyl group may be optionally substituted by one or or more $C_{1-3}$ alkyl groups, $C_{1-3}$ alkoxy groups or halogen atoms), trifluoro $C_{1-6}$ alkyl, or $C_{1-6}$ alkyl substituted by hydroxy, $C_{1-6}$ alkoxy, amino, $C_{1-6}$ alkylamino or di $C_{1-6}$ alkylamino or $R_1$ and $R_2$ may together with the nitrogen atom to which they are attached form a 5 to 10 membered ring which may be saturated or may contain at least one double bond, may be unsubstituted or may be substituted by one or more $C_{1-3}$ alkyl groups or a hydroxy group and/or may contain another heteroatom which is oxygen or sulphur;

Alk represents a straight or branched alkylene chain of 1 to 6 carbon atoms;

Q represents a furan or thiophene ring in which incorporation into the rest of the molecule is through bonds at the 2- and 5- positions, the furan ring optionally bearing a further substituent $R_6$ adjacent to the group $R_1R_2N$—Alk-, or Q represents a benzene ring in which incorporation into the rest of the molecule is through bonds at the 1- and 3- or 1- and 4-positions;

$R_6$ represents halogen or $C_{1-4}$ alkyl which may be substituted by hydroxy or $C_{1-4}$ alkoxy;

X represents —$CH_2$—, —O—, —S— or

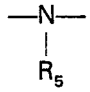

where $R_5$ represents hydrogen;

n represents zero, 1 or 2,

m represents 2, 3 or 4;

$R_3$ represents hydrogen, $C_{1-6}$ alkyl, $C_{3-6}$ alkenyl, phenyl $C_{1-6}$ alkyl (where the phenyl groups may be optionally substituted by one or more $C_{1-3}$ alkyl groups, $C_{1-3}$ alkoxy groups or halogen atoms), hydroxy $C_{2-6}$ alkyl or $C_{1-6}$ alkoxy $C_{1-6}$ alkyl; and

$R_4$ represents hydrogen, $C_{1-6}$ alkyl, $C_{3-6}$ alkenyl, phenyl $C_{1-6}$ alkyl (where the phenyl group may be optionally substituted by one or more $C_{1-3}$ alkyl groups, $C_{1-3}$ alkoxy groups or halogen atoms), hydroxy $C_{1-6}$ alkyl, acyloxy $C_{1-6}$ alkyl (where the acyl portion is benzoyl, phenyl $C_{2-7}$ alkanoyl or $C_{1-6}$ alkanoyl, in the first two cases the phenyl ring being optionally substituted by one or more $C_{1-3}$ alkyl groups, $C_{1-3}$ alkoxy groups or halogen atoms), $C_{1-6}$ alkoxy $C_{1-6}$ alkyl, phenoxy $C_{1-6}$ alkyl (where the phenyl group may be optionally substituted by one or more $C_{1-3}$ alkyl groups, $C_{1-3}$ alkoxy groups or halogen atoms), phenyl $C_{1-6}$ alkyloxy $C_{1-6}$ alkyl (where the phenyl group may be optionally substituted by one or more $C_{1-3}$ alkyl groups, $C_{1-3}$ alkoxy groups or halogen atoms), amino $C_{1-6}$ alkyl, $C_{1-6}$ alkylamino $C_{1-6}$ alkyl, di $C_{1-6}$ alkylamino $C_{1-6}$ alkyl, hydroxy, $C_{1-6}$ alkoxy or $C_{1-6}$ alkylthio, or the group $NR_7R_8$ where

$R_7$ represents hydrogen or $C_{1-6}$ alkyl and

$R_8$ represents the group $COR_9$ where $R_9$ represents hydrogen, $C_{1-6}$ alkyl, phenyl or phenyl $C_{1-6}$ alkyl (where in both cases, the phenyl group may be optionally substituted by one or more $C_{1-3}$ alkyl groups, $C_{1-3}$ alkoxy groups or halogen atoms), $C_{1-6}$ alkoxy, a 5 to 6 membered monocyclic heteroaryl group containing one or more heteroatoms selected from oxygen, nitrogen and sulphur, or a heteroaryl $C_{1-6}$ alkyl group where the heteroaryl group is a 5 or 6 membered monocyclic ring containing one or more heteroatoms selected from oxygen, nitrogen and sulphur, or

$R_8$ represents the group $SO_2R_{10}$ where $R_{10}$ represents $C_{1-6}$ alkyl or phenyl (optionally substituted by one or more $C_{1-3}$ alkyl groups, $C_{1-3}$ alkoxy groups or halogen atoms), or $R_8$ represents the group

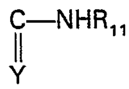

where Y is oxygen or sulphur and $R_{11}$ represents hydrogen $C_{1-6}$ alkyl, $C_{3-8}$ cycloalkyl, phenyl or phenyl $C_{1-6}$ alkyl (where, in both cases, the phenyl group may be optionally substituted by one or more $C_{1-3}$ alkyl groups, $C_{1-3}$ alkoxy groups or halogen atoms), which comprises:

(a) for the production of compounds in which $R_4$ represents a hydrogen atom, treating a diazonium salt (III)

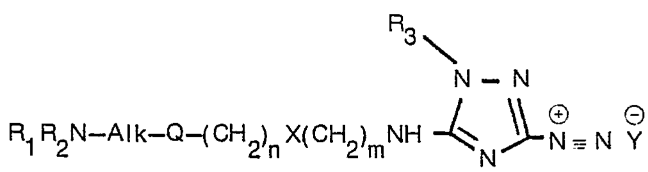

(III)

in which $R_1$, $R_2$ and $R_3$ are as defined in formula (I) or are groups convertible thereto, Alk, Q, n, X and m are as defined in formula (I) and $\overset{\ominus}{Y}$ is the anion corresponding to the acid used in the diazotization reaction, with a concentrated mineral acid to convert the group $-\overset{\oplus}{N}{\equiv}N\ \overset{\ominus}{Y}$ into a hydrogen atom;

(b) for the production of compounds in which $R_4$ represents the group $NR_7R_8$ where $R_8$ represents $COR_9$, $SO_2R_{10}$ or $C(=Y)NHR_{11}$, treating a amino triazole (IV)

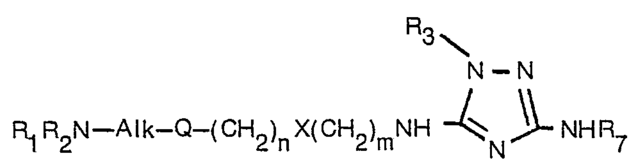

(IV)

in which $R_1$, $R_2$, $R_3$ and $R_7$ are as defined in formula (I) or are groups readily convertible thereto, with an activated derivative of either a carboxylic acid $R_9COOH$ or a sulphonic acid $R_{10}SO_3H$, where $R_9$ and $R_{10}$ are as defined in formula (I), or with an isocyanate or isothiocyanate $R'_{11}NCY$, in which $R'_{11}$ has any of the meanings defined for $R_{11}$ in formula (I) except hydrogen or represents an alkali metal atom or an alkoxycarbonyl group, to replace the hydrogen atom in the group $NHR_7$ by the group $COR_9$, $SO_2R_{10}$ or $C(=Y)NHR_{11}$ respectively;

(c) for the production of compounds in which $R_4$ is other than an alkoxy, alkylthio or acyloxyalkyl group, cyclising a compound of formula (V)

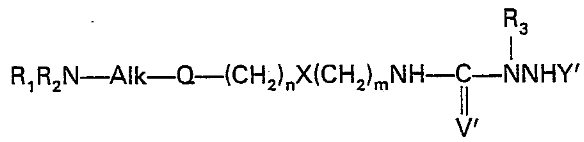
(V)

in which $R_1$, $R_2$, Alk, Q, X, $R_3$, n and m are as defined in formula (I) and V' is

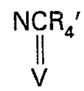

and Y' is hydrogen where V is oxygen or sulphur and $R'_4$ is a group as defined for $R_4$ or a group convertible thereto under the conditions of the cyclisation reaction or represents halogen or alkoxy; or V' is NH and Y' is

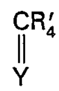

where Y is sulphur, oxygen or NH, except that when $R'_4$ is halogen or alkoxy Y cannot be NH, or V' is sulphur or oxygen and Y' is

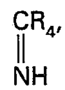

with optional protection and subsequent deprotection of any reactive groups in the starting material if desired;

(d) reducing the compound of formula (XV)

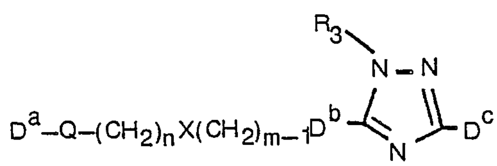
(XV)

in which Q, n, X, m and $R_3$ are as defined in formula (I), at least one of $D^a$, $D^b$ and $D^c$ represents a reducible group and the other(s) take the appropriate meaning corresponding to formula (I),

$D^a$ represents $R_1R_2NAlk$- or a group convertible thereto under reducing conditions where $R_1$, $R_2$ and Alk are as defined in formula (I);

$D^b$ represents $-CH_2NH-$, $-CONH-$ or $-CH=N-$

$D^c$ represents $R_4$ as defined in formula (I) or a group convertible thereto under reducing conditions, with optional protection and subsequent deprotection of any reactive groups in the starting material if desired;

(e) reacting a compound of formula (XX)

$$R_1R_2NAlkQE \qquad (XX)$$

in which E represents $(CH_2)_nX(CH_2)_mP$ or $CH_2P'$ where P and P' are leaving groups, with a triazole of the formula (XVII)

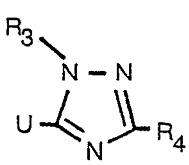
(XVII)

where U represents amino, HS $(CH_2)_mNH$ or HO $(CH_2)_mNH$, and $R_4$ is as defined in formula (I) or is a

group convertible thereto with optional protection and subsequent deprotection of any reactive groups in the starting material if desired;

(f) introducing the group $R_1R_2N$-Alk into the group Q present in a suitable intermediate by

(i) for the production of compounds in which Q is furan or substituted furan and Alk is methylene, reacting the intermediate with formaldehyde and an amine $R_1R_2NH$ or a salt thereof (where $R_1$ and $R_2$ are as defined in formula (I)) or

(ii) for the production of compounds in which $R_1$ and $R_2$ both represent methyl, Alk is methylene and Q is furan, substituted furan or thiophene, reacting the intermediate with the compound $(CH_3)_2\overset{\oplus}{N}=CH_2\overset{\ominus}{Cl}$, with in both cases optional protection and subsequent deprotection of any reactive groups in the starting material if desired;

(g) for the production of compounds in which $R_4$ is a secondary or tertiary hydroxyalkyl group, treating the corresponding compound where $R_4$ is the group $(CH_2)_{q-1}$ CHO, $(CH_2)_{q-1}$ $CO_2R_{12}$ or $(CH_2)_rCOR_{12}$ in which q is an integer from 1 to 6 inclusive, r is 0 to 4 and $R_{12}$ is an alkyl group, with an organometallic derivative;

(h) reacting a compound of formula (XXII)

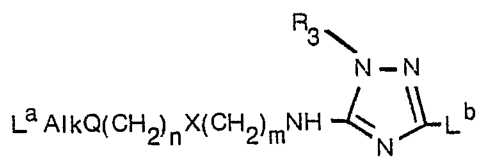

(XXII)

where either $L^a$ is $R_1R_2N$ and $L^b$ is the group $(CH_2)_q$ $L^c$ or $L^a$ is a group $L^d$ and $L^b$ is $R_4$, where $L^c$ and $L^d$ are leaving groups and q is an integer from 1 to 6 inclusive, with a compound capable of replacing $L^d$ by $R_1R_2N$ or $L^c$ so as to convert $L^b$ into the group $R_4$ with optional protection and subsequent deprotection of any reactive groups in the starting material if desired;

(i) for the production of compounds in which $R_4$ is an alkoxyalkyl group, converting the group $R_4$ in an intermediate in which this group is hydroxyalkyl into an alkoxyalkyl group by standard methods of interconversion;

(j) for the production of compounds in which $R_4$ is an acyloxyalkyl group, treating the corresponding hydroxyalkyl compound with an appropriate acid at elevated temperature; or

(k) for the production of compounds in which $R_4$ is a hydroxyalkyl group removing a hydroxyl protecting group from the hydroxyl group;

and where the compound of formula (I) is produced in the form of a free base, optionally converting the free base into a salt.

2. A process as claimed in claim 1 for the production of compounds in the form of a hydrochloride, hydrobromide, sulphate, methanesulphonate, acetate, maleate, succinate, citrate, fumarate, benzoate or tartrate salt.

3. A process as claimed in Claim 1 for the production of compounds in which

$R_4$ represents the group $NR_7R_8$ where $R_7$ and $R_8$ are as defined in Claim 1 except that $R_9$ is other than a heteroaryl or heteroarylalkyl group and $R_{11}$ is other than $C_{3-8}$ cycloalkyl or $R_4$ represents hydrogen, $C_{1-6}$ alkyl, $C_{3-6}$ alkenyl, phenyl $C_{1-6}$ alkyl (where the phenyl group may be optionally substituted by one or more $C_{1-3}$ alkyl groups, $C_{1-3}$ alkoxy groups or halogen atoms), hydroxy $C_{1-6}$ alkyl, $C_{1-6}$ alkoxy $C_{1-6}$ alkyl, phenoxy, $C_{1-6}$ alkyl (where the phenyl group may be optionally substituted by one or more $C_{1-3}$ alkyl groups, $C_{1-3}$ alkoxy groups or halogen atoms), amino $C_{1-6}$ alkyl, $C_{1-6}$ alkylamino $C_{1-6}$ alkyl, di $C_{1-6}$ alkylamino $C_{1-6}$ alkyl, hydroxy or $C_{1-6}$ alkoxy;

provided that when X represents an oxygen atom or —$NR_5$— and when n is zero then Q represents benzene.

4. A process as claimed in any of Claims 1 to 3, for the production of compounds in which Q represents benzene incorporated into the molecule through bonds at the 1- and 3-positions, n is zero and X is oxygen.

5. A process as claimed in Claim 4 for the production of compounds in which $R_4$ represents hydrogen, $C_{1-6}$ alkyl, hydroxy $C_{1-6}$ alkyl, $C_{1-6}$ alkoxy $C_{1-6}$ alkyl, acyloxy $C_{1-6}$ alkyl, phenyl $C_{1-6}$ alkyl (where the phenyl group may be optionally substituted by one or more $C_{1-3}$ alkyl groups, $C_{1-3}$ alkoxy, groups or halogen atoms) or hydroxy or the group $NHCOR_9$ where $R_9$ represents hydrogen, $C_{1-6}$ alkyl, $C_{1-6}$ alkoxy, phenyl (optionally substituted by one or more $C_{1-3}$ alkyl groups, $C_{1-3}$ alkoxy groups or halogen atoms), or a 5 or 6 membered monocyclic heteroaryl group containing one or more heteroatoms selected from oxygen, nitrogen and sulphur; or the group $NHCONHR_{11}$ where $R_{11}$ represents $C_{1-6}$ alkyl, phenyl (optionally substituted by one or more $C_{1-3}$ alkyl groups, $C_{1-3}$ groups or halogen atoms) or $C_{3-8}$ cycloalkyl.

6. A process as claimed in Claim 5 for the production of compounds in which $R_4$ represents hydroxy $C_{1-6}$ alkyl, hydroxy, $C_{1-6}$ alkoxy $C_{1-6}$ alkyl, benzyl or the group $NHCOR_9$ where $R_9$ is hydrogen, phenyl $C_{1-6}$ alkyl or $C_{1-6}$ alkoxy, or the group $NHCONHR_{11}$ where $R_{11}$ is phenyl.

7. A process as claimed in any of Claims 1 to 4, for the production of compounds in which $R_4$ represents hydroxy $C_{1-6}$ alkyl, acyloxy $C_{1-6}$ alkyl or $C_{1-6}$ alkoxy $C_{1-6}$ alkyl.

8. A process as claimed in Claim 7 for the production of compounds in which $R_4$ represents hydroxy $C_{1-6}$ alkyl.

9. A process as claimed in any of Claims 1 to 8, for the production of compounds in which m+n is 3 or 4.

10. A process as claimed in any of Claims 1 to 9, for the production of compounds in which Alk is $—CH_2—$.

11. A process as claimed in any of Claims 1 to 10, for the production of compounds in which $R_1$ represents hydrogen of $C_{1-4}$ alkyl and $R_2$ represents $C_{3-5}$ alkenyl, $C_{5-7}$ cycloalkyl, benzyl, $C_{1-8}$ alkyl or $C_{1-4}$ alkyl substituted by $C_{1-3}$ alkoxy, hydroxy, di-$C_{1-3}$ alkylamino or trifluoromethyl or $R_1$ and $R_2$ together with the nitrogen atom to which they are attached form a ring with 5 to 8 members and optionally containing one double bond and/or substituted by hydroxy or one or two methyl group(s).

12. A process as claimed in claim 11 for the production of compounds in which $R_1$ and $R_2$ represent $C_{1-3}$ alkyl, or $R_1$ and $R_2$ together with the nitrogen atom to which they are attached form a heterocyclic ring which is pyrrolidine, piperidine optionally substituted in the 4-position by methyl or hydroxy, tetrahydropyridine, morpholine, 2,6-dimethylmorpholine, hexamethyleneimine or heptamethyleneimine.

13. A process as claimed in any of Claims 1 to 12 for the production of compounds in which $R_3$ represents hydrogen, $C_{1-6}$ alkyl or hydroxy $C_{2-4}$ alkyl.

14. A process as claimed in claim 1 or 2, for the production of compounds corresponding to the formula (II)

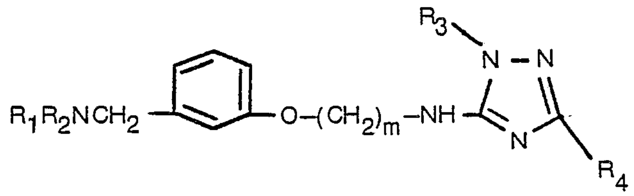

(II)

where $R_1$ and $R_2$ are methyl groups or together with the nitrogen atom to which they are attached form a pyrrolidino, piperidino or hexamethyleneimino group; m is 3 or 4; $R_3$ is hydrogen or methyl, and $R_4$ is a hydroxy $C_{1-6}$ alkyl, $C_{1-6}$ alkoxy $C_{1-6}$ alkyl, benzyl, formamido, $C_{2-7}$ alkanoylamino, $C_{1-6}$ alkoxycarbonylamino, hydroxy, benzoylamino or phenylcarbamoylamino group.

15. A process for the preparation of physiologically acceptable salt of the compound of formula (I) as defined in claim 1 or 2 which is 1 - methyl - 5 - [[3 - [3 - (1 - piperidinylmethyl)phenoxy]propyl]amino] - 1H - 1,2,4 - triazole - 3 - methanol which comprises converting the free base into a physiologically acceptable salt by standard methods of salt formation.

16. A process for the production of the succinate salt of 1 - methyl - 5 - [[3 - [3 - (1 - piperidinylmethyl)phenoxy]propyl]amino] - 1H - 1,2,4 - triazole - 3 - methanol which comprises converting the free base into the succinate salt by standard methods of salt formation.

17. A process as claimed in Claim 1(d) for the production of 1 - methyl - 5 - [[3 - [3 - (1 - piperidinylmethyl)phenoxy]propyl]amino] - 1H - 1,2,4 - triazole - 3 - methanol which comprises reducing methyl 1 - methyl - 5 - [[1 - oxo - 4 - [3 - (1 - piperidinylmethyl)phenoxy]propyl]-amino - 1H - 1,2,4 - triazole - 3 - carboxylate.

18. A process as claimed in Claim 1(c) for the production of 1 - methyl - 5 - [[3 - [3 - [(1 - piperidinylmethyl)phenoxy]propyl]amino] - 1H - 1,2,4 - triazole - 3 - methanol which comprises cyclising the product of reacting 3 - [3 - (1 - piperidinylmethyl)phenoxy]propanamine and methyl N - [2 - (acetyloxy)acetyl] - 1 - methyl - 2 - (phenylmethylene)hydrazine - carboximidothioate under acid conditions.

**Patentansprüche für die vertragsstaaten: BE CH DE FR GB IT LU SE**

1. Verbindungen der allgemeinen Formel (I)

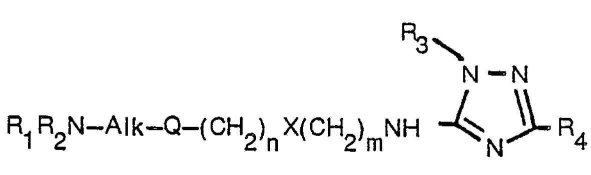

(I)

und ihre physiologisch annehmbaren Salze und Hydrate davon, worin

$R_1$ für Wasserstoff oder $C_{1-4}$-Alkyl steht und $R_2$ für $C_{1-10}$-Alkyl, $C_{3-8}$-Cycloalkyl, $C_{3-6}$-Alkenyl, Phenyl-$C_{1-6}$-alkyl (wobei die Phenylgruppe gegebenenfalls durch eine oder mehrere $C_{1-3}$-Alkylgruppen, $C_{1-3}$-Alkoxygruppen oder Halogenatome substituiert sein kann), Trifluor-$C_{1-6}$-alkyl oder $C_{1-6}$-Alkyl, substituiert durch Hydroxy, $C_{1-6}$-Alkoxy, Amino, $C_{1-6}$-Alkylamino oder Di-$C_{1-6}$-alkylamino steht oder $R_1$ und $R_2$ zusammen mit dem Stickstoffatom, an das sie angefügt sind, einen 5-bis 10-gliedrigen Ring bilden

können, der gesättigt sein kann oder der mindestens eine Doppelbindung enthalten kann, der unsubstituiert sein kann oder durch eine oder mehrere $C_{1-3}$-Alkylgruppen oder eine Hydroxygruppe substituiert sein kann und/oder der ein weiteres Heteroatom, nämlich Sauerstoff oder Schwefel, enthalten kann;

Alk für eine gerade oder verzweigte Alkylenkette mit 1 bis 6 Kohlenstoffatomen steht;

Q für einen Furan- oder Thiophenring, dessen Einarbeitung in den Rest des Moleküls durch Bindungen in 2- und 5-Stellungen erfolgt ist, wobei der Furanring gegebenenfalls einen weiteren Substituenten $R_6$ angrenzend an die Gruppe $R_1R_2N$-Alk- trägt oder Q einen Benzolring bedeutet, dessen Einarbeitung in den Rest des Moleküls durch Bindungen in 1- und 3- oder 1- und 4-Stellungen erfolgt ist;

$R_6$ für Halogen oder $C_{1-4}$-Alkyl, das durch Hydroxy oder $C_{1-4}$-Alkoxy substituiert sein kann, steht;

X für —$CH_2$—, —O—, —S— oder

$$-\underset{\underset{R_5}{|}}{N}-$$

steht, wobei $R_5$ für Wasserstoff steht;

n 0, 1 oder 2 ist;

m 2, 3 oder 4 ist;

$R_3$ für Wasserstoff, $C_{1-6}$-Alkyl, $C_{3-6}$-Alkenyl, Phenyl-$C_{1-6}$-alkyl (wobei die Phenylgruppe gegebenenfalls durch eine oder mehrere $C_{1-3}$-Alkylgruppen, $C_{1-3}$-Alkoxygruppen oder Halogenatome substituiert sein kann), Hydroxy-$C_{2-6}$-alkyl oder $C_{1-6}$-Alkoxy-$C_{1-6}$-alkyl steht; und

$R_4$ für Wasserstoff, $C_{1-6}$-Alkyl, $C_{3-6}$-Alkenyl, Phenyl-$C_{1-6}$-alkyl (wobei die Phenylgruppe gegebenenfalls durch eine oder mehrere ($C_{1-3}$-Alkylgruppen, $C_{1-3}$-Alkoxygruppen oder Halogenatome substituiert sein kann), Hydroxy-$C_{1-6}$-alkyl, Acyloxy-$C_{1-6}$-alkyl (wobei der Acylteil Benzoyl, Phenyl-$C_{2-7}$-alkanoyl oder $C_{1-6}$-Alkanoyl ist, wobei in den zwei erstgenannten Fällen der Phenylring gegebenenfalls durch eine oder mehrere $C_{1-3}$-Alkylgruppen, $C_{1-3}$-Alkoxygruppen oder Halogenatome substituierte ist), $C_{1-6}$-Alkoxy-$C_{1-6}$-alkyl, Phenoxy-$C_{1-6}$-alkyl (wobei die Phenylgruppe gegebenenfalls durch eine oder mehrere $C_{1-3}$-Alkylgruppen, $C_{1-3}$-Alkoxygruppen oder Halogenatome substituiert sein kann), Phenyl-$C_{1-6}$-alkyloxy-$C_{1-6}$-alkyl (wobei die Phenylgruppe gegebenenfalls durch eine oder mehrer $C_{1-3}$-Alkylgruppen, $C_{1-3}$-Alkoxygruppen oder Halogenatome substituiert sein kann), Amino-$C_{1-6}$-alkyl, $C_{1-6}$-Alkylamino-$C_{1-6}$-alkyl, Di-$C_{1-6}$-alkylamino-$C_{1-6}$-alkyl, Hydroxy, $C_{1-6}$-Alkoxy oder $C_{1-6}$-Alkylthio oder die Gruppe $NR_7R_8$ steht, wobei

$R_7$ für Wasserstoff oder $C_{1-6}$-Alkyl steht und

$R_8$ für die Gruppe $COR_9$ steht, wobei $R_9$ für Wasserstoff, $C_{1-6}$-Alkyl, Phenyl oder Phenyl-$C_{1-6}$-alkyl (wobei in beiden Fällen die Phenylgruppe gegebenenfalls durch eine oder mehrere $C_{1-3}$-Alkylgruppen, $C_{1-3}$-Alkoxygruppen oder Halogenatome substituiert sein kann), $C_{1-6}$-Alkoxy, eine 5- oder 6-gliedrige monocyclische Heteroarylgruppe, enthaltend ein oder mehrere Heteroatome, ausgewählt aus Sauerstoff, Stickstoff und Schwefel, oder eine Heteroaryl-$C_{1-6}$-alkylgruppe, worin die Heteroarylgruppe ein 5- oder 6-gliedriger monocyclischer Ring, enthaltend ein oder mehrere Heteroatome, ausgewählt aus Sauerstoff, Stickstoff und Schwefel, ist, steht, oder

$R_8$ für die Gruppe $SO_2R_{10}$ steht, wobei $R_{10}$ für $C_{1-6}$-Alkyl oder Phenyl (gegebenenfalls durch eine oder mehrere ($C_{1-3}$-Alkylgruppen, $C_{1-3}$-Alkoxygruppen oder Halogenatome susbtituiert) steht, oder $R_8$ für die Gruppe

$$\underset{\underset{Y}{\|}}{C}-NHR_{11}$$

steht, wobei Y für Sauerstoff oder Schwefel steht und $R_{11}$ für Wasserstoff, $C_{1-6}$-Alkyl, $C_{3-8}$-Cycloalkyl, Phenyl oder Phenyl-$C_{1-6}$-alkyl (wobei in beiden Fällen die Phenylgruppe gegebenenfalls durch eine oder mehrere $C_{1-3}$-Alkylgruppen, $C_{1-3}$-Alkoxygruppen oder Halogenatome substituiert sein kann) steht.

2. Verbindungen nach Anspruch 1, dadurch gekennzeichnet, daß sie in Form eines Hydrochlorid-, Hydrobromid-, Sulfat-, Methansulfonat-, Acetat-, Maleat-, Succinat-, Citrat-, Fumarat-, Benzoat- oder Tartratsalzes vorliegen.

3. Verbindungen nach Anspruch 1, dadurch gekennzeichnet, daß $R_4$ für die Gruppe $NR_7R_8$ steht, wobei $R_7$ und $R_8$ die Definition nach Anspruch 1 haben, mit der Ausnahme, daß $R_9$ eine andere Gruppe ist als eine Heteroaryl-oder Heteroarylalkylgruppe und daß $R_{11}$ eine andere Gruppe als $C_{3-8}$-Cycloalkyl ist; oder $R_4$ für Wasserstoff, $C_{1-6}$-Alkyl, $C_{3-6}$-Alkenyl, Phenyl-$C_{1-6}$-alkyl (wobei die Phenylgruppe gegebenenfalls durch eine oder mehrere $C_{1-3}$-Alkylgruppen, $C_{1-3}$-Alkoxygruppen oder Halogenatome substituiert sein kann), Hydroxy-$C_{1-6}$-alkyl, $C_{1-6}$-Alkoxy-$C_{1-6}$-alkyl, Phenoxy-$C_{1-6}$-alkyl (wobei die Phenylgruppe gegebenenfalls durch eine oder mehrere $C_{1-3}$-Alkylgruppen, $C_{1-3}$-Alkoxygruppen oder Halogenatome substituiert sein kann), Amino-$C_{1-6}$-alkyl, $C_{1-6}$-Alkylamino-$C_{1-6}$-alkyl, Di-$C_{1-6}$-alkylamino-$C_{1-6}$-alkyl, Hydroxy oder $C_{1-6}$-Alkoxy steht;

mit der Maßgabe, daß, wenn X ein Sauerstoffatom oder —$NR_5$— darstellt und wenn n 0 ist, Q für Benzol steht.

4. Verbindungen nach einem der Ansprüche 1 bis 3, dadurch gekennzeichnet, daß Q für Benzol steht, das in das Molekül durch Bindungen in 1- und 3-Stellungen eingefügt ist, n 0 ist und X für Sauerstoff steht.

5. Verbindungen nach Anspruch 4, dadurch gekennzeichnet, daß $R_4$ für Wasserstoff, $C_{1-6}$-Alkyl, Hydroxy-$C_{1-6}$-alkyl, $C_{1-6}$-Alkoxy-$C_{1-6}$-alkyl, Acyloxy-$C_{1-6}$-alkyl, Phenyl-$C_{1-6}$-alkyl (wobei die Phenylgruppe gegebenenfalls durch eine oder mehrere $C_{1-3}$-Alkylgruppen, $C_{1-3}$-Alkoxygruppen oder Halogenatome substituiert sein kann) oder Hydroxy oder die Gruppe $NHCOR_9$, worin $R_9$ für Wasserstoff, $C_{1-6}$-Alkyl, $C_{1-6}$-Alkoxy, Phenyl (gegebenenfalls durch eine oder mehrere $C_{1-3}$-Alkylgruppen, $C_{1-3}$-Alkoxygruppen oder Halogenatome substituiert) oder eine 5- oder 6-gliedrige monocyclische Heteroarylgruppe, enthaltend ein oder mehrere Heteroatome, ausgewählt aus Sauerstoff, Stickstoff und Schwefel, steht; oder die Gruppe $NHCONHR_{11}$, worin $R_{11}$ für $C_{1-6}$-Alkyl, Phenyl (gegebenenfalls durch eine oder mehrere $C_{1-3}$-Alkylgruppen, $C_{1-3}$-Alkoxygruppen oder Halogenatome substituiert) oder $C_{3-8}$-Cycloalkyl steht, steht.

6. Verbindungen nach Anspruch 5, dadurch gekennzeichnet, daß $R_4$ für Hydroxy-$C_{1-6}$-alkyl, Hydroxy, $C_{1-6}$-Alkoxy-$C_{1-6}$-alkyl, Benzyl oder die Gruppe $NHCOR_9$, wobei $R_9$ für Wasserstoff, Phenyl, $C_{1-6}$-Alkyl oder $C_{1-6}$-Alkoxy steht, oder die Gruppe $NHCONHR_{11}$, wobei $R_{11}$ für Phenyl steht, steht.

7. Verbindungen nach einem der Ansprüche 1 bis 4, dadurch gekennzeichnet, daß $R_4$ für Hydroxy-$C_{1-6}$-alkyl, Acyloxy-$C_{1-6}$-alkyl oder $C_{1-6}$-Alkoxy-$C_{1-6}$-alkyl steht.

8. Verbindungen nach Anspruch 7, dadurch gekennzeichnet, daß $R_4$ für Hydroxy-$C_{1-6}$-alkyl steht.

9. Verbindungen nach einem der Ansprüche 1 bis 8, dadurch gekennzeichnet, daß m+n 3 oder 4 ist.

10. Verbindungen nach einem der Ansprüche 1 bis 9, dadurch gekennzeichnet, daß Alk für $-CH_2-$ steht.

11. Verbindungen nach einem der Ansprüche 1 bis 10, dadurch gekennzeichnet, daß $R_1$ für Wasserstoff oder $C_{1-4}$-Alkyl steht und $R_2$ für $C_{3-5}$-Alkenyl, $C_{5-7}$-Cycloalkyl, Benzyl, $C_{1-8}$-Alkyl oder $C_{1-4}$-Alkyl, substituiert durch $C_{1-3}$-Alkoxy, Hydroxy, Di-$C_{1-3}$-alkylamino oder Trifluormethyl steht, oder $R_1$ und $R_2$ zusammen mit dem Stickstoffatom, an das sie angefügt sind, einen Ring mit 5 bis 8 Gliedern bilden, der gegebenenfalls eine Doppelbindung enthält und/oder durch Hydroxy oder eine oder zwei Methylgruppe(n) substituiert ist.

12. Verbindungen nach Anspruch 11, dadurch gekennzeichnet, daß $R_1$ und $R_2$ für $C_{1-3}$-Alkyl stehen oder $R_1$ und $R_2$ zusammen mit dem Stickstoffatom, an das sie angefügt sind, einen heterocyclischen Ring bilden, der Pyrrolidin, Piperidin, das gegebenenfalls in 4-Stellung durch Methyl oder Hydroxy substituiert ist, Tetrahydropyridin, Morpholin, 2,6-Dimethylmorpholin, Hexamethylenimin oder Heptamethylenimin ist.

13. Verbindungen nach einem der Ansprüche 1 bis 12, dadurch gekennzeichnet, daß $R_3$ für Wasserstoff, $C_{1-6}$-Alkyl oder Hydroxy-$C_{2-4}$-alkyl steht.

14. Verbindungen nach Anspruch 1 oder 2, entsprechend der Formel (II)

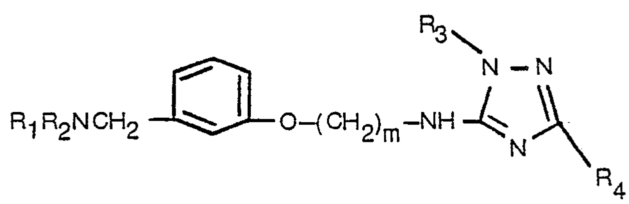

(II)

worin $R_1$ und $R_2$ Methylgruppen sind oder zusammen mit dem Stickstoffatom, an das sie angefügt sind, eine Pyrrolidino-, Piperidino- oder Hexamethyleniminogruppe bilden; m 3 oder 4 ist; $R_3$ für Wasserstoff oder Methyl steht und $R_4$ für eine Hydroxy-$C_{1-6}$-alkyl-, $C_{1-6}$-Alkoxy-$C_{1-6}$-alkyl-, Benzyl-, Formamido-, $C_{2-7}$-Alkanoylamino-, $C_{1-6}$-Alkoxycarbonylamino-, Hydroxy-, Benzoylamino- oder Phenylcarbamoylaminogruppe steht.

15. Verbindung nach Anspruch 1 oder 2, nämlich 1-Methyl-5-[[3-[3-(1-piperidinylmethyl)-phenoxy]propyl]amino]-1H-1,2,4-triazol-3-methanol und die physiologisch annehmbaren Salze davon.

16. Die Succinatsalz von 1-Methyl-5-[[3-[3-(1-piperidinylmethyl)phenoxy]propyl]amino]-1H-1,2,4-triazol-3-methanol.

17. Verbindung nach Anspruch 1 oder 2, nämlich 5-[[2-[[[5-[(Dimethylamino)methyl]-2-furanyl]-methyl]thio]ethyl]amino]-1-methyl-1H-1,2,4-triazol-3-methanol und die physiologisch annehmbaren Salze davon.

18. Verbindung nach Anspruch 1 oder 2, nämlich 5-[[2-[[[5-[(Dimethylamino)methyl]-4-methyl-2-furanyl]methyl]thio]ethyl]amino]-1-methyl-1H-1,2,4-triazol-3-methanol und die physiologisch annehmbaren Salze davon.

19. Verbindung nach Anspruch 1 oder 2, nämlich 1-Methyl-5-[[3-[3-[(dimethylamino)-methyl]phenoxy]propyl]amino]-1H-1,2,4-triazol-3-methanol und die physiologisch annehmbaren Salze davon.

20. Verbindung nach Anspruch 1 oder 2, nämlich 5-[[3-[3-(Piperidinylmethyl)phenoxy]propyl]-amino]-1H-1,2,4-triazol-3-on und die physiologisch annehmbaren Salze davon.

21 Verbindungen nach Anspruch 1 oder 2, nämlich 1-Methyl-5-[[4-[3-(1-piperidinylmethyl)-phenoxy]butyl]amino]-1H-1,2,4-triazol-3-methanol,
N-[1-Methyl-5-[[3-[3-(1-piperidinylmethyl)phenoxy]propyl]amino]-1H-1,2,4-triazol-3-yl]formamid
3-Methoxymethyl-1-methyl-5-[[4-[3-(1-piperidinylmethyl)phenoxy]butyl]amino]-1H-1,2,4-triazol und die physiologisch annehmbaren Salze davon.

22. Verbindungen nach Anspruch 1 oder 2, nämlich 3-Phenylmethyl-N-[3-[3-(dimethylamino-methyl)phenoxy]propyl]-1H-1,2,4-triazol-5-amin
5-[4-[3-(1-piperidinylmethyl)phenoxy]butyl]amino-1H-1,2,4-triazol-3-methanol,
3-Phenylmethyl-N-[3-[3-(1-piperidinylmethyl)phenoxy]propyl]-1H-1,2,4-triazol-5-amin,
Ethyl-1-methyl-5-[[3-[3-[(dimethylamino)methyl]phenoxy]propyl]amino]-1H-1,2,4-triazol-3-carbamat,
N-[5-[[3-[3-[(Dimethylamino)methyl]phenoxy]propyl]amino]-1-methyl-1H-1,2,4-triazol-3-yl]benzamid
N-[5-[[3-[3-(1-Piperidinylmethyl)phenoxy]propyl]amino]-1-methyl-1H-1,2,4-triazol-3-yl]-N'-phenylharnstoff und die physiologisch annehmbaren Salze davon.

23. Verfahren zur Herstellung der Verbindungen nach Anspruch 1 oder 2, dadurch gekennzeichnet, daß man

(a) zur Herstellung von Verbindungen, bei denen $R_4$ ein Wasserstoffatom darstellt, ein Diazoniumsalz (III)

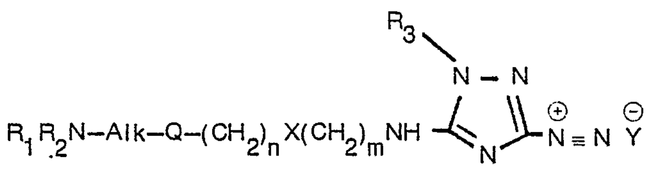

(III)

worin $R_1$, $R_2$ und $R_3$ die im Zusammenhang mit der Formel (I) angegebenen Definitionen haben oder Gruppen sind, die in solche Gruppen umwandelbar sind, Alk, Q, n, X und m die im Zusammenhang mit der Formel (I) angegebenen Definitionen haben und $\overset{\ominus}{Y}$ das Anion ist, das der bei der Diazotisierungsreaktion verwendeten Säure entspricht, mit einer konzentrierten Mineralsäure behandelt, um die Gruppe $-\overset{\oplus}{N}{\equiv}N\ \overset{\ominus}{Y}$ in ein Wasserstoffatom umzuwandeln;

(b) zur Herstellung von Verbindungen, bei denen $R_4$ die Gruppe $NR_7R_8$ darstellt, wobei $R_8$ für $COR_9$, $SO_2R_{10}$ oder $C(=Y)NHR_{11}$ steht, ein Aminotriazol (IV)

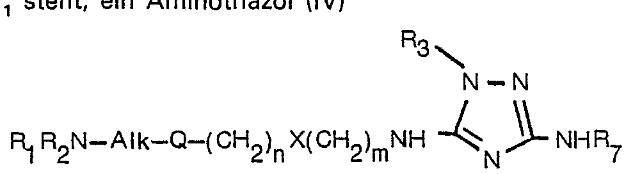

(IV)

worin, $R_1$, $R_2$, $R_3$ und $R_7$ die im Zusammenhang mit der Formel (I) angegebenen Definitionen haben oder Gruppen sind, die leicht in solche Gruppen umwandelbar sind, mit einem aktiverten Derivat entweder einer Carbonsäure $R_9COOH$ oder einer Sulfonsäure $R_{10}SO_3H$, wobei $R_9$ und $R_{10}$ die in Zusammenhang mit der Formel (I) angegebenen Definitionen haben, oder mit einem Isocyanat oder Isothiocyanat $R'_{11}NCY$, wobei $R'_{11}$ eine der für $R_{11}$ in der Formel (I) angegebenen Bedeutungen, ausgenommen Wasserstoff, hat oder ein Alkalimetallatom oder eine Alkoxycarbonylgruppe bedeutet, behandelt, um das Wasserstoffatom in der Gruppe $NHR_7$ durch die Gruppe $COR_9$, $SO_2R_{10}$ bzw. $C(=Y)NHR_{11}$ zu ersetzen;

(c) zur Herstellung von Verbindungen, bei denen $R_4$ eine andere Bedeutung als eine Alkoxy-, Alkylthio- oder Acyloxyalkylgruppe hat, eine Verbindung der Formel (V)

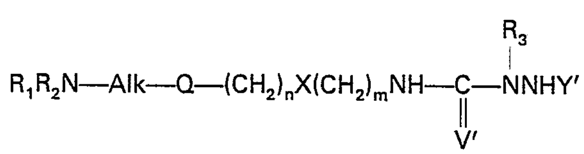

(V)

worin $R_1$, $R_2$, Alk, Q, X, $R_3$, n und m die im Zusammenhang mit der Formel (I) angegebenen Definitionen haben und V' für

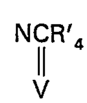

steht und Y' für Wasserstoff steht, wobei V für Sauerstoff oder Schwefel steht und $R'_4$ eine wie für $R_4$ definierte Gruppe oder eine bei den Bedingungen der Cyclisierungsreaktion in eine solche Gruppe umwandelbare Gruppe bedeutet oder für Halogen oder Alkoxy steht; oder V' für NH steht und Y' für

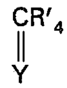

steht, wobei Y für Schwefel, Sauerstoff oder NH steht, mit der Ausnahme, daß, wenn $R'_4$ für Halogen oder Alkoxy steht, Y nicht die Bedeutung NH haben kann, oder V' für Schwefel oder Sauerstoff steht und Y' für

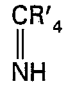

steht, gegebenenfalls unter Schutz irgendwelcher reaktiver Gruppen in dem Ausgangsmaterial und nachfolgender Abspaltung der Schutzgruppe, wenn es gewünscht wird, cyclisiert;

(d) eine Verbindung der Formel (XV)

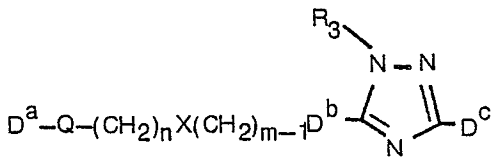

(XV)

worin Q, n, X, m und $R_3$ die im Zusammenhang mit der Formel (I) angegebenen Definitionen haben und wobei mindestens eine der Gruppen $D^a$, $D^b$ und $D^c$ eine reduzierbare Gruppe darstellt und die andere(n) die entsprechende Bedeutung entsprechend der Formel (I) einnehmen,

$D^a$ für $R_1R_2NAlk$— oder eine unter reduzierenden Bedingungen in eine solche Gruppe umwandelbare Gruppe steht, wobei $R_1$, $R_2$ und Alk die im Zusammenhang mit der Formel (I) angegebenen Definitionen haben;

$D^b$ für $—CH_2NH—$, —CONH— oder —CH=N— steht;

$D^c$ für $R_4$, wie im Zusammenhang mit der Formel (I) definiert, oder eine unter reduzierenden Bedingungen in eine solche Gruppe umwandelbare Gruppe steht, gegebenenfalls unter Schutz irgendwelcher reaktiver Gruppen in dem Ausgangsmaterial und nachfolgender Abspaltung der Schutzgruppe, wenn es gewünscht wird, reduziert;

(e) eine Verbindung der Formel (XX)

$$R_1R_2NAlkQE \qquad (XX)$$

worin E für $(CH_2)_nX(CH_2)_mP$ oder $CH_2P'$ steht, wobei P und P' verlassende Gruppen sind, mit einem Triazol der Formel (XVII)

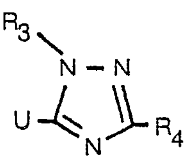

(XVII)

worin U für Amino, HS $(CH_2)_mNH$ oder HO $(CH_2)_mNH$ steht und $R_4$ die im Zusammenhang mit der Formel (I) angegebene Definition hat oder eine Gruppe ist, die in eine solche Gruppe umwandelbar ist, gegebenenfalls unter Schutz irgendwelcher reaktiver Gruppen in dem Ausgangsmaterial und nachfolgender Abspaltung der Schutzgruppe, wenn es gewünscht wird, umsetz;

(f) die Gruppe $R_1R_2N$—Alk in die in einem geeigneten Zwischenprodukt vorhandene Gruppe Q einführt, indem man

i. zur Herstellung von Verbindungen, bei denen Q Furan oder substituiertes Furan ist und Alk Methylen ist, das Zwischenprodukt mit Formaldehyd und einem Amin $R_1R_2NH$ oder einem Salz davon (wobei $R_1$ und $R_2$ die im Zusammenhang mit der Formel (I) angegebenen Definitionen haben) umsetzt oder

ii. zur Herstellung von Verbindungen, vei denen beide Gruppen $R_1$ und $R_2$ Methyl darstellen, Alk Methylen ist und Q Furan, substituiertes Furan oder Thiophen ist, das Zwischenprodukt mit der Verbindung $(CH_3)_2\overset{\oplus}{N}=CH_2\overset{\ominus}{Cl}$, in beiden Fällen gegebenenfalls unter Schutz irgendwelcher reeaktiver Gruppen in dem Ausgangsmaterial und nachfolgender Abspaltung der Schutzgruppe, wenn es gewünscht wird, umsetzt;

(g) zur Herstellung von Verbindungen, bei denen $R_4$ eine sekundäre oder tertiäre Hydroxyalkylgruppe ist, die entsprechende Verbindung, bei der $R_4$ die Gruppe $(CH_2)_{q-1}$ CHO, $(CH_2)_{q-1}$ $CO_2R_{12}$ oder $(CH_2)_rCOR_{12}$ ist wobei q eine ganze Zahl von 1 bis einschließlich 6 ist, r 0 bis 4 ist und $R_{12}$ eine Alkylgruppe ist, mit einem Organometallderivat behandelt;

(h) eine Verbindung der Formel (XXII)

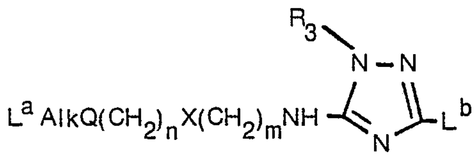

(XXII)

worin entweder $L^a$ für $R_1R_2N$ und $L^b$ für die Gruppe $(CH_2)_q$ $L^c$ steht oder $L^a$ für die Gruppe $L^d$ und $L^b$ für $R_4$ steht, wobei $L^c$ und $L^d$ verlassende Gruppen sind und q eine ganze Zahl von 1 bis einschließlich 6 ist, mit einer Verbindung, die dazu imstande ist, $L^d$ durch $R_1R_2N$ oder $L^c$ zu ersetzen, so daß $L^b$ in die Gruppe $R^4$ umgewandelt wird, gegebenenfalls unter Schutz iregendwelcher reaktiver Gruppen in dem Ausgangsmaterial und nachfolgender Abspaltung der Schutzgruppe, wenn es gewünscht wird, umsetzt;

(i) zur Herstellung von Verbindungen, bei denen $R_4$ eine Alkoxyalkylgruppe ist, die Gruppe $R_4$ in ein Zwischenprodukt, in dem diese Gruppe Hydroxylalkyl ist, in eine Alkoxyalkylgruppe durch Standardmethoden einer Interkonversion umwandelt;

(j) zur Herstellung von Verbindungen, bei denen $R_4$ eine Acyloxyalkylgruppe ist, die entsprechende Hydroxyalkylverbindung mit einer geeigneten Säure bei erhöhter Temperatur behandelt; oder

(k) zur Herstellung von Verbindungen, bei denen $R_4$ eine Hydroxyalkylgruppe ist, eine Hydroxylschutzgruppe von der Hydroxylgruppe entfernt;

und, wenn die Verbindung der Formel (I) in Form einer freien Base erzeugt wird, gegebenenfalls die freie Base in ein Salz umwandelt.

24. Arzneimittel, dadurch gekennzeichnet, daß es eine Verbindung nach einem der Ansprüche 1 bis 22 zusammen mit mindestens einem pharmazeutisch annehmbaren Träger oder Verdünnungsmittel und gegebenenfalls einem oder mehreren Wirkstoffen enthält.

25. Arzneimittel nach Anspruch 24, dadurch gekennzeichnet, daß es in einer für die orale Verabreichungs angepaßten Form vorliegt.

26. Arzneimittel nach Anspruch 25, dadurch gekennzeichnet, daß es 5 mg bis 1 g der Verbindung der Formel (I) enthält.

27. Arzneimittel nach Anspruch 26, dadurch gekennzeichnet, daß es 5 mg bis 250 mg der Verbindung der Formel (I) enthält.

**Patentansprüche fü den Vertragsstaat: AT**

1. Verfahren zur Herstellung von Verbindungen der allgemeinen Formel (I)

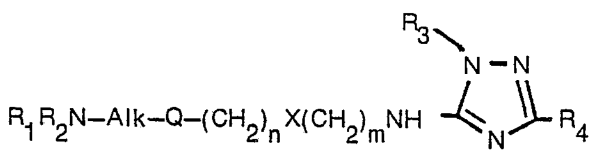

(I)

und ihrer physiologisch annehmbarer Salze und Hydrate davon, worin

$R_1$ für Wasserstoff oder $C_{1-4}$-Alkyl steht und $R_2$ für $C_{1-10}$-Alkyl, $C_{3-8}$-Cycloalkyl, $C_{3-6}$-Alkenyl, Phenyl-$C_{1-6}$-alkyl (wobei die Phenylgruppe gegebenenfalls durch eine oder mehrere $C_{1-3}$-Alkylgruppen, $C_{1-3}$-Alkoxygruppen oder Halogenatome substituiert sein kann), Trifluor-$C_{1-6}$-alkyl oder $C_{1-6}$-Alkyl, substituiert durch Hydroxy, $C_{1-6}$-Alkoxy, Amino, $C_{1-6}$-Alkylamino oder Di—$C_{1-6}$-alkylamino steht oder $R_1$ und $R_2$ zusammen mit dem Stickstoffatom, an das sie angefügt sind, einen 5-bis 10-gliedrigen Ring bilden können, der gesättigt sein kann oder der mindestens eine Doppelbindung enthalten kann, der unsubstituiert sein kann oder durch eine oder mehrere $C_{1-3}$-Alkylgruppen oder eine Hydroxygruppe substituiert sein kann und/oder der ein weiteres Heteroatom, nämlich Sauerstoff oder Schwefel, enthalten kann;

Alk für gerade oder verzweigte Alkylenkette mit 1 bis 6 Kohlenstoffatomen steht;

Q für einen Furan- oder Thiophenring, dessen Einarbeitung in den Rest des Moleküls durch Bindungen in 2- und 5-Stellungen erfolgt ist, wobei der Furanring gegebenenfalls einen weiteren Substituenten $R_6$ angrenzend an die Gruppe $R_1R_2N$-Alk- trägt oder Q einen Benzolring bedeutet, dessen Einarbeitung in den Rest des Moleküls durch Bindungen in 1- und 3- oder 1- und 4-Stellungen erfolgt ist;

$R_6$ für Halogen oder $C_{1-4}$-Alkyl, das durch Hydroxy oder $C_{1-4}$-Alkoxy substituiert sein kann, steht;

X für —$CH_2$—, —O—, —S— oder

—N—
|
$R_5$

steht, wobei $R_5$ für Wasserstoff steht;

n 0, 1 oder 2 ist;

m 2, 3 oder 4 ist;

$R_3$ für Wasserstoff, $C_{1-6}$-Alkyl, $C_{3-6}$-Alkenyl, Phenyl-$C_{1-6}$-alkyl (wobei die Phenylgruppen gegebenenfalls durch eine oder mehrere $C_{1-3}$ Alkylgruppen, $C_{1-3}$-Alkoxygruppen oder Halogenatome substituiert sein können), Hydroxy-$C_{2-6}$-alkyl oder $C_{1-6}$-Alkoxy-$C_{1-6}$-alkyl steht; und

$R_4$ für Wasserstoff, $C_{1-6}$-Alkyl, $C_{3-6}$-Alkenyl, Phenyl-$C_{1-6}$-alkyl (wobei die Phenylgruppe gegebenenfalls durch eine oder mehrere $C_{1-3}$-Alkylgruppen, $C_{1-3}$-Alkoxygruppen oder Halogenatome substituiert sein kann), Hydroxy-$C_{1-6}$-alkyl, Acyloxy-$C_{1-6}$-alkyl (wobei der Acylteil Benzoyl, Phenyl-$C_{2-7}$-alkanoyl oder $C_{1-6}$-Alkanoyl ist, wobei in den zwei erstgenannten Fällen der Phenylring gegebenenfalls durch eine oder mehrere $C_{1-3}$-Alkylgruppen, $C_{1-3}$-Alkoxygruppen oder Halogenatome substituiert ist), $C_{1-6}$-Alkoxy-$C_{1-6}$-alkyl, Phenoxy-$C_{1-6}$-alkyl (wobei die Phenylgruppe gegebenenfalls durch eine oder mehrere $C_{1-3}$-Alkylgruppen, $C_{1-3}$-Alkoxygruppen oder Halogenatome susbtituiert sein kann), Phenyl-$C_{1-6}$-alkyloxy-$C_{1-6}$-alkyl (wobei die Phenylgruppe gegebenenfalls durch eine oder mehrere $C_{1-3}$-Alkylgruppen, $C_{1-3}$-Alkoxygruppen oder Halogenatome substituiert sein kann), Amino-$C_{1-6}$-alkyl, $C_{1-6}$-Alkylamino-$C_{1-6}$-alkyl, Di-$C_{1-6}$-alkylamino-$C_{1-6}$-alkyl, Hydroxy, $C_{1-6}$-Alkoxy oder $C_{1-6}$-Alkylthio oder die Gruppe $NR_7R_8$ steht, wobei

$R_7$ für Wasserstoff oder $C_{1-6}$-Alkyl steht und

R für die Gruppe $COR_9$ steht, wobei $R_9$ für Wasserstoff, $C_{1-6}$-Alkyl, Phenyl oder Phenyl-$C_{1-6}$-alkyl (wobei in beiden Fällen die Phenylgruppe gegebenenfalls durch eine oder mehrere $C_{1-3}$-Alkylgruppen, $C_{1-3}$-Alkoxygruppen oder Halogenatome substituiert sein kann), $C_{1-6}$-Alkoxy, eine 5- oder 6-gliedrige monocyclische Heteroarylgruppe, enthaltend ein oder mehrere Heteroatome, ausgewählt aus Sauerstoff, Stickstoff und Schwefel, oder eine Heteroaryl-$C_{1-6}$-alkylgruppe, worin die Heteroarylgruppe ein 5- oder 6-gliedriger monocyclischer Ring, enthaltend ein oder mehrere Heteroatome, ausgewählt aus Sauerstoff, Stickstoff und Schwefel, ist, steht, oder

$R_8$ für die Gruppe $SO_2R_{10}$ steht, wobei $R_{10}$ für $C_{1-6}$-Alkyl oder Phenyl (gegebenenfalls durch eine oder mehrere $C_{1-3}$-Alkylgruppen, $C_{1-3}$-Alkoxygruppen oder Halogenatome substituiert) steht, oder $R_8$ für die Gruppe

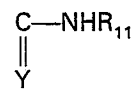

steht, wobei

Y für Sauerstoff oder Schwefel steht und $R_{11}$ für Wasserstoff, $C_{1-6}$-Alkyl, $C_{3-8}$-Cycloalkyl, Phenyl oder Phenyl-$C_{1-6}$-alkyl (wobei in beiden Fällen die Phenylgruppe gegebenenfalls durch eine oder mehrere $C_{1-3}$-Alkylgruppen, $C_{1-3}$-Alkoxygruppen oder Halogenatome susbtituiert sein kann) steht,

dadurch gekennzeichnet, daß man

(a) zur Herstellung von Verbindungen, bei denen $R_4$ ein Wasserstoffatom darstellt, eine Diazoniumsalz (III)

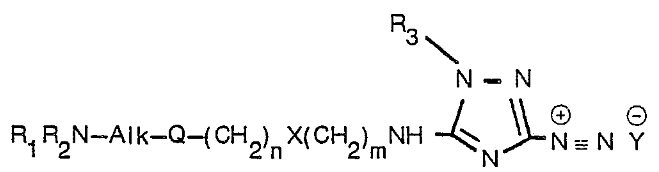

(III)

worin $R_1$, $R_2$ und $R_3$ die im Zusammenhang mit der Formel (I) angegebenen Definitionen haben oder Gruppen sind, die in solche Gruppen umwandelbar sind, Alk, Q, n, X und m die im Zusammenhang mit der Formel (I) angegebenen Definitionen haben und $\overset{\ominus}{Y}$ das Anion ist, das der bei der Diazotisierungsreaction verwendeten Säure entspricht, mit einer konzentrierten Mineralsäure behandelt, um die Gruppe $-\overset{\oplus}{N}{\equiv}N\ \overset{\ominus}{Y}$ in ein Wasserstoffatom umzuwandeln;

(b) zur Herstellung von Verbindungen, bei denen $R_4$ die Gruppe $NR_7R_8$ darstellt, wobei $R_8$ für $COR_9$, $SO_2R_{10}$ oder $C(=Y)NHR_{11}$ steht, ein Aminotriazol (IV)

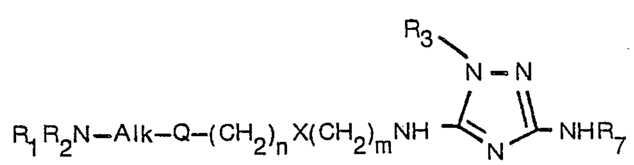

(IV)

worin $R_1$, $R_2$, $R_3$ und $R_7$ die im Zusammenhang mit der Formel (I) angegebenen Definitionen haben oder Gruppen sind, die leicht in solche Gruppen umwandelbar sind, mit einem aktivierten Derivat entweder einer Carbonsäure $R_9COOH$ oder einer Sulfonsäure $R_{10}SO_3H$, wobei $R_9$ und $R_{10}$ die im Zusammenhang mit der Formel (I) angegebenen Definitionen haben, oder mit einem Isocyanat oder Isothiocyanat

$R'_{11}NCY$, wobei $R'_{11}$ eine der für $R_{11}$ in der Formel (I) angegebenen Bedeutungen, ausgenommen Wasserstoff, hat oder ein Alkalimetallatom oder eine Alkoxycarbonylgruppe bedeutet, behandelt, um das Wasserstoffatom in der Gruppe $NHR_7$ durch die Gruppe $COR_9$, $SO_2R_{10}$ bzw. $C(=Y)NHR_{11}$ zu ersetzen;

(c) zur Herstellung von Verbindungen, bei denen $R_4$ eine andere Bedeutung als eine Alkoxy-, Alkylthio- oder Acyloxyalkylgruppe hat, eine Verbindung der Formel (V)

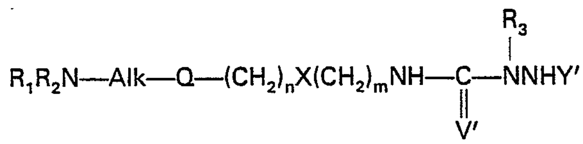

(V)

worin $R_1$, $R_2$, Alk, Q, X, $R_3$, n und m die im Zusammenhang mit der Formel (I) angegebenen Definitionen haben und V' für

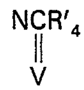

steht und Y' für Wasserstoff steht, wobei V für Sauerstoff oder Schwefel steht und $R'_4$ eine wie für $R_4$ definierte Gruppe oder eine bei den Bedingungen der Cyclisierungsreaktion in eine solche Gruppe umwandelbare Gruppe bedeutet oder für Halogen oder Alkoxy steht; oder V' für NH steht und Y' für

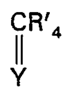

steht, wobei Y für Schwefel, Sauerstoff oder NH steht, mit der Ausnahme, daß, wenn $R'_4$ für Halogen oder Alkoxy steht, Y nicht die Bedeutung NH haben kann, oder V' für Schwefel oder Sauerstoff steht und Y' für

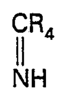

steht, gegebenenfalls unter Schutz irgendwelcher reaktiver Gruppen in dem Ausgangsmaterial und nachfolgender Abspaltung der Schutzgruppe, wenn es gewünscht wird, cyclisiert;

(d) eine Verbindung der Formel (XV)

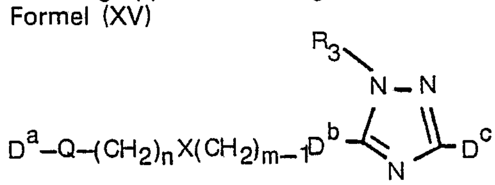

(XV)

worin Q, n, X, m und $R_3$ die im Zusammenhang mit der Formel (I) angegebenen Definitionen haben und wobei mindestens eine der Gruppen $D^a$, $D^b$ und $D^c$ eine reduzierbare Gruppe darstellt und die andere(n) die entsprechende Bedeutung entsprechend der Formel (I) einnehmen,

$D^a$ für $R_1R_2NAlk$— oder eine unter reduzierenden Bedingungen in eine solche Gruppe umwandelbare Gruppen steht, wobei $R_1$, $R_2$ und Alk die im Zusammenhang mit der Formel (I) angegebenen Definitionen haben;

$D^b$ für $—CH_2NH—$, —CONH— oder —CH=N— steht;

$D^c$ für $R_4$ wie im Zusammenhang mit der Formel (I) definiert, oder eine unter reduzierenden Bedingungen in eine solche Gruppe umwandelbare Gruppe steht, gegebenenfalls unter Schutz irgendwelcher reaktiver Gruppen in dem Ausgangsmaterial und nachfolgender Abspaltung der Schutzgruppe, wenn es gewünscht wird, reduziert;

(e) eine Verbindung der Formel (XX)

$$R_1R_2NAlkQE \qquad (XX)$$

worin E für $(CH_2)_nX(CH_2)_mP$ oder $CH_2P'$ steht, wobei P und P' verlassende Gruppen sind, mit einem Triazol der Formel (XVII)

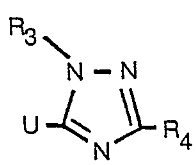

(XVII)

worin U für Amino, HS $(CH_2)_m$ oder HO $(CH_2)_mNH$ steht und $R_4$ die im Zusammenhang mit der Formel (I) angegebene Definition hat oder eine Gruppe ist, die in eine solche Gruppe umwandelbare ist, gegebenenfalls unter Schutz irgendwelcher reaktiver Gruppen in dem Ausgangsmaterial und nachfolgender Abspaltung der Schutzgruppe, wenn es gewünscht wird, umsetzt;

(f) die Gruppe $R_1R_2N$—Alk in die in einem geeigneten Zwischenprodukt vorhandene Gruppe Q einführt, indem man

i. zur Herstellung von Verbindungen, bei denen Q Furan oder substituiertes Furan ist und Alk Methylen ist, das Zwischenprodukt mit Formaldehyd und einem Amin $R_1R_2NH$ oder einem Salz davon (wobei $R_1$ und $R_2$ die im Zusammenhang mit der Formel (I) angegebenen Definitionen haben) umsetzt oder

ii. zur Herstellung von Verbindungen, bei denen beide Gruppen $R_1$ und $R_2$ Methyl darstellen, Alk Methylen ist und Q Furan, substituiertes Furan oder Thiophen ist, das Zwischenprodukt mit der Verbindung $(CH_3)_2\overset{\oplus}{N}{=}CH_2\overset{\ominus}{Cl}$, in beiden Fällen gegebenenfalls unter Schutz irgendwelcher reaktiver Gruppen in dem Ausgangsmaterial und nachfolgender Abspaltung der Schutzgruppe, wenn es gewünscht wird, umsetzt;

(g) zur Herstellung von Verbindungen, bei denen $R_4$ eine sekundäre oder tertiäre Hydroxyalkylgruppe ist, die entsprechende Verbindung, bei der $R_4$ die Gruppe $(CH_{2q-1}$ CHO, $(CH_2)_{q-1}$ $CO_2R_{12}$ oder $(CH_2)_rCOR_{12}$ ist, wobei q eine ganze Zahl von 1 bis einschließlich 6 ist, r 0 bis 4 ist und $R_{12}$ eine Alkylgruppe ist, mit einem Organometallderivat behandelt;

(h) eine Verbindung der Formel (XXII)

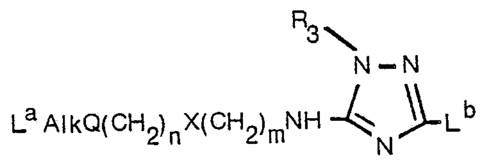

(XXII)

worin entweder $L^a$ für $R_1R_2N$ und $L^b$ für die Gruppe $(CH_2)_q$ $L^c$ steht oder $L^a$ für die Gruppe $L^d$ und $L^b$ für $R_4$ steht, wobei $L^c$ und $L^d$ verlassende Gruppen sind und q eine ganze Zahl von 1 bis einschließlich 6 ist, mit einer Verbindung, die dazu imstande ist, $L^d$ durch $R_1R_2N$ oder $L^c$ zu ersten, so daß $L^b$ in die Gruppe $R^4$ umgewandelt wird, gegebenenfalls unter Schutz irgendwelcher reaktiver Gruppen in dem Ausgangsmaterial und nachfolgender Abspaltung der Schutzgruppe, wenn es gewünscht wird, umsetzt;

(i) zur Herstellung von Verbindungen, bei denen $R_4$ eine Alkoxyalkylgruppe ist, die Gruppe $R_4$ in ein Zwischenprodukt, in dem diese Gruppe Hydroxyalkyl ist, in eine Alkoxyalkylgruppe durch Standardmethoden einer Interkonversion umwandelt;

(j) zur Herstellung von Verbindungen, bei denen $R_4$ eine Acyloxyalkylgruppe ist, die entsprechende Hydroxyalkylverbindung mit einer geeigneten Säure bei erhöhter Temperatur behandelt; oder

(k) zur Herstellung von Verbindungen, bei denen $R_4$ eine Hydroxyalkylgruppe ist, eine Hydroxylschutzgruppe von der Hydroxylgruppe entfernt;

und, wenn die Verbindung der Formel (I) in Form einer freien Base erzeugt wird, gegebenenfalls die freie Base in ein Salz umwandelt.

2. Verfahren nach Anspruch 1 zur Herstellung von Verbindungen in Form eines Hydrochlorid-, Hydrobromid-, Sulfat-, Methansulfonat-, Acetat-, Maleat-, Succinat-, Citrat-, Fumarat-, Benzoat- oder Tartratsalzes.

3. Verfahren nach Anspruch 1 zur Herstellung von Verbindungen, bei denen $R_4$ für die Gruppe $NR_7R_8$ steht, wobei $R_7$ und $R_8$ die Definition nach Anspruch 1 haben, mit der Ausnahme, daß $R_9$ eine andere Gruppe ist als eine Heteroaryl- oder Heteroarylalkylgruppe und daß $R_{11}$ eine andere Gruppe als $C_{3-8}$-Cycloalkyl ist; oder $R_4$ für Wasserstoff, $C_{1-6}$-Alkyl, $C_{3-6}$-Alkenyl, Phenyl-$C_{1-6}$-alkyl (wobei die Phenylgruppe gegebenenfalls durch eine oder mehrere $C_{1-3}$-Alkylgruppen, $C_{1-3}$-Alkoxygruppen oder Halogenatome substituiert sein kann), Hydroxy-$C_{1-6}$-alkyl, $C_{1-6}$-Alkoxy-$C_{1-6}$-alkyl, Phenoxy-$C_{1-6}$-alkyl (wobei die Phenylgruppe gegebenenfalls durch eine oder mehrere $C_{1-3}$-Alkylgruppen, $C_{1-3}$-Alkoxygruppen oder Halogenatome substituiert sein kann), Amino-$C_{1-6}$-alkyl, $C_{1-6}$-Alkylamino-$C_{1-6}$-alkyl, Di-$C_{1-6}$-alkylamino-$C_{1-6}$-alkyl, Hydroxy oder $C_{1-6}$-Alkoxy steht;

mit der Maßgabe, daß, wenn X ein Sauerstoffatom oder —$NR_5$— darstellt und wenn n 0 ist, Q für Benzol steht.

4. Verfahren nach einem der Ansprüche 1 bis 3 zur Herstellung von Verbindungen, bei denen Q für Benzol steht, das in das Molekül durch Bindungen in 1- und 3-Stellungen eingefügt ist, n 0 ist und X für Sauerstoff steht.

5. Verfahren nach Anspruch 4 zur Herstellung von Verbindungen, bei denen $R_4$ für Wasserstoff, $C_{1-6}$-Alkyl, Hydroxy-$C_{1-6}$-alkyl, $C_{1-6}$-Alkoxy-$C_{1-6}$-alkyl, Acyloxy-$C_{1-6}$-alkyl, Phenyl-$C_{1-6}$-alkyl (wobei die Phenylgruppe gegebenenfalls durch eine oder mehrere $C_{1-3}$-Alkylgruppen, $C_{1-3}$-Alkoxygruppen oder Halogenatome substituiert sein kann) oder Hydroxy oder die Gruppe $NHCOR_9$, worin $R_9$ für Wasserstoff, $C_{1-6}$-Alkyl, $C_{1-6}$-Alkoxy, Phenyl (gegebenenfalls durch eine oder mehrere ($C_{1-3}$-Alkylgruppen, $C_{1-3}$-Alkoxygruppen oder Halogenatome substituiert) oder eine 5- oder 6-gliedrige monocyclische Heteroarylgruppe, enthaltend ein oder mehrere Heteroatome, ausgewählt aus Sauerstoff, Stickstoff und Schwefel, steht; oder die Gruppe $NHCONHR_{11}$, worin $R_{11}$ für $C_{1-6}$-Alkyl, Phenyl (gegebenenfalls durch eine oder mehrere $C_{1-3}$-Alkylgruppen, $C_{1-3}$-Alkoxygruppen oder Halogenatome substituiert) oder $C_{3-8}$-Cycloalkyl steht, steht.

6. Verfahren nach Anspruch 5 zur Herstellung von Verbindungen, bei denen $R_4$ für Hydroxy-$C_{1-6}$-alkyl, Hydroxy, $C_{1-6}$-Alkoxy-$C_{1-6}$-alkyl, Benzyl oder die Gruppe $NHCOR_9$, wobei $R_9$ für Wasserstoff, Phenyl, $C_{1-6}$-Alkyl oder $C_{1-6}$-Alkoxy steht, oder die Gruppe $NHCONHR_{11}$, wobei $R_{11}$ für Phenyl steht, steht.

7. Verfahren nach einem der Ansprüche 1 bis 4 zur Herstellung von Verbindungen, bei denen $R_4$ für Hydroxy-$C_{1-6}$-alkyl, Acyloxy-$C_{1-6}$-alkyl oder $C_{1-6}$-Alkoxy-$C_{1-6}$-alkyl steht.

8. Verfahren nach Anspruch 7 zur Herstellung von Verbindungen, bei denen $R_4$ für Hydroxy-$C_{1-6}$-alkyl steht.

9. Verfahren nach einem der Ansprüche 1 bis 8 zur Herstellung von Verbindungen, bei denen m+n 3 oder 4 ist.

10. Verfahren nach einem der Ansprüche 1 bis 9 zur Herstellung von Verbindungen, bei denen Alk für $—CH_2—$ steht.

11. Verfahren nach einem der Ansprüche 1 bis 10 zur Herstellung von Verbindungen, bei denen $R_1$ für Wasserstoff oder $C_{1-4}$-Alkyl steht und $R_2$ für $C_{3-5}$-Alkenyl, $C_{5-7}$-Cycloalkyl, Benzyl, $C_{1-8}$-Alkyl oder $C_{1-4}$-Alkyl, substituiert durch $C_{1-3}$-Alkoxy, Hydroxy, Di-$C_{1-3}$-alkylamino oder Trifluormethyl steht, oder $R_1$ und $R_2$ zusammen mit dem Stickstoffatom, an das sie angefügt sind, einen Ring mit 5 bis 8 Gliedern bilden, der gegebenenfalls eine Doppelbindung enthält und/oder durch Hydroxy oder eine oder zwei Methylgruppe(n) substituiert ist.

12. Verfahren nach Anspruch 11 zur Herstellung von Verbindungen, bei denen $R_1$ und $R_2$ für $C_{1-3}$-Alkyl stehen oder $R_1$ und $R_2$ zusammen mit dem Stickstoffatom, an das sie angefügt sind, einen heterocyclischen Ring bilden, der Pyrrolidin, Piperidin, das gegebenenfalls in 4-Stellung durch Methyl oder Hydroxy substituiert ist, Tetrahydropyridin, Morpholin, 2,6-Dimethylmorpholin, Hexamethylenimin oder Heptamethylenimin ist.

13. Verfahren nach einem der Ansprüche 1 bis 12 zur Herstellung von Verbindungen, bei denen $R_3$ für Wasserstoff, $C_{1-6}$-Alkyl oder Hydroxy-$C_{2-4}$-alkyl steht.

14. Verfahren nach Anspruch 1 oder 2 zur Herstellung von Verbindungen entsprechend der Formel (II)

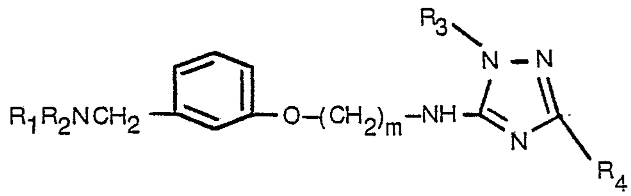

(II)

worin $R_1$ und $R_2$ Methylgruppen sind oder zusammen mit dem Stickstoffatom, an das sie angefügt sind, eine Pyrrolidino-, Piperidino- oder Hexamethyleniminogruppe bilden; m 3 oder 4 ist; $R_3$ für Wasserstoff oder Methyl steht und $R_4$ für eine Hydroxy-$C_{1-6}$-alkyl-, $C_{1-6}$-Alkoxy-$C_{1-6}$-alkyl-, Benzyl-, Formamido-, $C_{2-7}$-Alkanoylamino-, $C_{1-6}$-Alkoxycarbonylamino-, Hydroxy-, Benzoylamino- oder Phenylcarbamoylaminogruppe steht.

15. Verfahren zur Herstellung eines physiologisch annehmbaren Salzes der Verbindung der Formel (I) nach Anspruch 1 oder 2, nämlich 1-Methyl-5[[3-[3-(1-piperidinylmethyl)phenoxy]propyl]-amino]-1H-1,2,4-triazol-3-methanol, dadurch gekennzeichnet, daß man die freie Base durch Standardmethoden der Salzbildung in ein physiologisch annehmbares Salz umwandelt.

16. Verfahren zur Herstellung des Succinatsalzes von 1-Methyl-5-[[3-[3-(1-piperidinylmethyl)-phenoxy]propyl]amino]-1H-1,2,4-triazol-3-methanol, dadurch gekennzeichnet, daß man die freie Base in das Succinatsalz durch Standardmethoden der Salzbildung umwandelt.

17. Verfahren nach Anspruch 1(d) zur Herstellung von 1-Methyl-5-[[3-[3-(1-piperidinylmethyl)-phenoxy]propyl]amino]-1H-1,2,4-triazol-3-methanol, dadurch gekennzeichnet, daß man Methyl-1-methyl-5-[[1-oxo-4-[3-(1-piperidinylmethyl)phenoxy]propyl]amino]-1H-1,2,4-triazol-3-carboxylat reduziert.

18. Verfahren nach Anspruch 1(c) zur Herstellung von 1-Methyl-5-[[3-[3-[(1-piperidinylmethyl)-phenoxy]propyl]amino]-1H-1,2,4-triazol-3-methanol, dadurch gekennzeichnet, daß man das Produkt der Umsetzung von 3-[3-(1-Piperidinylmethyl)phenoxy]propanamin und Methyl-N-[2-(acetyloxy)-acetyl]-1-methyl-2-(phenylmethylen)hydrazin-carboximidothioat unter sauren Bedingungen cyclisiert.

**Revendications pour les Etats contractants: BE CH DE FR GB IT LI LU NL SE**

1. Composés de formule générale I

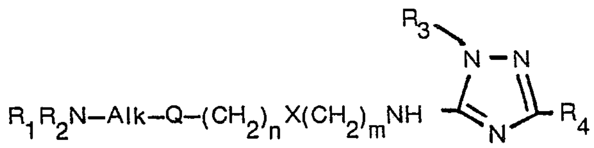

(I)

et leurs sels tolérés par l'organisme et hydrates de ces composés, dans lesquels:

$R_1$ représente l'hydrogène ou un groupe alkyle en C1—C4 et $R_2$ représente un groupe alkyle en C1—C10, cycloalkyle en C3—C8, alcényle en C3—C6, phényl-(alkyle en C1—C6) (dans lequel le groupe phényle peut éventuellement être substitué par un ou plusieurs groupes alkyle en C1—C3, alcoxy en C1—C3 ou atomes d'halogènes), trifluoroalkyle en C1—C6 ou alkyle en C1—C6 substitué par des groupes hydroxy, alcoxy en C1—C6, amino, alkylamin en C1—C6, ou di-(alkyle en C1—C6)-amino ou bien $R_1$ et $R_2$ peuvent former ensemble et avec l'atome d'azote sur lequel ils sont fixés un noyau de 5 à 10 chaînons qui peut être saturé ou peut contenir au moins une double liaison, peut être non substitué ou peut être substitué par un ou plusieurs groupes alkyle en C1—C3 ou par un groupe hydroxy et/ou peut contenir un autre hétéroatome qui est l'oxygène ou le soufre;

Alk représente une chaîne alkylène droite ou ramifiée en C1—C6;

Q représente un cycle furanne ou thiophène relié au reste de la molécule par des liaisons en positions 2 et 5, le cycle furanne portant facultativement un autre substituant $R_6$ voisin du groupe $R_1R_2N$—Alk—, ou bien Q représente un noyau benzénique relié au reste de la molécule par des liaisons dans les positions 1 et 3 ou 1 et 4;

$R_6$ représente un halogène ou un groupe alkyle en C1—C4 qui peut être substitué par des groupes hydroxy ou alcoxy en C1—C4;

X représente —$CH_2$—, —O—, —S— ou

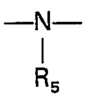

dans lequel $R_5$ représente l'hydrogène;

n est égal à 0, 1 ou 2;

m est égal à 2, 3 ou 4;

$R_3$ représente l'hydrogène, un groupe alkyle en C1—C6, alcényle en C3—C6, phényl-(alkyle en C1—C6) (dans lequel le groupe phényle peut être facultativement substitué par un ou plusieurs groupes alkyle en C1—C3, alcoxy en C1—C3 ou atomes d'halogènes), hydroxyalkyle en C2—C6 ou (alcoxy en C1—C6)-alkyle en C1—C6;

et $R_4$ répresente l'hydrogène, un groupe alkyle en C1—C6; alcényle en C3—C6, phényl-(alkyle en C1—C6) (dans lequel le groupe phényle peut être facultativement substitué par un ou plusieurs groupes alkyle en C1—C3, alcoxy en C1—C3 ou atomes d'halogènes), hydroxyalkyle en C1—C6, acyloxy-(alkyle en C1—C6) (dans lequel la partie acyle est un radical benzoyle, phényl-alcanoyle en C2—C7 ou alcanoyle en C1—C6, le noyau phényle étant facultativement substitué dans les deux premiers cas par un ou plusieurs groupes alkyle en C1—C3, alcoxy en C1—C3 ou atomes d'halogènes), (alcoxy en C1—C6)-alkyle en C1—C6, phénoxy-(alkyle en C1—C6) (dans lequel le groupe phényle peut être facultativement substitué par un ou plusieurs groupes alkyle en C1—C3, alcoxy en C1—C3 ou atomes d'halogènes), phényl-(alkyloxy en C1—C6)-alkyle en C1—C6 (dans lequel le groupe phényle peut être facultativement substitué par un ou plusieurs groupes alkyle en C1—C3, alcoxy en C1—C3 ou atomes d'halogènes), aminoalkyle en C1—C6, (alkylamino en C1—C6)-alkyle en C1—C6, di-(alkylamino en C1—C6)-alkyle en C1—C6, hydroxy, alcoxy en C1—C6 ou alkylthio en C1—C6 ou le groupe $NR_7R_8$ dans lequel

$R_7$ représente l'hydrogène ou un groupe alkyle en C1—C6, et

$R_8$ représente le groupe $COR_9$ dans lequel $R_9$ représente l'hydrogène, un groupe alkyle en C1—C6, phényle ou phényl-(alkyle en C1—C6) (dans les deux derniers cas, le groupe phényle pouvant être facultativement substitué par un ou plusieurs groupes alkyle en C1—C3, alcoxy en C1—C3 ou atomes d'halogènes), alcoxy en C1—C6, un groupe hétéroaryle monocyclique à 5 ou 6 chaînons contenant un ou plusieurs hétéroatomes choisis parmi l'oxygène, l'azote et le soufre, ou un groupe hétéroaryl-(alkyle en C1—C6) dans lequel le groupe hétéroaryle est un noyau monocyclique à 5 ou 6 chaînons contenant un ou plusieurs hétéroatomes choisis parmi l'oxygène, l'azote et le soufre, ou bien

$R_8$ représente le groupe $SO_2R_{10}$ dans lequel $R_{10}$ représente un groupe alkyle en C1—C6 ou phényle (éventuellement substitué par un ou plusieurs groupes alkyle en C1—C3, alcoxy en C1—C3 ou atomes d''halogènes), ou bien

$R_8$ représente le groupe

$$\underset{\substack{\| \\ Y}}{C}—NHR_{11}$$

dans lequel Y représente l'oxygène ou le soufre et $R_{11}$ représente l'hydrogène, un groupe alkyle en C1—C6, cycloalkyle en C3—C8, phényle ou phényl-(alkyle en C1—C6) (le groupe phényle pouvant, dans les deux cas, être facultativement substitué par un ou plusieurs groupes alkyle en C1—C3, alcoxy en C1—C3 ou atomes d'halogènes).

2. Composés selon la Rev. 1, à l'état de chlorhydrate, bromhydrate, sulfate, méthane-sulfonate, acétate, maléate, succinate, citrate, fumarate, benzoate ou tartrate.

3. Composés selon la Rev. 1, dans lesquels:

$R_4$ représente le groupe $NR_7R_8$ dans lequel $R_7$ et $R_8$ ont les significations indiquées dans la Rev. 1, sauf que $R_9$ ne représente pas un groupe hétéroaryle ou hétéroarylalkyle et $R_{11}$ ne représente pas un groupe cycloalkyle en C3—C8; ou bien $R_4$ représente l'hydrogène, un groupe alkyle en C1—C6, alcényle en C3—C6, phényl-(alkyle en C1—C6) (dans lequel le groupe phényle peut être facultativement substitué par un ou plusieurs groupes alkyle en C1—C3, alcoxy en C1—C3 ou atomes d'halogènes), hydroxyalkyle en C1—C6, (alcoxy en C1—C6)-alkyle en C1—C6, (phénoxy)-alkyle en C1—C6 (dans lequel le groupe phényle peut être facultativement substitué par un ou plusieurs groupes alkyle en C1—C3, alcoxy en C1—C3 ou atomes d'halogènes), aminoalkyle en C1—C6, (alkylamino en C1—C6)-alkyle en C1—C6, di-(alkylamino en C1—C6)-alkyle en C1—C6, hydroxy ou alcoxy en C1—C6;

étant spécifié que lorsque X représente un atome d'oxygène ou $—NR_5—$ et que n est égal à 0, Q représente le benzène.

4. Composés selon l'une quelconque des Rev. 1 à 3, dans lesquels Q représente le benzène relié à la molécule par des liaisons dans les positions 1 et 3, n est égal à 0 et X représente l'oxygène.

5. Composés selon la Rev. 4, dans lesquels $R_4$ représente l'hydrogène, un groupe alkyle en C1—C6, hydroxyalkyle en C1—C6, (alcoxy en C1—C6)-alkyle en C1—C6), acyloxy-(alkyle en C1—C6), phényl-(alkyle en C1—C6) (dans lequel le groupe phényle peut étre facultativement substitué par un ou plusieurs groupes alkyle en C1—C3, alcoxy en C1—C3 ou atomes d'halogènes) ou hydroxy, ou le groupe $NHCOR_9$ dans lequel $R_9$ représente l'hydrogène, un groupe alkyle en C1—C6, alcoxy en C1—C6, phényle(éventuellement substitué par un ou plusieurs groupes alkyle en C1—C3, alcoxy en C1—C3 ou atomes d'halogènes), ou un groupe hétéroaryle monocyclique à 5 ou 6 chaînons contenant un ou plusieurs hétéroatomes choisis parmi l'oxygène, l'azote ou le soufre; ou le groupe $NHCONHR_{11}$ dans lequel $R_{11}$ représente un groupe alkyle en C1—C6, phényle (éventuellement substitué par un ou plusieurs groupes alkyle en C1—C3, alcoxy en C1—C3 ou atomes d'halogènes) ou cycloalkyle en C3—C8.

6. Composés selon la Rev. 5, dans lesquels $R_4$ représente un groupe hydroxyalkyle en C1—C6, hydroxy (alcoxy en C1—C6)-alkyle en C1—C6, benzyle ou le groupe $NHCOR_9$ dans lequel $R_9$ représente l'hydrogène, un groupe phényle, alkyle en C1—C6 ou alcoxy en C1—C6, ou le groupe $NHCONHR_{11}$ dans lequel $R_{11}$ représente un groupe phényle.

7. Composés selon l'une quelconque des Rev. 1 à 4, dans lesquels $R_4$ représente un groupe hydroxyalkyle en C1—C6, acyloxy-(alkyle en C1—C6) ou (alcoxy en C1—C6)-alkyle en C1—C6.

8. Composés selon la Rev. 7, dans lesquels $R_4$ représente un groupe hydroxyalkyle en C1—C6.

9. Composés selon l'une quelconque des Rev. 1 à 8, dans lesquels $m + n$ est égal à 3 ou 4.

10. Composés selon l'une quelconque des Rev. 1 à 9, dans lesquels Alk représente $—CH_2—$.

11. Composés selon l'une quelconque des Rev. 1 à 10, dans lesquels $R_1$ représente l'hydrogène ou un groupe alkyle en C1—C4 et $R_2$ représente un groupe alcényle en C3—C5, cycloalkyle en C5—C7, benzyle, alkyle en C1—C8 ou alkyle en C1—C4, substitué par des groupes alcoxy en C1—C3, hydroxy, di-(alkyle en C1—C3)-amino ou trifluorométhyle, ou bien $R_1$ et $R_2$ forment ensemble et avec l'atome d'azote sur lequel ils sont fixés un noyau à 5 ou 8 chaînons contenant facultativement une double liaison et/ou substitué par un groupe hydroxy ou 1 ou 2 groupes méthyle.

12. Composés selon la Rev. 11, dans lesquels $R_1$ et $R_2$ représentent des groupes alkyle en C1—C3, ou bien $R_1$ et $R_2$ forment ensemble et avec l'atome d'azote sur lequel ils sont fixés un noyau hétérocyclique qui est un noyau pyrrolidine, pipéridine éventuellement substitué en position 4 par un groupe méthyle ou hydroxy, tétrahydropyridine, morpholine, 2,6-diméthylmorpholine, hexaméthylène-imine ou heptaméthylène-imine.

13. Composés selon l'une quelconque des Rev. 1 à 12, dans lesquels $R_3$ représente l'hydrogène, un groupe alkyle en C1—C6 ou hydroxyalkyle en C2—C4.

14. Composés selon la Rev. 1 ou 2, répondant à la formule II

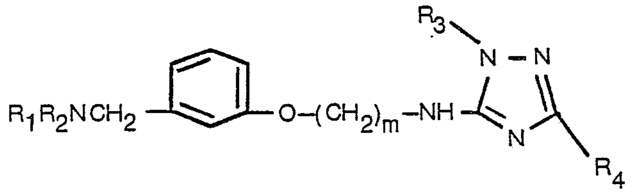

(II)

dans laquelle $R_1$ et $R_2$ représentent des groupes méthyle ou forment ensemble et avec l'atome d'azote sur lequel ils sont fixés un groupe pyrrolidino, pipéridino ou hexaméthylène-imino; m est égal à 3 ou 4; $R_3$ représente l'hydrogène ou un groupe méthyle et $R_4$ un groupe hydroxy, alkyle en C1—C6, (alcoxy en C1—C6)-alkyle en C1—C6, benzyle, formamido, alcanoylamino en C2—C7, (alcoxy en C1—C6)-carbonylamino, hydroxy, benzoylamino ou phénylcarbamoylamino.

15. Le composé selon la Rev. 1 ou 2, consistant en le 1 - méthyl - 5 - [[3 - [3 - (1 - pipéridinylméthyl)phénoxy]propyl] - amino] - 1H - 1,2,4 - triazole - 3 - méthanol et ses sels tolérés par l'organisme.

16. Le succinate du 1 - méthyl - 5 - [[3 - [3 - (1 - pipéridinylméthyl) - phénoxy] - propyl] - amino] - 1H - 1,2,4 - triazole - 3 - méthanol.

17. Le composé selon la Rev. 1 ou 2, consistant en le 5 - [[2 - [[[5 - [(diméthylamino) - méthyl] - 2 - furannyl] - méthyl] - thiol] - éthyl] - amino] - 1 - méthyl - 1H - 1,2,4 - triazole - 3 - méthanol et ses sels tolérés par l'organisme.

18. Le composé selon la Rev. 1 ou 2, consistant en le 5 - [[2 - [[[5 - [(diméthylamino) - méthyl] - 4 - méthyl - 2 - furannyl] - méthyl] - thio] - éthyl] - amino] - 1 - méthyl - 1H - 1,2,4 - triazole - 3 - méthanol et ses sels tolérés par l'organisme.

19. Le composé selon la Rev. 1 ou 2, consistant en le 1 - méthyl - 5 - [[3 - [3 - [(diméthylamino) - méthyl] - phénoxy] - propyl] - amino] - 1H - 1,2,4 - triazole - 3 - méthanol et ses sels tolérés par l'organisme.

20. Le composé selon la Rev. 1 ou 2, consistant en la 5 - [[3 - [3 - (pipéridinlyméthyl) - phénoxy] - propyl] - amino] - 1H - 1,2,4 - triazole - 3 - one et ses sels tolérés par l'organisme.

21. Composés selon la Rev. 1 ou 2, consistant en
1 - méthyl - 5 - [[4 - [3 - (1 - pipéridinylméthyl)phénoxy]butyl]amino] - 1H - 1,2,4 - triazole - 3 - méthanol,
N - [1 - méthyl - 5 - [[3 - [3 - (1 - pipéridinylméthyl)phénoxy]propyl]amino] - 1H - 1,2,4 - triazole - 3 - yl]formamide,
3 - méthoxyméthyl - 1 - méthyl - 5 - [[4 - [3 - (1 - pipéridinylméthyl)phénoxy]butyl]amino] - 1H - 1,2,4 - triazole et leurs sels tolérés par l'organisme.

22. Composés selon la Rev. 1 ou 2, consistant en
3 - phénylméthyl - N - [3 - [3 - (diméthylaminométhyl)phénoxy]propyl] - 1H - 1,2,4 - triazole - 5 - amine,
5 - [4 - [3 - (1 - pipéridinylméthyl)phénoxy]butyl]amino - 1H - 1,2,4 - triazole - 3 - méthanol,
3 - phénylméthyl - N - [3 - [3 - (1 - pipéridinylméthyl)phénoxy]propyl] - 1H - 1,2,4 - triazole - 5 - amine,
1 - méthyl - 5 - [[3 - [3 - [(diméthylamino)méthyl]phénoxy]propyl]amino] - 1H - 1,2,4 - triazole - 3 - carbamate d'éthyle,
N - [5 - [[3 - [3 - [(diméthylamino)méthyl]phénoxy]propyl]amino] - 1 - méthyl - 1H - 1,2,4 - triazole - 3 - yl]benzamide,
N - [5 - [[3 - [3 - (1 - pipéridinylméthyl)phénoxy]propyl]amino] - 1 - méthyl - 1H - 1,2,4 - triazole - 3 - yl] - N' - phénylurée,
et leurs sels tolérés par l'organisme.

23. Un procédé pour préparer les composés selon la Rev. 1 ou 2, qui comprend:

(a) pour la préparation de composés dans lesquels $R_4$ représente un atome d'hydrogène, le traitement d'un sel de diazonium III

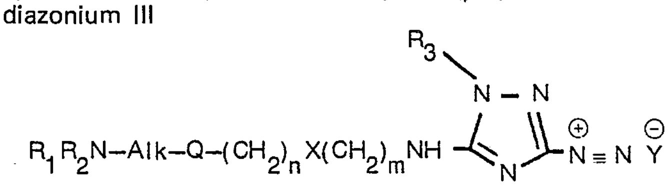

(III)

dans lequel $R_1$, $R_2$ et $R_3$ ont les significations indiquées en référence à la formule I ou représentent des groupes convertibles en ces significations, Alk, Q, n, X et m ont les significations indiquées en référence à la formule I et $\overset{\ominus}{Y}$ est l'aniun correspondant à l'acide utilisé dans la réaction de diazotation, par un acide minéral concentre qui convertit le groupe $-\overset{\oplus}{N}\equiv N\overset{\ominus}{Y}$ en un atome d'hydrogène;

(b) pour la préparation de composés dans lesquels $R_4$ représente le groupe $NR_7R_8$ et $R_8$ représente $COR_9$, $SO_2R_{10}$ ou $C(=Y)NHR_{11}$, le traitement d'un aminotriazole IV

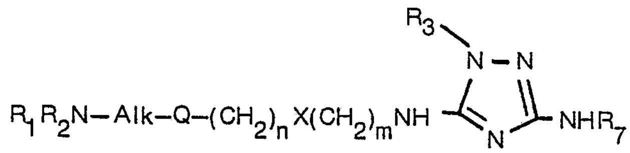

(IV)

dans lequel $R_1$, $R_2$, $R_3$ et $R_7$ ont les significations indiquées en référence à la formule I ou représentent des groupes facilement convertibles en ces significations, par un dérivé activé d'un acide carboxylique $R_9COOH$ ou d'un acide sulfonique $R_{10}SO_3H$, $R_9$ et $R_{10}$ ayant les significations indiquées en référence à la formule I, ou par un isocyanate ou isothiocyanate $R'_{11}NCY$, dans lequel $R'_{11}$ a l'une quelconque des significations données pour $R_{11}$ en référence à la formule I à l'exception de l'hydrogène, ou représente un atome de métal alcalin ou un groupe alcoxycarbonyle, afin de remplacer l'atome d'hydrogène du groupe $NHR_7$ respectivement par le groupe $COR_9$, $SO_2R_{10}$ ou $C(=Y)NHR_{11}$;

(c) pour la préparation de composés dans lesquels $R_4$ a une signification autre qu'un groupe alcoxy, alkylthio ou acyloxyalkyle, la cyclisation d'un composé de formule V

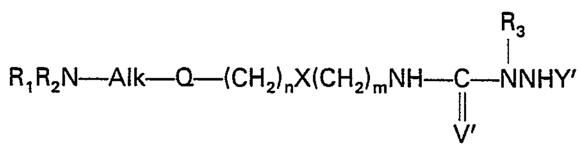

(V)

dans laquelle $R_1$, $R_2$, Alk, Q, X, $R_3$, n et m ont les significations indiquées en référence à la formule I et V' représente le groupe

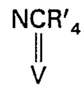

et Y' représente l'hydrogène lorsque V représente l'oxygène ou le soufre et $R'_4$ est un groupe tel que défini pour $R_4$ ou un groupe convertible en un tel groupe dans les conditions de la réaction de cyclisation ou bien représente un halogène ou un groupe alcoxy; ou bien V' représente NH et Y' représente

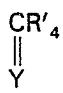

dans lequel Y représente le soufre, l'oxygène ou NH, Y ne pouvant représenter NH lorsque $R'_4$ représente un halogène ou un groupe alcoxy, ou bien V' représente le soufre ou l'oxygène et Y' le groupe

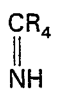

avec protection facultative et élimination subséquente des groupes protecteurs de groupes réactifs quelconques du produit de départ si on le désire;

(d) la réduction d'un composé de formule XV

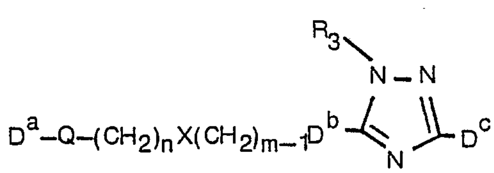

(XV)

dans laquelle Q, n, X, m et $R_3$ ont les significations indiquées en référence à la formule I, l'un au moins des symboles $D^a$, $D^b$, et $D^c$ représente un groupe réductible et le ou les autres ont les significations appropriées correspondant à la formule I,

$D^a$ représent $R_1R_2NAlk$- ou un groupe convertible en ce dernier dans des conditions réductrices, $R'_1$, $R_2$ et Alk ayant les significations indiquées en référence à la formule I;

$D^b$ représente $—CH_2NH—$, $—CONH—$ ou $—CH{=}N—$;

$D^c$ représente $R_4$ tel que défini en référence à la formule I ou un groupe convertible en un tel groupe dans des conditions réductrices, avec protection facultative et élimination subséquente des groupes protecteurs de groupes réactifs quelconques du produit de départ si on le désire;

(e) la réaction d'un composé de formule XX

$$R_1R_2NAlkQE \quad (XX)$$

dans laquelle E représente $(CH_2)_nX(CH_2)_mP$ ou $CH_2P'$, P et P' représentant des groupes éliminables, avec un triazole de formule XVII

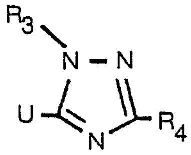

(XVII)

dans laquelle U représente un groupe amino $HS(CH_2)_mNH$ ou $HO\ (CH_2)_mNH$, et $R_4$ a la signification indiquée en référence à la formule I ou représente un groupe convertible en une telle signification avec protection facultative et élimination subséquente des groupes protecteurs de groupes réactifs quelconques du produit de départ si on le désire;

(f) l'introduction du groupe $R_1R_2N$—Alk dans le groupe Q présent dans un produit intermédiaire approprié par l'un des moyens suivants:

(i) pour la préparation de composés dans lesquels Q représente le furanne ou le furanne substitué, et Alk représente le groupe méthylène, une réaction du produit intermédiaire avec le formaldéhyde et une amine $R_1R_2NH$ ou un sel d'une telle amine ($R_1$ et $R_2$ ayant les significations indiquées en référence à la formule I) ou bien

(ii) pour la préparation de composés dans lesquels $R_1$ et $R_2$ représentent tous deux des groupes méthyle, Alk représente un groupe méthylène et Q le furanne, le furanne substitué ou le thiophène, la réaction du produit intermédiaire avec le composé $(CH_3)_2\overset{\oplus}{N}{=}CH_2\overset{\ominus}{Cl}$, avec dans les deux cas protection facultative et élimination subséquente des groupes protecteurs de groupes réactifs quelconques du produit de départ si on le désire;

(g) pour la préparation de composés dans lesquels $R_4$ représente un groupe hydroxyalkyle secondaire ou tertiaire, le traitement du composé correspondant dans lequel $R_4$ représente le groupe $(CH_2)_{q-1}$ CHO, $(CH_2)_{q-1}CO_2R_{12}$ ou $(CH_2)_rCOR_{12}$ dans lesquels q est un nombre de 1 à 6 inclus, r un nombre de 0 à 4 et $R_{12}$ un groupe alkyle, avec un dérivé organométallique;

(h) la réaction d'un composé de formule XXII

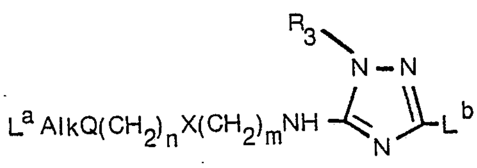

(XXII)

dans laquelle ou bien $L^a$ représente $R_1R_2N$ et $L^b$ représente le groupe $(CH_2)_qL^c$, ou bien $L^a$ est un groupe $L^d$ et $L^b$ représente $R_4$, $L^c$ et $L^d$ étant des groupes éliminables et q un nombre de 1 à 6 inclus, avec un composé capable de remplacer $L^d$ par $R_1R_2N$ ou $L^c$ de manière à convertir $L^b$ en le groupe $R_4$, avec protection facultative et élimination subséquente des groupes protecteurs de groupes réactifs quelconques du produit de départ si on le désire;

(i) pour la préparation de composés dans lesquels $R_4$ représente un groupe alcoxyalkyle, la conversion du groupe $R_4$ d'un produit intermédiaire dans lequel ce groupe est un groupe hydroxyalkyle en un groupe alcoxyalkyle par des modes opératoires classiques d'interconversion;

(j) pour la préparation de composés dans lesquels $R_4$ représente un groupe acyloxyalkyle, le traitement du composé correspondant à un groupe hydroxyalkyle par un acide approprié à température élevée; ou bien

(k) pour la préparation de composés dans lesquels $R_4$ est un groupe hydroxyalkyle, l'élimination d'un groupe hydroxy, du groupe protecteur le protégeant;

et lorsque le composé de formule I est obtenu à l'état de base libre, la conversion éventuelle de la base libre en un sel.

24. Une composition pharmaceutique comprenant un composé selon l'une quelconque des Rev. 1 à 22 avec au moins un véhicule ou diluant acceptable pour l'usage pharmaceutique et, facultativement, un ou plusieurs autres composants actifs.

25. Une composition pharmaceutique selon la Rev. 24, sous forme adaptée à l'administration orale.

26. Une composition pharmaceutique selon la Rev. 25, contenant 5 mg à 1 g du composé de formule 1.

27. Une composition pharmaceutique selon la Rev. 26, contenant 5 mg à 250 mg du composé de formule 1.

**Revendications pour l'Etat contractant: AT**

1. Un procédé de préparation des composés de formule générale I

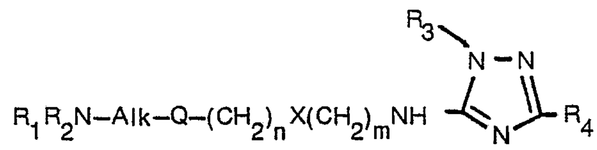

(I)

et de leurs sels tolérés par l'organisme et hydrates de ces composés, dans lesquels:

$R_1$ représente l'hydrogène ou un groupe alkyle en C1—C4 et $R_2$ représente un groupe alkyle en C1—C10, cycloalkyle en C3—C8, alcényle en C3—C6, phényl-(alkyle en C1—C6) (dans lequel le groupe phényle peut éventuellement être substitué par un ou plusieurs groupes alkyle en C1—C3, alcoxy en C1—C3 ou atomes d'halogènes), trifluoroalkyle en C1—C6 ou alkyle en C1—C6 substitué par des groupes hydroxy, alcoxy en C1—C6, amino, alkylamin en C1—C6, ou di-(alkyle en C1—C6)-amino ou bien $R_1$ et $R_2$ peuvent former ensemble et avec l'atome d'azote sur lequel ils sont fixés un noyau de 5 à 10 chaînons qui peut être saturé ou peut contenir au moins une double liaison, peut être non substitué ou peut être substitué par un ou plusieurs groupes alkyle en C1—C3 ou par un groupe hydroxy et/ou peut contenir un autre hétéroatome qui est l'oxygène ou le soufre;

Alk représente une chaîne alkylène droite ou ramifiée en C1—C6;

Q représente un cycle furanne ou thiophène relié au reste de la molécule par des liaisons en positions 2 et 5, le cycle furanne portant facultativement un autre substituant $R_6$ voisin du groupe $R_1R_2N$—Alk—, ou bien Q représente un noyau benzénique relié au reste de la molécule par des liaisons dans les positions 1 et 3 ou 1 et 4;

$R_6$ représente un halogène ou un groupe alkyle en C1—C4 qui peut être substitué par des groupes hydroxy ou alcoxy en C1—C4;

X représente —$CH_2$—, —O—, —S— ou

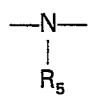

dans lequel $R_5$ représente l'hydrogène;

n est égal à 0, 1 ou 2;

m est égal à 2, 3 ou 4;

$R_3$ représente l'hydrogène, un groupe alkyle en C1—C6, alcényle en C3—C6, phényl-(alkyle en C1—C6) (dans lequel le groupe phényle peut être facultativement substitué par un ou plusieurs groupes alkyle en C1—C3, alcoxy en C1—C3 ou atomes d'halogènes), hydroxyalkyle en C2—C6 ou (alcoxy en C1—C6)-alkyle en C1—C6;

et $R_4$ répresente l'hydrogène, un groupe alkyle en C1—C6; alcényle en C3—C6, phényl-(alkyle en C1—C6) (dans lequel le groupe phényle peut être facultativement substitué par un ou plusieurs groupes alkyle en C1—C3, alcoxy en C1—C3 ou atomes d'halogènes), hydroxyalkyle en C1—C6, acyloxy-(alkyle en C1—C6) (dans lequel la partie acyle est un radical benzoyle, phényl-alcanoyle en C2—C7 ou alcanoyle en C1—C6, le noyau phényle étant facultativement substitué dans les deux premiers cas par un ou plusieurs groupes alkyle en C1—C3, alcoxy en C1—C3 ou atomes d'halogènes), (alcoxy en C1—C6)-alkyle en C1—C6, phénoxy-(alkyle en C1—C6) (dans lequel le groupe phényle peut être facultativement substitué par un ou plusieurs groupes alkyle en C1—C3, alcoxy en C1—C3 ou atomes d'halogènes), phényl-(alkyloxy en C1—C6)-alkyle en C1—C6 (dans lequel le groupe phényle peut être facultativement substitué par un ou plusieurs groupes alkyle en C1—C3, alcoxy en C1—C3 ou atomes d'halogènes), aminoalkyle en C1—C6, (alkylamino en C1—C6)-alkyle en C1—C6, di-(alkylamino en C1—C6)-alkyle en C1—C6, hydroxy, alcoxy en C1—C6 ou alkylthio en C1—C6 ou le groupe $NR_7R_8$ dans lequel

$R_7$ représente l'hydrogène ou un groupe alkyle en C1—C6, et

$R_8$ représente le groupe $COR_9$ dans lequel $R_9$ représente l'hydrogène, un groupe alkyle en C1—C6, phényle ou phényl-(alkyle en C1—C6) (dans les deux derniers cas, le groupe phényle pouvant être facultativement substitué par un ou plusieurs groupes alkyle en C1—C3, alcoxy en C1—C3 ou atomes d'halogènes), alcoxy en C1—C6, un groupe hétéroaryle monocyclique à 5 ou 6 chaînons contenant un ou plusieurs hétéroatomes choisis parmi l'oxygène, l'azote et le soufre, ou un groupe hétéroaryl-(alkyle en C1—C6) dans lequel le groupe hétéroaryle est un noyau monocyclique à 5 ou 6 chaînons contenant un ou plusieurs hétéroatomes choisis parmi l'oxygène, l'azote et le soufre, ou bien

$R_8$ représente le groupe $SO_2R_{10}$ dans lequel $R_{10}$ représente un groupe alkyle en C1—C6 ou phényle (éventuellement substitué par un ou plusieurs groupes alkyle en C1—C3, alcoxy en C1—C3 ou atomes d'halogènes), ou bien

$R_8$ représente le groupe

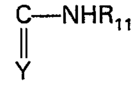

dans lequel Y représente l'oxygène ou le soufre et $R_{11}$ représente l'hydrogène, un groupe alkyle en C1—C6, cycloalkyle en C3—C8, phényle ou phényl-(alkyle en C1—C6) (le groupe phényle pouvant, dans les deux cas, être facultativement substitué par un ou plusieurs groupes alkyle en C1—C3, alcoxy en C1—C3 ou atomes d'halogènes), qui comprend:

(a) pour la préparation de composés dans lesquels $R_4$ représente un atome d'hydrogène, le traitement d'un sel de diazonium III

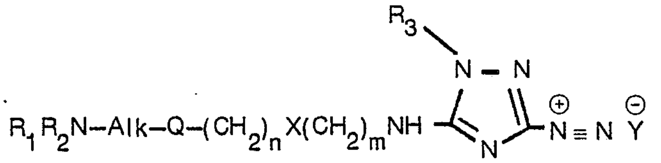

(III)

dans lequel $R_1$, $R_2$ et $R_3$ ont les significations indiquées en référence à la formule I ou représentent des groupes convertibles en ces significations, Alk, Q, n, X et m ont les significations indiquées en référence à la formule I et $\overset{\ominus}{Y}$ est l'anion correspondant à l'acide utilisé dans la réaction de diazotation, par un acide minéral concentré qui convertit le groupe $—\overset{\oplus}{N}{\equiv}N\overset{\ominus}{Y}$ en un atome d'hydrogène;

(b) pour la préparation de composés dans lesquels $R_4$ représente le groupe $NR_7R_8$ où $R_8$ représente $COR_9$, $SO_2R_{10}$ ou C $(=Y)NHR_{11}$, le traitement d'un aminotriazole IV

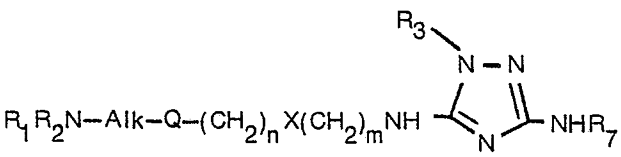

(IV)

dans lequel $R_1$, $R_2$, $R_3$ et $R_7$ ont les significations indiquées en référence en à la formule I ou représentent des groupes facilement convertibles en ces significations, par un dérivé activé d'un acide carboxylique $R_9COOH$ ou d'un acide sulfonique $R_{10}SO_3H$, $R_9$ et $R_{10}$ ayant les significations indiquées en référence à la formule I, ou par un isocyanate ou isothiocyanate $R'_{11}NCY$, dans lequel $R'_{11}$ a l'une quelconque des significations données pour $R_{11}$ en référence à la formule I à l'exception de l'hydrogène, ou représente un atome de métal alcalin ou un groupe alcoxycarbonyle, afin de remplacer l'atome d'hydrogène du groupe $NHR_7$ respectivement par le groupe $COR_9$, $SO_2R_{10}$ ou $C(=Y)NHR_{11}$;

(c) pour la préparation de composés dans lesquels $R_4$ a une signification autre qu'un groupe alcoxy, alkylthio ou acyloxyalkyle, la cyclisation d'un composé de formule V

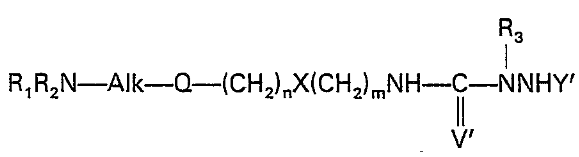

(V)

dans laquelle $R_1$, $R_2$, Alk, Q, X, $R_3$, n et m ont les significations indiquées en référence à la formule I et V' représente le groupe

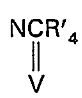

et Y' représente l'hydrogène lorsque V représente l'oxygène ou le soufre et $R'_4$ est un groupe tel que défini pour $R_4$ ou un groupe convertible en un tel groupe dans les conditions de la réaction de cyclisation ou bien représente un halogène ou un groupe alcoxy; ou bien V' représente NH et Y' représente

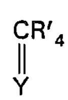

dans lequel Y représente le soufre, l'oxygène ou NH, Y ne pouvant représenter NH lorsque $R'_4$

représente un halogène ou un groupe alcoxy, ou bien V′ représente le soufre ou l'oxygène et Y′ le groupe

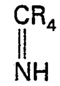

avec protection facultative et élimination subséquente des groupes protecteurs de groupes réactifs quelconques du produit de départ si on le désire;

(d) la réduction d'un composé de formule XV

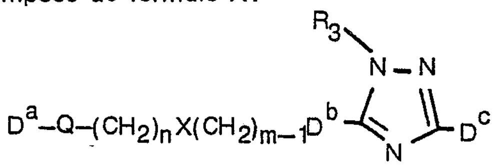

(XV)

dans laquelle Q, n, X, m et $R_3$ ont les significations indiquées en référence à la formule I, l'un au moins des symboles $D^a$, $D^b$, et $D^c$ représente un groupe réductible et le ou les autres ont les significations appropriées correspondant à la formule I,

$D^a$ représente $R_1R_2NAlk$- ou un groupe convertible en ce dernier dans des conditions réductrices, $R_1$, $R_2$ et Alk ayant les significations indiquées en référence à la formule I;

$D^b$ représente $—CH_2NH—$, $—CONH—$ ou $CH{=}N—$;

$D^c$ représente $R_4$ tel que défini en référence à la formule I ou un groupe convertible en un tel groupe dans des conditions réductrices, avec protection facultative et élimination subséquente des groupes protecteurs de groupes réactifs quelconques du produit de départ si on le désire;

(e) la réaction d'un composé de formule XX

$$R_1R_2NAlkQE \qquad (XX)$$

dans laquelle E représente $(CH_2)_nX(CH_2)_mP$ ou $CH_2P'$, P et P′ représentant des groupes éliminables, avec un triazole de formule XVII

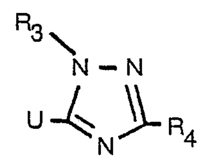

(XVII)

dans laquelle U représente un groupe amino $HS(CH_2)_mNH$ ou $HO(CH_2)_mNH$, et $R_4$ a la signification indiquée en référence à la formule I ou représente un groupe convertible en une telle signification avec protection facultative et élimination subséquente des groupes protecteurs de groupes réactifs quelconques du produit de départ si on le désire;

(f) l'introduction du groupe $R_1R_2N—Alk$ dans le groupe Q présent dans un produit intermédiaire approprié par l'un des moyens suivants:

(i) pour la préparation de composés dans lesquels Q représente le furanne ou le furanne substitué, et Alk représente le groupe méthylène, une réaction du produit intermédiaire avec le formaldéhyde et une amine $R_1R_2NH$ ou un sel d'une telle amine ($R_1$ et $R_2$ ayant les significations indiquées en référence à la formule I) ou bien

(ii) pour la préparation de composés dans lesquels $R_1$ et $R_2$ représentent tous deux des groupes méthyle, Alk représente un groupe méthylène et Q le furanne, le furanne substitué ou le thiophène, la réaction du produit intermédiaire avec le composé $(CH_3)_2\overset{\oplus}{N}{=}CH_2\overset{\ominus}{Cl}$, avec dans les deux cas protection facultative et élimination subséquente des groupes protecteurs de groupes réactifs quelconques du produit de départ si on le désire;

(g) pour la préparation de composés dans lesquels $R_4$ représente un groupe hydroxyalkyle secondaire ou tertiaire, le traitement du composé correspondant dans lequel $R_4$ représente le groupe $(CH_2)_{q-1}CHO$, $(CH_2)_{q-1}CO_2R_{12}$ ou $(CH_2)_rCOR_{12}$ dans lesquels q est un nombre de 1 à 6 inclus, r un nombre de 0 à 4 et $R_{12}$ un groupe alkyle, avec un dérivé organométallique;

(h) la réaction d'un composé de formule XXII

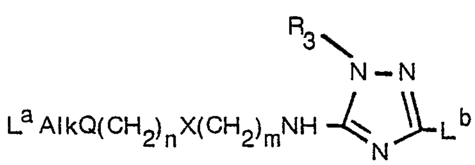

(XXII)

dans laquelle ou bien $L^a$ représente $R_1R_2N$ et $L^b$ représente le groupe $(CH_2)_qL^c$, ou bien $L^a$ est un groupe

$L^d$ et $L^b$ représente $R_4$, $L^c$ et $L^d$ étant des groupes éliminables et q un nombre de 1 à 6 inclus, avec un composé capable de remplacer $L^d$ par $R_1R_2N$ ou $L^c$ de manière à convertir $L^b$ en le groupe $R_4$, avec protection facultative et élimination subséquente des groupes protecteurs de groupes réactifs quelconques du produit de départ si on le désire;

(i) pour la préparation de composés dans lesquels $R_4$ représente un groupe alcoxyalkyle, la conversion du groupe $R_4$ d'un produit intermédiaire dans lequel ce groupe est un groupe hydroxyalkyle en un groupe alcoxyalkyle par des modes opératoires classiques d'interconversion;

(j) pour la préparation de composés dans lesquels $R_4$ représente un groupe acyloxyalkyle, le traitement du composé correspondant à un groupe hydroxyalkyle par un acide approprié à température élevée; ou bien

(k) pour la préparation de composés dans lesquels $R_4$ est un groupe hydroxyalkyle, l'élimination d'un groupe hydroxy, du groupe protecteur le protégeant;

et lorsque le composé de formule I est obtenu à l'état de base libre, la conversion éventuelle de la base libre en un sel.

2. Un procédé selon la Rev. 1, pour la préparation des composés sous la forme de chlorhydrate, bromhydrate, sulfate, méthane-sulfonate, acétate, maléate, succinate, citrate, fumarate, benzoate ou tartrate.

3. Un procédé selon la Rev. 1, pour la préparation de composés dans lesquels $R_4$ représente le groupe $NR_7R_8$ dans lequel $R_7$ et $R_8$ ont les significations indiquées dans la Rev. 1, sauf que $R_9$ ne représente pas un groupe hétéroaryle ou hétéroarylalkyle et $R_{11}$ ne représente pas un groupe cycloalkyle en C3—C8; ou bien $R_4$ représente l'hydrogène, un groupe alkyle en C1—C6, alcényle en C3—C6, phényl-(alkyle en C1—C6) (dans lequel le groupe phényle peut être facultativement substitué par un ou plusieurs groupes alkyle en C1—C3, alcoxy en C1—C3 ou atomes d'halogènes), hydroxyalkyle en C1—C6, (alcoxy en C1—C6)-alkyle en C1—C6, (phénoxy)-alkyle en C1—C6 (dans lequel le groupe phényle peut être facultativement substitué par un ou plusieurs groupes alkyle en C1—C3, alcoxy en C1—C3 ou atomes d'halogènes), aminoalkyle en C1—C6, (alkylamino en C1—C6)-alkyle en C1—C6, di-(alkylamino en C1—C6)-alkyle en C1—C6, hydroxy ou alcoxy en C1—C6;

étant spécifié que lorsque X représente un atome d'oxygène ou $—NR_5—$ et que n est égal à 0, Q représente le benzène.

4. Un procédé selon l'une quelconque des Rev. 1 à 3, pour la préparation de composés dans lesquels Q représente le benzène relié au reste de la molécule par des liaisons dans les positions 1 et 3, n est égal à 0 et X représente l'oxygène.

5. Un procédé selon la Rev. 4, pour la préparation de composés dans lesquels $R_4$ représente l'hydrogène, un groupe alkyle en C1—C6, hydroxyalkyle en C1—C6, (alcoxy en C1—C6)-alkyle en C1—C6, acyloxy-(alkyle en C1—C6), phényl-(alkyle en C1—C6) (dans lequel le groupe phényle peut être facultativement substitué par un ou plusieurs groupes alkyle en C1—C3, alcoxy en C1—C3 ou atomes d'halogènes) ou hydroxy, ou le groupe $NHCOR_9$ dans lequel $R_9$ représente l'hydrogène, un groupe alkyle en C1—C6, alcoxy en C1—C6, phényle (éventuellement substitué par un ou plusieurs groupes alkyle en C1—C3, alcoxy en C1—C3 ou atomes d'halogènes), ou un groupe hétéroaryle monocylique à 5 ou 6 chaînons contenant un ou plusieurs hétéroatomes choisis parmi l'oxygène, l'azote ou le soufre; ou le groupe $NHCONHR_{11}$ dans lequel $R_{11}$ représente un groupe alkyle en C1—C6, phényle (éventuellement substitué par un ou plusieurs groupes alkyle en C1—C3, alcoxy en C1—C3 ou atomes d'halogènes) ou cycloalkyle en C3—C8

6. Un procédé selon la Rev. 5, pour la préparation de composés dans lesquels $R_4$ représente un groupe hydroxyalkyle en C1—C6, hydroxy, alcoxy en C1—C6-(alkyle en C1—C6), benzyle ou le groupe $NHCOR_9$ dans lequel $R_9$ représente l'hydrogène, un groupe phényl-(alkyle en C1—C6) ou alcoxy en C1—C6 ou le groupe $NHCONHR_{11}$ dans lequel $R_{11}$ est un groupe phényle.

7. Un procédé selon l'une quelconque des Rev. 1 à 4, pour la préparation de composés dans lesquels $R_4$ représente un groupe hydroxyalkyle en C1—C6, acyloxy-(alkyle en C1—C6) ou (alcoxy en C1—C6)-alkyle en C1—C6.

8. Un procédé selon la Rev. 7, pour la préparation de composés dans lesquels $R_4$ représente un groupe hydroxyalkyle en C1—C6.

9. Un procédé selon l'une quelconque des Rev. 1 à 8, pour la préparation de composés dans lesquels $m + n = 3$ ou 4.

10. Un procédé selon l'une quelconque des Rev. 1 à 9, pour la préparation de composés dans lesquels Alk représente $—CH_2—$.

11. Un procédé selon l'une quelconque des Rev. 1 à 10, pour la préparation de composés dans lesquels $R_1$ représente l'hydrogène ou un groupe alkyle en C1—C4 et $R_2$ représente un groupe alcényle en C3—C5, cycloalkyle en C5—C7, benzyle, alkyle en C1—C8 ou alkyle en C1—C4 substitué par un groupe alcoxy en C1—C3, hydroxy, di-(alkyle en C1—C3)-amino ou trifluorométhyle, ou bien $R_1$ et $R_2$ forment ensemble et avec l'atome d'azote sur lequel ils sont fixés un noyau à 5 ou 8 chaînons contenant facultativement une double liaison et/ou substitué par un groupe hydroxy ou 1 ou 2 groupes méthyle.

12. Un procédé selon la Rev. 11, pour la préparation de composés dans lequels $R_1$ et $R_2$ représentent des groupes alkyle en C1—C3 ou bien $R_1$ et $R_2$ forment ensemble et avec l'atome d'azote

sur lequel ils sont fixés un noyau hétérocyclique consistant en un noyau pyrrolidine, pipéridine éventuellement substitué en position 4 par un groupe méthyle ou hydroxy, tétrahydropyridine, morpholine, 2,6-diméthylmorpholine, hexaméthylène-imine ou heptaméthylène-imine.

13. Un procédé selon l'une quelconque des Rev. 1 à 12, pour la préparation de composés dans lesquels $R_3$ représente l'hydrogène, un groupe alkyle en C1—C6 ou hydroxyalkyle en C2—C4.

14. Un procédé selon la Rev. 1 ou 2, pour la préparation de composés répondant à la formule II

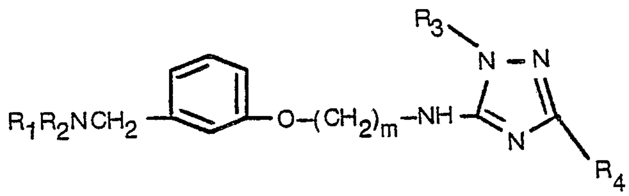

(II)

dans laquelle $R_1$ et $R_2$ représentent des groupes méthyle ou forment ensemble et avec l'atome d'azote sur lequel ils sont fixés un groupe pyrrolidino, pipéridino ou hexaméthylène-imino; m est égal à 3 ou 4; $R_3$ représente l'hydrogène ou un groupe méthyle et $R_4$ un groupe hydroxy, alkyle en C1—C6, (alcoxy en C1—C6)-alkyle en C1—C6, benzyle, formamido, alcanoylamino en C2—C7, (alcoxy en C1—C6)-carbonylamino, hydroxy, benzoylamino ou phénylcarbamoylamino.

15. Un procédé pour la préparation d'un sel toléré par l'organisme du composé de formule I tel que défini dans la Rev. 1 ou 2 et consistant en le 1 - méthyl - 5 - [[3 - [3 - (1 - pipéridinylméthyl) - phénoxy] - propy] - amino] - 1H - 1,2,4 - triazole - 3 - méthanol, qui consiste à convertir la base libre en un sel toléré par l'organisme par des modes opératoires classiques de formation de sels.

16. Un procédé pour la préparation du succinate du 1 - méthyl - 5 - [[3 - [3 - (1 - pipéridinylméthyl) - phénoxy] - propyl] - amino] - 1H - 1,2,4 - triazole - 3 - méthanol qui consiste à convertir la base libre en le succinate par des modes opératoirs classiques de formation de sels.

17. Un procédé selon la Rev. 1 (d) pour la préparation du 1 - méthyl - 5 - [[3 - [3 - (1 - pipéridinylméthyl) - phénoxy] - propyl] - amino] - 1H - 1,2,4 - triazole - 3 - méthanol, consistant à réduire le 1 - méthyl - 5 - [[1 - oxo - 4 - [3 - (1 - pipéridinylméthyl) - phénoxy] - propyl] - amino - 1H - 1,2,4 - triazole - 3 - carboxylate de méthyle.

18. Un procédé selon la Rev. 1 (c), pour la préparation du 1 - méthyl - 5 - [[3 - [3 - [(1 - pipéridinylméthyl) - phénoxy] - propyl] - amino] - 1H - 1,2,4 - triazole - 3 - méthanol, qui consiste à cycliser le produit de réaction de la 3 - [3 - (1 - pipéridinylméthyl) - phénoxy] - propanamine et du N - [2 - (acétyloxy) - acétyl] - 1 - méthyl - 2 - (phénylméthylène) - hydrazinecarboximidothioate de méthyle en milieu acide.